# EUROPEAN PATENT SPECIFICATION

(11) **EP 2 857 039 B1**
(45) Date of publication and mention of the grant of the patent: **20.11.2019**
(21) Application number: 13797290.7
(22) Date of filing: 30.05.2013
(51) Int. Cl.: A61K 39/395, A61P 25/28, C07K 16/18, C12N 15/02, C12P 21/08, C07K 16/46

(54) **THERAPEUTIC AGENT OR PROPHYLACTIC AGENT FOR DEMENTIA**
THERAPEUTIKUM ODER PROPHYLAKTIKUM GEGEN DEMENZ
AGENT THÉRAPEUTIQUE OU AGENT PROPHYLACTIQUE POUR LA DÉMENCE

(30) Priority: 31.05.2012 JP 2012124336
(43) Date of publication of application: 08.04.2015
(73) Proprietor: Osaka City University, Osaka-shi, Osaka 558-8585 (JP); Teijin Pharma Limited, Tokyo 100-0013 (JP)
(72) Inventor: MORI, Hiroshi, Osaka-shi Osaka 545-8585 (JP); TOMIYAMA, Takami, Osaka-shi Osaka 545-8585 (JP); MATSUMOTO, Yoichi, Hino-shi Tokyo 191-0065 (JP); EGUCHI, Hiroshi, Chiyoda-ku Tokyo 100-0013 (JP); KUNORI, Yuichi, Hino-shi Tokyo 191-0065 (JP)
(74) Representative: Jones Day
(86) International application number: PCT/JP2013/065090
(87) International publication number: WO 2013/180238

(56) References cited:
- WO-A1-97/34145
- WO-A2-2010/115843
- WO-A2-2010/142423
- WO-A2-2010/144711
- WO-A2-2010/144711
- JP-A- 2008 013 566
- US-A1- 2002 086 009
- US-A1- 2008 050 383
- ALLAL BOUTAJANGOUT ET AL: "Passive immunization targeting pathological phospho-tau protein in a mouse model reduces functional decline and clears tau aggregates from the brain", JOURNAL OF NEUROCHEMISTRY, vol. 118, no. 4, 1 August 2011 (2011-08-01), pages 658-667, XP055081677, ISSN: 0022-3042, DOI: 10.1111/j.1471-4159.2011.07337.x
- TOMOHIRO UMEDA ET AL: "Passive immunotherapy of tauopathy targeting pSer413-tau: a pilot study in mice", ANNALS OF CLINICAL AND TRANSLATIONAL NEUROLOGY, 1 January 2015 (2015-01-01), pages n/a-n/a, XP055174169, ISSN: 2328-9503, DOI: 10.1002/acn3.171
- X. CHAI ET AL: "Passive immunization with anti-tau antibodies in two transgenic models: Reduction of tau pathology and delay of disease progression", JOURNAL OF BIOLOGICAL CHEMISTRY, 1 August 2011 (2011-08-01), XP055005064, ISSN: 0021-9258, DOI: 10.1074/jbc.M111.229633
- WISNIEWSKI T ET AL: "Murine models of Alzheimer's disease and their use in developing immunotherapies", BIOCHIMICA ET BIOPHYSICA ACTA. MOLECULAR BASIS OF DISEASE, AMSTERDAM, NL, vol. 1802, no. 10, 1 October 2010 (2010-10-01), pages 847-859, XP027235769, ISSN: 0925-4439 [retrieved on 2010-05-13]
- A. BOUTAJANGOUT ET AL: "Immunotherapy Targeting Pathological Tau Prevents Cognitive Decline in a New Tangle Mouse Model", JOURNAL OF NEUROSCIENCE, vol. 30, no. 49, 8 December 2010 (2010-12-08), pages 16559-16566, XP055203597, ISSN: 0270-6474, DOI: 10.1523/JNEUROSCI.4363-10.2010
- NOBLE, W. ET AL.: 'Advances in tau-based drug discovery' EXPERT OPIN. DRUG DISCOV. vol. 6, no. 8, 2011, pages 797 - 810, XP055180333

### SEQUENCE LISTING

<110> Osaka City Univercity & Teijin Pharma Limited
<120> Therapeutic agents for demetia
<130> AB606
<150> JP2012-124336
   <151> 2012-05-31
<160> 48
<170> PatentIn version 3.1
<210> 1
   <211> 441
   <212> PRT
   <213> Homo sapiens
<400> 1
<210> 2
   <211> 412
   <212> PRT
   <213> Homo sapiens
<400> 2
   Met Ala Glu Pro Arg Gln Glu Phe Glu Val Met Glu Asp His Ala Gly
   1               5                   10                  15
   Thr Tyr Gly Leu Gly Asp Arg Lys Asp Gln Gly Gly Tyr Thr Met His
               20                  25                  30
   Gln Asp Gln Glu Gly Asp Thr Asp Ala Gly Leu Lys Glu Ser Pro Leu
           35                  40                  45
   Gln Thr Pro Thr Glu Asp Gly Ser Glu Glu Pro Gly Ser Glu Thr Ser
       50                  55                  60
   Asp Ala Lys Ser Thr Pro Thr Ala Glu Ala Glu Glu Ala Gly Ile Gly
   65                  70                  75                  80
   Asp Thr Pro Ser Leu Glu Asp Glu Ala Ala Gly His Val Thr Gln Ala
                   85                  90                  95
   Arg Met Val Ser Lys Ser Lys Asp Gly Thr Gly Ser Asp Asp Lys Lys
               100                 105                 110
   Ala Lys Gly Ala Asp Gly Lys Thr Lys Ile Ala Thr Pro Arg Gly Ala
           115                 120                 125
   Ala Pro Pro Gly Gln Lys Gly Gln Ala Asn Ala Thr Arg Ile Pro Ala
       130                 135                 140
   Lys Thr Pro Pro Ala Pro Lys Thr Pro Pro Ser Ser Gly Glu Pro Pro
   145                 150                 155                 160
   Lys Ser Gly Asp Arg Ser Gly Tyr Ser Ser Pro Gly Ser Pro Gly Thr
                   165                 170                 175
   Pro Gly Ser Arg Ser Arg Thr Pro Ser Leu Pro Thr Pro Pro Thr Arg
               180                 185                 190
   Glu Pro Lys Lys Val Ala Val Val Arg Thr Pro Pro Lys Ser Pro Ser
   195 200 205
   Ser Ala Lys Ser Arg Leu Gln Thr Ala Pro Val Pro Met Pro Asp Leu
   210 215 220
   Lys Asn Val Lys Ser Lys Ile Gly Ser Thr Glu Asn Leu Lys His Gln
   225 230 235 240
   Pro Gly Gly Gly Lys Val Gln Ile Ile Asn Lys Lys Leu Asp Leu Ser
   245 250 255
   Asn Val Gln Ser Lys Cys Gly Ser Lys Asp Asn Ile Lys His Val Pro
   260 265 270
   Gly Gly Gly Ser Val Gln Ile Val Tyr Lys Pro Val Asp Leu Ser Lys
   275 280 285
   Val Thr Ser Lys Cys Gly Ser Leu Gly Asn Ile His His Lys Pro Gly
   290 295 300
   Gly Gly Gln Val Glu Val Lys Ser Glu Lys Leu Asp Phe Lys Asp Arg
   305 310 315 320
   Val Gln Ser Lys Ile Gly Ser Leu Asp Asn Ile Thr His Val Pro Gly
   325 330 335
   Gly Gly Asn Lys Lys Ile Glu Thr His Lys Leu Thr Phe Arg Glu Asn
   340 345 350
   Ala Lys Ala Lys Thr Asp His Gly Ala Glu Ile Val Tyr Lys Ser Pro
   355 360 365
   Val Val Ser Gly Asp Thr Ser Pro Arg His Leu Ser Asn Val Ser Ser
   370 375 380
   Thr Gly Ser Ile Asp Met Val Asp Ser Pro Gln Leu Ala Thr Leu Ala
   385 390 395 400
   Asp Glu Val Ser Ala Ser Leu Ala Lys Gln Gly Leu
   405 410
<210> 3
   <211> 383
   <212> PRT
   <213> Homo sapiens
<400> 3
   Met Ala Glu Pro Arg Gln Glu Phe Glu Val Met Glu Asp His Ala Gly
   1               5                   10                  15
   Thr Tyr Gly Leu Gly Asp Arg Lys Asp Gln Gly Gly Tyr Thr Met His
               20                  25                  30
   Gln Asp Gln Glu Gly Asp Thr Asp Ala Gly Leu Lys Ala Glu Glu Ala
           35                  40                  45
   Gly Ile Gly Asp Thr Pro Ser Leu Glu Asp Glu Ala Ala Gly His Val
       50                  55                  60
   Thr Gln Ala Arg Met Val Ser Lys Ser Lys Asp Gly Thr Gly Ser Asp
   65                  70                  75                  80
   Asp Lys Lys Ala Lys Gly Ala Asp Gly Lys Thr Lys Ile Ala Thr Pro
                   85                  90                  95
   Arg Gly Ala Ala Pro Pro Gly Gln Lys Gly Gln Ala Asn Ala Thr Arg
               100                 105                 110
   Ile Pro Ala Lys Thr Pro Pro Ala Pro Lys Thr Pro Pro Ser Ser Gly
           115                 120                 125
   Glu Pro Pro Lys Ser Gly Asp Arg Ser Gly Tyr Ser Ser Pro Gly Ser
       130                 135                 140
   Pro Gly Thr Pro Gly Ser Arg Ser Arg Thr Pro Ser Leu Pro Thr Pro
   145                 150                 155                 160
   Pro Thr Arg Glu Pro Lys Lys Val Ala Val Val Arg Thr Pro Pro Lys
   165                 170                 175
   Ser Pro Ser Ser Ala Lys Ser Arg Leu Gln Thr Ala Pro Val Pro Met
   180                 185                 190
   Pro Asp Leu Lys Asn Val Lys Ser Lys Ile Gly Ser Thr Glu Asn Leu
           195                 200                 205
   Lys His Gln Pro Gly Gly Gly Lys Val Gln Ile Ile Asn Lys Lys Leu
   210                 215                 220
   Asp Leu Ser Asn Val Gln Ser Lys Cys Gly Ser Lys Asp Asn Ile Lys
   225                 230                 235                 240
   His Val Pro Gly Gly Gly Ser Val Gln Ile Val Tyr Lys Pro Val Asp
                   245                 250                 255
   Leu Ser Lys Val Thr Ser Lys Cys Gly Ser Leu Gly Asn Ile His His
   260 265 270
   Lys Pro Gly Gly Gly Gln Val Glu Val Lys Ser Glu Lys Leu Asp Phe
   275 280 285
   Lys Asp Arg Val Gln Ser Lys Ile Gly Ser Leu Asp Asn Ile Thr His
   290 295 300
   Val Pro Gly Gly Gly Asn Lys Lys Ile Glu Thr His Lys Leu Thr Phe
   305 310 315 320
   Arg Glu Asn Ala Lys Ala Lys Thr Asp His Gly Ala Glu Ile Val Tyr
   325 330 335
   Lys Ser Pro Val Val Ser Gly Asp Thr Ser Pro Arg His Leu Ser Asn
   340 345 350
   Val Ser Ser Thr Gly Ser Ile Asp Met Val Asp Ser Pro Gln Leu Ala
   355 360 365
   Thr Leu Ala Asp Glu Val Ser Ala Ser Leu Ala Lys Gln Gly Leu
   370 375 380
<210> 4
   <211> 410
   <212> PRT
   <213> Homo sapiens
<400> 4
   Met Ala Glu Pro Arg Gln Glu Phe Glu Val Met Glu Asp His Ala Gly
   1 5 10 15
   Thr Tyr Gly Leu Gly Asp Arg Lys Asp Gln Gly Gly Tyr Thr Met His
   20 25 30
   Gln Asp Gln Glu Gly Asp Thr Asp Ala Gly Leu Lys Glu Ser Pro Leu
   35 40 45
   Gln Thr Pro Thr Glu Asp Gly Ser Glu Glu Pro Gly Ser Glu Thr Ser
   50 55 60
   Asp Ala Lys Ser Thr Pro Thr Ala Glu Asp Val Thr Ala Pro Leu Val
   65 70 75 80
   Asp Glu Gly Ala Pro Gly Lys Gln Ala Ala Ala Gln Pro His Thr Glu
   85 90 95
   Ile Pro Glu Gly Thr Thr Ala Glu Glu Ala Gly Ile Gly Asp Thr Pro
               100                 105                 110
   Ser Leu Glu Asp Glu Ala Ala Gly His Val Thr Gln Ala Arg Met Val
           115                 120                 125
   Ser Lys Ser Lys Asp Gly Thr Gly Ser Asp Asp Lys Lys Ala Lys Gly
       130                 135                 140
   Ala Asp Gly Lys Thr Lys Ile Ala Thr Pro Arg Gly Ala Ala Pro Pro
   145                 150                 155                 160
   Gly Gln Lys Gly Gln Ala Asn Ala Thr Arg Ile Pro Ala Lys Thr Pro
                   165                 170                 175
   Pro Ala Pro Lys Thr Pro Pro Ser Ser Gly Glu Pro Pro Lys Ser Gly
               180                 185                 190
   Asp Arg Ser Gly Tyr Ser Ser Pro Gly Ser Pro Gly Thr Pro Gly Ser
   195                 200                 205
   Arg Ser Arg Thr Pro Ser Leu Pro Thr Pro Pro Thr Arg Glu Pro Lys
   210                 215                 220
   Lys Val Ala Val Val Arg Thr Pro Pro Lys Ser Pro Ser Ser Ala Lys
   225                 230                 235                 240
   Ser Arg Leu Gln Thr Ala Pro Val Pro Met Pro Asp Leu Lys Asn Val
                   245                 250                 255
   Lys Ser Lys Ile Gly Ser Thr Glu Asn Leu Lys His Gln Pro Gly Gly
               260                 265                 270
   Gly Lys Val Gln Ile Val Tyr Lys Pro Val Asp Leu Ser Lys Val Thr
           275                 280                 285
   Ser Lys Cys Gly Ser Leu Gly Asn Ile His His Lys Pro Gly Gly Gly
       290                 295                 300
   Gln Val Glu Val Lys Ser Glu Lys Leu Asp Phe Lys Asp Arg Val Gln
   305                 310                 315                 320
   Ser Lys Ile Gly Ser Leu Asp Asn Ile Thr His Val Pro Gly Gly Gly
                   325                 330                 335
   Asn Lys Lys Ile Glu Thr His Lys Leu Thr Phe Arg Glu Asn Ala Lys
               340                 345                 350
   Ala Lys Thr Asp His Gly Ala Glu Ile Val Tyr Lys Ser Pro Val Val
   355 360 365
   Ser Gly Asp Thr Ser Pro Arg His Leu Ser Asn Val Ser Ser Thr Gly
   370 375 380
   Ser Ile Asp Met Val Asp Ser Pro Gln Leu Ala Thr Leu Ala Asp Glu
   385 390 395 400
   Val Ser Ala Ser Leu Ala Lys Gln Gly Leu
   405 410
<210> 5
   <211> 381
   <212> PRT
   <213> Homo sapiens
<400> 5
   Met Ala Glu Pro Arg Gln Glu Phe Glu Val Met Glu Asp His Ala Gly
   1 5 10 15
   Thr Tyr Gly Leu Gly Asp Arg Lys Asp Gln Gly Gly Tyr Thr Met His
   20 25 30
   Gln Asp Gln Glu Gly Asp Thr Asp Ala Gly Leu Lys Glu Ser Pro Leu
   35 40 45
   Gln Thr Pro Thr Glu Asp Gly Ser Glu Glu Pro Gly Ser Glu Thr Ser
   50 55 60
   Asp Ala Lys Ser Thr Pro Thr Ala Glu Ala Glu Glu Ala Gly Ile Gly
   65 70 75 80
   Asp Thr Pro Ser Leu Glu Asp Glu Ala Ala Gly His Val Thr Gln Ala
   85 90 95
   Arg Met Val Ser Lys Ser Lys Asp Gly Thr Gly Ser Asp Asp Lys Lys
   100 105 110
   Ala Lys Gly Ala Asp Gly Lys Thr Lys Ile Ala Thr Pro Arg Gly Ala
   115 120 125
   Ala Pro Pro Gly Gln Lys Gly Gln Ala Asn Ala Thr Arg Ile Pro Ala
   130 135 140
   Lys Thr Pro Pro Ala Pro Lys Thr Pro Pro Ser Ser Gly Glu Pro Pro
   145 150 155 160
   Lys Ser Gly Asp Arg Ser Gly Tyr Ser Ser Pro Gly Ser Pro Gly Thr
   165 170 175
   Pro Gly Ser Arg Ser Arg Thr Pro Ser Leu Pro Thr Pro Pro Thr Arg
   180 185 190
   Glu Pro Lys Lys Val Ala Val Val Arg Thr Pro Pro Lys Ser Pro Ser
   195 200 205
   Ser Ala Lys Ser Arg Leu Gln Thr Ala Pro Val Pro Met Pro Asp Leu
   210 215 220
   Lys Asn Val Lys Ser Lys Ile Gly Ser Thr Glu Asn Leu Lys His Gln
   225 230 235 240
   Pro Gly Gly Gly Lys Val Gln Ile Val Tyr Lys Pro Val Asp Leu Ser
   245 250 255
   Lys Val Thr Ser Lys Cys Gly Ser Leu Gly Asn Ile His His Lys Pro
   260 265 270
   Gly Gly Gly Gln Val Glu Val Lys Ser Glu Lys Leu Asp Phe Lys Asp
   275 280 285
   Arg Val Gln Ser Lys Ile Gly Ser Leu Asp Asn Ile Thr His Val Pro
   290 295 300
   Gly Gly Gly Asn Lys Lys Ile Glu Thr His Lys Leu Thr Phe Arg Glu
   305 310 315 320
   Asn Ala Lys Ala Lys Thr Asp His Gly Ala Glu Ile Val Tyr Lys Ser
   325 330 335
   Pro Val Val Ser Gly Asp Thr Ser Pro Arg His Leu Ser Asn Val Ser
   340 345 350
   Ser Thr Gly Ser Ile Asp Met Val Asp Ser Pro Gln Leu Ala Thr Leu
   355 360 365
   Ala Asp Glu Val Ser Ala Ser Leu Ala Lys Gln Gly Leu
   370 375 380
<210> 6
   <211> 352
   <212> PRT
   <213> Homo sapiens
<400> 6
   Met Ala Glu Pro Arg Gln Glu Phe Glu Val Met Glu Asp His Ala Gly
   1               5                   10                  15
   Thr Tyr Gly Leu Gly Asp Arg Lys Asp Gln Gly Gly Tyr Thr Met His
               20                  25                  30
   Gln Asp Gln Glu Gly Asp Thr Asp Ala Gly Leu Lys Ala Glu Glu Ala
           35                  40                  45
   Gly Ile Gly Asp Thr Pro Ser Leu Glu Asp Glu Ala Ala Gly His Val
       50                  55                  60
   Thr Gln Ala Arg Met Val Ser Lys Ser Lys Asp Gly Thr Gly Ser Asp
   65                  70                  75                  80
   Asp Lys Lys Ala Lys Gly Ala Asp Gly Lys Thr Lys Ile Ala Thr Pro
                   85                  90                  95
   Arg Gly Ala Ala Pro Pro Gly Gln Lys Gly Gln Ala Asn Ala Thr Arg
               100                 105                 110
   Ile Pro Ala Lys Thr Pro Pro Ala Pro Lys Thr Pro Pro Ser Ser Gly
           115                 120                 125
   Glu Pro Pro Lys Ser Gly Asp Arg Ser Gly Tyr Ser Ser Pro Gly Ser
       130                 135                 140
   Pro Gly Thr Pro Gly Ser Arg Ser Arg Thr Pro Ser Leu Pro Thr Pro
   145                 150                 155                 160
   Pro Thr Arg Glu Pro Lys Lys Val Ala Val Val Arg Thr Pro Pro Lys
   165                 170                 175
   Ser Pro Ser Ser Ala Lys Ser Arg Leu Gln Thr Ala Pro Val Pro Met
   180                 185                 190
   Pro Asp Leu Lys Asn Val Lys Ser Lys Ile Gly Ser Thr Glu Asn Leu
           195                 200                 205
   Lys His Gln Pro Gly Gly Gly Lys Val Gln Ile Val Tyr Lys Pro Val
       210                 215                 220
   Asp Leu Ser Lys Val Thr Ser Lys Cys Gly Ser Leu Gly Asn Ile His
   225                 230                 235                 240
   His Lys Pro Gly Gly Gly Gln Val Glu Val Lys Ser Glu Lys Leu Asp
   245 250 255
   Phe Lys Asp Arg Val Gln Ser Lys Ile Gly Ser Leu Asp Asn Ile Thr
   260 265 270
   His Val Pro Gly Gly Gly Asn Lys Lys Ile Glu Thr His Lys Leu Thr
   275 280 285
   Phe Arg Glu Asn Ala Lys Ala Lys Thr Asp His Gly Ala Glu Ile Val
   290 295 300
   Tyr Lys Ser Pro Val Val Ser Gly Asp Thr Ser Pro Arg His Leu Ser
   305 310 315 320
   Asn Val Ser Ser Thr Gly Ser Ile Asp Met Val Asp Ser Pro Gln Leu
   325 330 335
   Ala Thr Leu Ala Asp Glu Val Ser Ala Ser Leu Ala Lys Gln Gly Leu
   340 345 350
<210> 7
   <211> 5
   <212> PRT
   <213> Mus musculus
<400> 7
   Ser Phe Ala Met Asn
   1 5
<210> 8
   <211> 5
   <212> PRT
   <213> Mus musculus
<400> 8
   Ser Phe Ala Leu Asn
   1 5
<210> 9
   <211> 19
   <212> PRT
   <213> Mus musculus
<400> 9
   His Ile Arg Ser Lys Thr Asn Asn Tyr Ala Thr Phe Tyr Ala Asp Ser
   1 5 10 15
   Val Lys Asp
<210> 10
   <211> 19
   <212> PRT
   <213> Mus musculus
<400> 10
   His Ile Arg Ser Lys Thr Asn Asn Tyr Val Thr Phe Tyr Ala Asp Ser
   1               5                   10                  15
   Val Lys Gly
<210> 11
   <211> 19
   <212> PRT
   <213> Mus musculus
<400> 11
   His Ile Arg Ser Lys Ser Asn Asn Tyr Val Thr Phe Tyr Ala Asp Ser
   1               5                   10                  15
   Val Lys Asp
<210> 12
   <211> 19
   <212> PRT
   <213> Mus musculus
<400> 12
   His Ile Arg Ser Lys Ala Asn His Tyr Val Thr Phe Tyr Ala Asp Ser
   1               5                   10                  15
   Val Lys Asp
<210> 13
   <211> 10
   <212> PRT
   <213> Mus musculus
<400> 13
   Arg Gly Pro Arg Asp Ser Trp Phe Gly Tyr
   1               5                   10
<210> 14
   <211> 16
   <212> PRT
   <213> Mus musculus
<400> 14
   Arg Ser Ser Gln Asn Ile Val His Ser Asn Gly Asn Thr Tyr Leu Glu
   1               5                   10                  15
<210> 15
   <211> 16
   <212> PRT
   <213> Mus musculus
<400> 15
   Arg Ser Ser Gln Asn Ile Val His Thr Asn Gly Asn Thr Tyr Leu Glu
   1               5                   10                  15
<210> 16
   <211> 7
   <212> PRT
   <213> Mus musculus
<400> 16
   Thr Val Ser Asn Arg Phe Ser
   1               5
<210> 17
   <211> 9
   <212> PRT
   <213> Mus musculus
<400> 17
   Phe Gln Gly Ser His Leu Pro Leu Thr
   1               5
<210> 18
   <211> 121
   <212> PRT
   <213> Mus musculus
<400> 18
   Glu Val His Leu Val Glu Ser Gly Gly Glu Leu Val Gln Pro Lys Gly
   1               5                   10                  15
   Ser Leu Lys Leu Ser Cys Ala Ala Ser Gly Phe Ala Phe Asn Ser Phe
               20                  25                  30
   Ala Met Asn Trp Val Arg Gln Thr Pro Gly Lys Gly Leu Asn Trp Val
           35                  40                  45
   Ala His Ile Arg Ser Lys Thr Asn Asn Tyr Ala Thr Phe Tyr Ala Asp
       50                  55                  60
   Ser Val Lys Asp Arg Phe Thr Ile Ser Arg Asp Asp Ser Gln Asn Met
   65                  70                  75                  80
   Leu Tyr Leu Gln Met Asn Asn Leu Lys Thr Glu Asp Thr Gly Ile Tyr
   85 90 95
   Tyr Cys Val Arg Arg Gly Pro Arg Asp Ser Trp Phe Gly Tyr Trp Gly
   100 105 110
   Gln Gly Thr Leu Val Thr Val Ser Ala
   115 120
<210> 19
   <211> 121
   <212> PRT
   <213> Mus musculus
<400> 19
   Glu Val Gln Leu Val Glu Ser Gly Gly Gly Leu Val Gln Pro Lys Gly
   1 5 10 15
   Ser Leu Lys Leu Ser Cys Ala Ala Ser Gly Phe Ala Phe Asn Ser Phe
   20 25 30
   Ala Leu Asn Trp Val Arg Gln Ala Pro Gly Lys Ser Leu Glu Trp Val
   35 40 45
   Val His Ile Arg Ser Lys Thr Asn Asn Tyr Ala Thr Phe Tyr Ala Asp
   50 55 60
   Ser Val Lys Asp Arg Phe Thr Val Ser Arg Asp Asp Ser Gln Asn Met
   65 70 75 80
   Val Tyr Leu Gln Met Asn Asn Leu Lys Thr Glu Asp Thr Gly Ile Tyr
   85 90 95
   Tyr Cys Val Arg Arg Gly Pro Arg Asp Ser Trp Phe Gly Tyr Trp Gly
   100 105 110
   Gln Gly Thr Leu Val Thr Val Ser Ala
   115 120
<210> 20
   <211> 121
   <212> PRT
   <213> Mus musculus
<400> 20
   Glu Val Gln Leu Val Glu Ser Gly Gly Gly Leu Val Gln Pro Lys Gly
   1 5 10 15
   Ser Leu Lys Leu Ser Cys Ala Ala Ser Gly Phe Ala Phe Asn Ser Phe
   20 25 30
   Ala Leu Asn Trp Val Arg Gln Ala Pro Gly Lys Ser Leu Glu Trp Val
   35 40 45
   Val His Ile Arg Ser Lys Thr Asn Asn Tyr Ala Thr Phe Tyr Ala Asp
   50 55 60
   Ser Val Lys Asp Arg Phe Thr Val Ser Arg Asp Asp Ser Gln Ser Met
   65 70 75 80
   Val Tyr Leu Gln Met Asn Asn Leu Lys Thr Glu Asp Thr Gly Ile Tyr
   85 90 95
   Tyr Cys Val Arg Arg Gly Pro Arg Asp Ser Trp Phe Gly Tyr Trp Gly
   100 105 110
   Gln Gly Thr Leu Val Thr Val Ser Ala
   115 120
<210> 21
   <211> 121
   <212> PRT
   <213> Mus musculus
<400> 21
   Glu Val His Leu Val Glu Ser Gly Gly Gly Leu Val Gln Pro Lys Gly
   1 5 10 15
   Ser Leu Lys Leu Ser Cys Ala Ala Ser Gly Phe Ala Phe Asn Ser Phe
   20 25 30
   Ala Met Asn Trp Val Arg Gln Ala Pro Gly Lys Ala Leu Asp Trp Ile
   35 40 45
   Ala His Ile Arg Ser Lys Thr Asn Asn Tyr Val Thr Phe Tyr Ala Asp
   50 55 60
   Ser Val Lys Gly Arg Phe Ser Ile Ser Arg Asp Asp Ser Gln Ser Met
   65 70 75 80
   Val Tyr Leu Gln Met Asn Asn Leu Gln Thr Glu Asp Thr Gly Ile Tyr
   85 90 95
   Tyr Cys Val Arg Arg Gly Pro Arg Asp Ser Trp Phe Gly Tyr Trp Gly
   100 105 110
   Gln Gly Thr Leu Val Thr Val Ser Ala
   115 120
<210> 22
   <211> 121
   <212> PRT
   <213> Mus musculus
<400> 22
   Glu Ile Gln Leu Val Glu Ser Gly Gly Gly Leu Val Gln Pro Lys Gly
   1 5 10 15
   Ser Leu Lys Leu Ser Cys Ala Ala Ser Gly Phe Ala Phe Ser Ser Phe
   20 25 30
   Ala Leu Asn Trp Val Arg Gln Ala Pro Gly Lys Ser Leu Asp Trp Val
   35 40 45
   Ala His Ile Arg Ser Lys Ala Asn His Tyr Val Thr Phe Tyr Ala Asp
   50 55 60
   Ser Val Lys Asp Arg Phe Thr Val Ser Arg Asp Asp Ser Gln Ser Met
   65 70 75 80
   Val Tyr Leu Gln Met Asn Asn Leu Lys Thr Glu Asp Thr Gly Ile Tyr
   85 90 95
   Tyr Cys Val Arg Arg Gly Pro Arg Asp Ser Trp Phe Gly Tyr Trp Gly
   100 105 110
   Gln Gly Thr Leu Val Thr Val Ser Ala
   115 120
<210> 23
   <211> 121
   <212> PRT
   <213> Mus musculus
<400> 23
   Glu Val His Leu Val Glu Ser Gly Gly Gly Leu Val Gln Pro Lys Gly
   1 5 10 15
   Ser Leu Lys Leu Ser Cys Ala Ala Ser Gly Phe Ala Phe Asn Ser Phe
   20 25 30
   Ala Met Asn Trp Val Arg Gln Ala Pro Gly Lys Gly Leu Asp Trp Ile
   35 40 45
   Ala His Ile Arg Ser Lys Thr Asn Asn Tyr Val Thr Phe Tyr Ala Asp
   50 55 60
   Ser Val Lys Gly Arg Phe Ser Ile Ser Arg Asp Asp Ser Gln Ser Met
   65 70 75 80
   Val Tyr Leu Gln Met Asn Asn Leu Gln Thr Glu Asp Thr Gly Ile Tyr
   85 90 95
   Tyr Cys Val Arg Arg Gly Pro Arg Asp Ser Trp Phe Gly Tyr Trp Gly
   100 105 110
   Gln Gly Thr Leu Val Thr Val Ser Ala
   115 120
<210> 24
   <211> 121
   <212> PRT
   <213> Mus musculus
<400> 24
   Glu Val Gln Leu Val Glu Ser Gly Gly Gly Leu Val Gln Pro Lys Gly
   1               5                   10                  15
   Ser Leu Lys Leu Ser Cys Ala Ala Ser Gly Phe Ala Phe Asn Ser Phe
               20                  25                  30
   Ala Met Asn Trp Val Arg Gln Ala Pro Gly Lys Gly Leu Asp Trp Ile
           35                  40                  45
   Ala His Ile Arg Ser Lys Ser Asn Asn Tyr Val Thr Phe Tyr Ala Asp
       50                  55                  60
   Ser Val Lys Asp Arg Phe Ile Ile Ser Arg Asp Asp Ser Gln Ser Met
   65                  70                  75                  80
   Ile Tyr Leu Gln Met Asn Asn Leu Lys Thr Glu Asp Ser Gly Ile Tyr
                   85                  90                  95
   Tyr Cys Val Arg Arg Gly Pro Arg Asp Ser Trp Phe Gly Tyr Trp Gly
               100                 105                 110
   Gln Gly Thr Leu Val Thr Val Ser Ala
           115                 120
<210> 25
   <211> 109
   <212> PRT
   <213> Mus musculus
<400> 25
   Asp Val Leu Met Thr Gln Thr Pro Leu Ser Leu Pro Val Ser Leu Gly
   1 5 10 15
   Asp His Ala Ser Ile Ser Cys Arg Ser Ser Gln Asn Ile Val His Ser
   20 25 30
   Asn Gly Asn Thr Tyr Leu Glu Trp Tyr Leu Gln Lys Pro Gly Gln Ser
   35 40 45
   Pro Lys Val Leu Ile Tyr Thr Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Leu Pro Leu Thr Phe Gly Gly Gly Thr Lys Leu
   100 105
<210> 26
   <211> 109
   <212> PRT
   <213> Mus musculus
<400> 26
   Asp Ile Leu Met Thr Gln Thr Pro Leu Ser Leu Pro Val Ser Leu Gly
   1 5 10 15
   Asp Gln Ala Ser Ile Ser Cys Arg Ser Ser Gln Asn Ile Val His Ser
   20 25 30
   Asn Gly Asn Thr Tyr Leu Glu Trp Tyr Leu Gln Lys Pro Gly Gln Ser
   35 40 45
   Pro Lys Val Leu Ile Tyr Thr Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Leu Pro Leu Thr Phe Gly Gly Gly Thr Lys Leu
   100 105
<210> 27
   <211> 109
   <212> PRT
   <213> Mus musculus
<400> 27
   Gly Val Leu Met Thr Gln Ile Pro Leu Ser Leu Pro Val Ser Leu Gly
   1               5                   10                  15
   Asp Gln Ala Ser Ile Ser Cys Arg Ser Ser Gln Asn Ile Val His Ser
               20                  25                  30
   Asn Gly Asn Thr Tyr Leu Glu Trp Tyr Leu Gln Lys Pro Gly Gln Ser
           35                  40                  45
   Pro Lys Val Leu Ile Tyr Thr Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Leu Pro Leu Thr Phe Gly Gly Gly Thr Lys Leu
               100                 105
<210> 28
   <211> 109
   <212> PRT
   <213> Mus musculus
<400> 28
   Asp Val Leu Leu Thr Gln Thr Pro Leu Ser Leu Pro Val Ser Leu Gly
   1               5                   10                  15
   Asp Gln Ala Ser Ile Ser Cys Arg Ser Ser Gln Asn Ile Val His Ser
               20                  25                  30
   Asn Gly Asn Thr Tyr Leu Glu Trp Tyr Leu Gln Lys Pro Gly Gln Ser
           35                  40                  45
   Pro Lys Val Leu Ile Tyr Thr Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
   85 90 95
   Ser His Leu Pro Leu Thr Phe Gly Gly Gly Thr Lys Leu
   100 105
<210> 29
   <211> 109
   <212> PRT
   <213> Mus musculus
<400> 29
   Val Val Leu Met Thr Gln Thr Pro Leu Ser Leu Pro Val Ser Leu Gly
   1 5 10 15
   Asp Gln Ala Ser Ile Ser Cys Arg Ser Ser Gln Asn Ile Val His Ser
   20 25 30
   Asn Gly Asn Thr Tyr Leu Glu Trp Tyr Leu Gln Lys Pro Gly Gln Ser
   35 40 45
   Pro Lys Val Leu Ile Tyr Thr Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Leu Pro Leu Thr Phe Gly Gly Gly Thr Lys Leu
   100 105
<210> 30
   <211> 109
   <212> PRT
   <213> Mus musculus
<400> 30
   Asp Val Leu Met Thr Gln Ser Pro Leu Ser Leu Pro Val Ser Leu Gly
   1               5                   10                  15
   Asp Gln Ala Ser Ile Ser Cys Arg Ser Ser Gln Asn Ile Val His Thr
               20                  25                  30
   Asn Gly Asn Thr Tyr Leu Glu Trp Tyr Leu Gln Lys Pro Gly Gln Ser
           35                  40                  45
   Pro Lys Val Leu Ile Tyr Thr Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Leu Pro Leu Thr Phe Gly Gly Gly Thr Lys Leu
               100                 105
<210> 31
   <211> 8
   <212> PRT
   <213> Artificial
<220>
   <223> Synthetic peptide
<220>
   <221> MOD_RES
   <222> (4) .. (4)
   <223> PHOSPHORYLATION
<400> 31
   Asn Val Ser Ser Thr Gly Ser Cys
   1               5
<210> 32
   <211> 14
   <212> PRT
   <213> Artificial
<220>
   <223> Synthetic peptide
<220>
   <221> MOD_RES
   <222> (9)..(9)
   <223> PHOSPHORYLATION
<400> 32
   Gly Cys His Leu Ser Asn Val Ser Ser Thr Gly Ser Ile Asp
   1 5 10
<210> 33
   <211> 17
   <212> PRT
   <213> Artificial
<220>
   <223> Synthetic peptide
<220>
   <221> MOD_RES
   <222> (9)..(9)
   <223> PHOSPHORYLATION
<400> 33
   Pro Arg His Leu Ser Asn Val Ser Ser Thr Gly Ser Ile Asp Met Val
   1 5 10 15
   Asp
<210> 34
   <211> 17
   <212> PRT
   <213> Artificial
<220>
   <223> Synthetic peptide
<400> 34
   Pro Arg His Leu Ser Asn Val Ser Ser Thr Gly Ser Ile Asp Met Val
   1 5 10 15
   Asp
<210> 35
   <211> 25
   <212> PRT
   <213> Artificial
<220>
   <223> Synthetic peptide
<220>
   <221> MOD_RES
   <222> (15) .. (15)
   <223> PHOSPHORYLATION
<400> 35
   Gly Cys Ser Gly Thr Ser Pro Arg His Leu Ser Asn Val Ser Ser Thr
   1 5 10 15
   Gly Ser Ile Asp Met Val Asp Ser Pro
   20 25
<210> 36
   <211> 6
   <212> PRT
   <213> Artificial
<220>
   <223> Synthetic peptide
<220>
   <221> MOD_RES
   <222> (5) .. (5)
   <223> PHOSPHORYLATION
<400> 36
   Gly Leu Lys Glu Ser Pro
   1 5
<210> 37
   <211> 10
   <212> PRT
   <213> Artificial
<220>
   <223> Synthetic peptide
<220>
   <221> MOD_RES
   <222> (5) .. (5)
   <223> PHOSPHORYLATION
<400> 37
   Ser Gly Tyr Ser Ser Pro Gly Ser Pro Gly
   1 5 10
<210> 38
   <211> 11
   <212> PRT
   <213> Artificial
<220>
   <223> Synthetic peptide
<220>
   <221> MOD_RES
   <222> (5) .. (5)
   <223> PHOSPHORYLATION
<400> 38
   Ser Ser Pro Gly Ser Ser Pro Gly Thr Pro Cys
   1 5 10
<210> 39
   <211> 11
   <212> PRT
   <213> Artificial
<220>
   <223> Synthetic peptide
<220>
   <221> MOD_RES
   <222> (5) .. (5)
   <223> PHOSPHORYLATION
<400> 39
   Ser Arg Ser Arg Thr Pro Ser Leu Pro Thr Pro
   1 5 10
<210> 40
   <211> 11
   <212> PRT
   <213> Artificial
<220>
   <223> Synthetic peptide
<220>
   <221> MOD_RES
   <222> (7)..(7)
   <223> PHOSPHORYLATION
<400> 40
   Ser Arg Ser Arg Thr Pro Ser Leu Pro Thr Pro
   1 5 10
<210> 41
   <211> 11
   <212> PRT
   <213> Artificial
<220>
   <223> Synthetic peptide
<220>
   <221> MOD_RES
   <222> (5) .. (5)
   <223> PHOSPHORYLATION
<220>
   <221> MOD_RES
   <222> (7)..(7)
   <223> PHOSPHORYLATION
<400> 41
   Ser Arg Ser Arg Thr Pro Ser Leu Pro Thr Pro
   1 5 10
<210> 42
   <211> 9
   <212> PRT
   <213> Artificial
<220>
   <223> Synthetic peptide
<220>
   <221> MOD_RES
   <222> (5) .. (5)
   <223> PHOSPHORYLATION
<400> 42
   Pro Ser Leu Pro Thr Pro Pro Thr Arg
   1 5
<210> 43
   <211> 11
   <212> PRT
   <213> Artificial
<220>
   <223> Synthetic pepide
<220>
   <221> MOD_RES
   <222> (7)..(7)
   <223> PHOSPHORYLATION
<400> 43
   Gly Cys Ser Pro Val Val Ser Gly Asp Thr Ser
   1 5 10
<210> 44
   <211> 8
   <212> PRT
   <213> Artificial
<220>
   <223> Synthetic peptide
<220>
   <221> MOD_RES
   <222> (4)..(4)
   <223> PHOSPHORYLATION
<400> 44
   Ser Asn Val Ser Ser Thr Gly Cys
   1 5
<210> 45
   <211> 19
   <212> PRT
   <213> Artificial
<220>
   <223> Synthetic peptide
<220>
   <221> MOD_RES
   <222> (9)..(9)
   <223> PHOSPHORYLATION
<400> 45
   Pro Arg His Leu Ser Asn Val Ser Ser Thr Gly Ser Ile Asp Met Val
   1               5                   10                  15
   Asp Ser Pro
<210> 46
   <211> 19
   <212> PRT
   <213> Artificial
<220>
   <223> Synthetic peptide
<400> 46
   Pro Arg His Leu Ser Asn Val Ser Ser Thr Gly Ser Ile Asp Met Val
   1               5                   10                  15
   Asp Ser Pro
<210> 47
   <211> 32
   <212> PRT
   <213> Artificial
<220>
   <223> Synthetic peptide
<220>
   <221> MOD_RES
   <222> (20)..(20)
   <223> PHOSPHORYLATION
<220>
   <221> MOD_RES
   <222> (28)..(28)
   <223> PHOSPHORYLATION
<400> 47
   Gly Cys Arg Glu Asn Ala Lys Ala Lys Thr Asp His Gly Ala Glu Ile
   1               5                   10                  15
   Val Tyr Lys Ser Pro Val Val Ser Gly Asp Thr Ser Pro Arg His Leu
               20                  25                  30
<210> 48
   <211> 32
   <212> PRT
   <213> Artificial
<220>
   <223> Synthetic peptide
<400> 48
   Gly Cys Arg Glu Asn Ala Lys Ala Lys Thr Asp His Gly Ala Glu Ile
   1               5                   10                  15
   Val Tyr Lys Ser Pro Val Val Ser Gly Asp Thr Ser Pro Arg His Leu
               20                  25                  30

## Description

### [Technical field]

The present invention relates to a therapeutic agent or prophylactic agent for cognitive disorders. More specifically, the invention relates to a novel anti-phosphorylated protein or peptide antibody having an excellent effect for improving cognitive function, and to a therapeutic agent or prophylactic agent for cognitive disorders comprising anti-phosphorylated tau antibody, or antigen that elicits anti-phosphorylated tau antibody.

### [Background Art]

A cognitive disorder is a state in which developed intelligence deteriorates due to some acquired cause, constituting a hindrance to social adaptation. Cognitive disorders are classified as neurodegenerative diseases, vascular cognitive disorders, prion diseases, infectious diseases, metabolic/endocrine disorders, trauma and cerebral disorders, and toxic disorders (NPL 1). As of 2010, there are currently about 2.1 million cognitive disorder patients in Japan, with a morbidity prevalence rate of about 8-10%, or even more than 10%, among the elderly over age 65, and this has been recognized as a serious problem in the worldwide aging society (NPL 2). The data on the underlying diseases of cognitive disorders indicate that the majority are neurodegenerative diseases such as AD and FTLD, with approximately 35% Alzheimer's disease (AD), approximately 15% a combination of AD and cerebrovascular disease, and 5% neurodegenerative diseases such as FrontoTemporal Lobar Degeneration (FTLD) (NPL 2). Cognitive disorder due to neurodegenerative disease is characterized by insidious onset of memory impairment and/or personality changes which progresses over a period of at least 6 months or more. A consistent factor in neurodegenerative processes exhibiting a high degree of correlation with the degree of impairment of cognitive function is the presence of neurofibrillary tangles (NFT) (NPL 3).

Tau (protein) is a protein encoded by the MAPT gene located on chromosome 17 (17q21) in humans, and it is one of the microtubule-binding proteins abundantly expressed in the central nervous system. Tau has been found to be a major constituent protein in the paired helical filaments and straight filaments forming NFT in AD, one of the most prominent neurodegenerative diseases, and its intracellular accumulation has been demonstrated in a variety of neuropathological conditions. The diseases caused by intracellular accumulation of tau are collectively referred to as "tauopathies" (NPL 4). The neurodegenerative diseases included among tauopathies are Alzheimer's disease (AD), cortical-basal ganglia degeneration (CBD or CBS), progressive supranuclear palsy, Pick's disease, (argyrophilic grain dementia (argyrophilic grain disease), Multiple system tauopathy with dementia (MSTD), chromosome 17-linked frontotemporal dementia with Parkinsonism (FTDP-17), neurofibrillary tangle dementia, diffuse neurofibrillary tangles with calcification (DNTC), white matter tauopathy with globular glial inclusions (WMT-GGI) and frontotemporal lobar degeneration with tau-positive inclusions (FTLD-tau), but non-neurodegenerative diseases, including infectious diseases such as von Economo's postencephalitic Parkinson's disease and subacute sclerosing panencephalitis, and trauma-induced conditions such as boxer's encephalopathy, are also included among tauopathies (NPL 4).

The structure of the MAPT gene on the genome is found to be a protein consisting of 13 exons, with multiple isoforms due to alternative splicing (NPL 4). A feature of the structure of tau is that it comprises an N-terminal acidic domain containing 0-2 repetitive sequences (N) of 29 amino acids depended on alternative splicing of exon 2 and exon 3 (N0-N2), an intermediate domain rich in proline, and a C-terminal microtubule-binding domain (encoded by exons 9 to 12) containing 3 (3R) or 4 (4R) repetitive sequences (R) that contribute to microtubule binding (NPL 3 and 4). Therefore, tau has 6 representative isoforms, 3R0N (352 amino acids) ·3R1N (381 amino acids) ·3R2N (410 amino acids) ·4R0N (383 amino acids) ·4R1N (412 amino acids) and 4R2N (441 amino acids), depending on the number of 29 amino acid repetitive sequences (N) and microtubule-binding repetitive sequences (R) that it contains. Of these isotypes, only 3R0N is present in the embryonic brain, whereas all 6 isotypes are present in the adult human brain, with the 4R type being most abundant (NPL 3). The difference between the 3R and 4R isotypes is whether exon 10 is removed by alternative splicing (3R) or present (4R). Several isoforms of tau exist, therefore, but the amino acid numbers (1-441) of the longest isoform 4R2N (SEQ ID NO: 1) are represented for identification of the amino acid numbers at corresponding positions. For example, the designation "Ser413" indicates the serine which is the 413th amino acid residue in 4R2N (SEQ ID NO: 1), although this serine is the 384th amino acid residue in 4R1N (SEQ ID NO: 2), the 355th in 4R0N (SEQ ID NO: 3), the 382nd in 3R2N (SEQ ID NO: 4), the 353rd in 3R1N (SEQ ID NO: 5), and the 324th in 3R0N (SEQ ID NO: 6).

Regarding the role of tau in neurodegenerative diseases, it was first discovered that a relationship exists between mutation of the MAPT gene and accumulation of tau in chromosome 17-linked frontotemporal dementia with Parkinsonism (FTDP-17), with more than 40 different gene mutations in the MAPT gene having been reported in FTDP-17 (NPL 4). It has been suggested that such gene mutations may lead to alterations in the proportion of tau isoforms and change the interaction of mutant tau to microtubules, thus contributing to establishment of pathology. However, unlike familial neurodegenerative diseases, mutations in MAPT are usually not found in sporadic neurodegenerative diseases such as AD. Furthermore, one of the features of accumulating tau in neurodegenerative diseases is a high degree of modification by phosphorylation. Moreover, in patients exhibiting mild cognitive function impairment (MCI), a correlation is seen between levels of phosphorylated tau in the spinal fluid and pituitary atrophy, suggesting phosphorylated tau as a highly reliable biomarker for neurodegeneration in patients with tauopathies (NPL 5). For this reason, it has been attempted to use enzyme inhibitors against kinases, and particularly GSK-3 beta, as enzymes involved in phosphorylation, in order to inhibit excessive phosphorylation of tau, and development has progressed in this area (NPL 5). However, because kinases such as GSK-3 beta are enzymes that are implicated not only in disease but also in function control in normal physiological processes, side-effects have been a source of concern. In fact, since some of the sites where tau is phosphorylated by GSK-3 beta coincide with the sites of tau phosphorylation seen in fetal and normal human brains (NPL 3), there are possibilities to affect normal tau function.

The conventional wisdom has been that extracellular tau leaks out of the cell as a consequence of cell death of degenerated neurons, but recent research has suggested that following excessive intracellular phosphorylation, tau is processed and is actively secreted out of the cell. Phosphorylated tau secreted out of the cell is thought to be dephosphorylated at certain phosphorylation sites, subsequently acting on muscarine receptors M1 and M3 of surrounding cells, and thus promoting intracellular tau phosphorylation and contributing to eliciting cell death (NPL 6, NPL 7). As consensus is building that tau functions as a factor with extracellular activity, there is increasing focus on its possibilities in therapeutic agents, using drug components that are macromolecules such as antibodies which cannot be easily caused to exhibit intracellular activity. However, as mentioned above, extracellularly secreted tau may be partially processed and may become dephosphorylated, potentially undergoing further modification beyond the structural information for excessively phosphorylated tau that has been targeted in the past. It has also been suggested that drugs that act on portions of dephosphorylated tau may affect the function of normal tau. When pathology-associated tau is to be targeted with antibodies or the like, it is even more important to select the entity that will act on a given pathology-specific site, i.e. tau phosphorylation epitope, and selection of the epitope becomes even more difficult due to the complexity of this information.

Inventions relating to immunotherapy for tauopathies with tau protein as the target have been reported, being aimed at executing specific action against tau (NPL 5, PTL 1, PTL 2, PTL 3). Immunotherapy is conducted with the purpose of eliciting production of specific antibodies by administration of peptide vaccines and the like, and it is expected to have reduced side-effects due to its high specificity to target proteins or peptides. It has been reported that motor function is improved in animal models expressing mutant tau, by immunization of the model animals by vaccination using partial peptides of phosphorylated tau (having the amino acid residues corresponding to Ser396 and Ser404 phosphorylated, and having the amino acid residue corresponding to Ser262 phosphorylated). However, these reports are studies using transgenic mice (Tg mice) with induced gene mutations such as P301L (a mutation from proline to leucine at the 301st amino acid residue of tau), and although such Tg mice serve as gene mutation models for FTDP-17, a type of familial neurodegenerative disease, they do not represent most neurodegenerative diseases among tauopathies that are not accompanied by tau gene mutations, and particularly sporadic neurodegenerative diseases. In addition, since P301Ltg mice are models of motor function impairment and are not models representing cognitive function impairment, which is the problem with human cognitive disorders (NPL 8), it is difficult to know whether the results in these animal models can be applied to treatment of human cognitive disorders. In PTL 4, the effect of tauopathy treatment is examined, administering an antibody that participates in antigen-antibody reaction with tau peptide having Ser409 phosphorylated. However, peptide vaccines are costly, require high total dosages and require long periods to exhibit their effects. Furthermore, the effects of peptide vaccines and the reactivity of the immune response in administered humans and animals differ according to genetic background, and effective antibody production cannot always be elicited in every individual. Therefore, while immunotherapy by passive immunization with antibodies has potential, a very large number of sites are phosphorylated in tau, and virtually no information exists regarding which antibodies for which phosphorylation sites are effective to use. In addition, the currently available antibodies cannot at all be considered to have sufficient function for use in therapy, based on their effects in animal models.

Furthermore, when an antibody is to be used as a base compound for a therapeutic agent or prophylactic agent it is necessary to also consider the amount of antibody used for treatment, in order to avoid side-effects and minimize problems of medical cost, and this is especially important in relation to doses for chronic diseases or genetic diseases. For example, the dose for treatment with Actemra^{R} (tocilizumab), which is human anti-IL-6R antibody, is 8 mg per 1 kg of body weight for 1 to 4 weeks, and the dose for treatment with Soliris^{R} (eculizumab), which is humanized anti-complement C5 antibody, is 600-900 mg per adult per administration, for 2 to 4 weeks. These are superior antibodies developed by selection from among a large number of antibodies, but their dosages are relatively large compared to the currently used antibody drugs. Thus, an effect must be exhibited with dosages equal to or less than these, for antibody drugs that will be developed in the future. In addition, while the brain is the organ to be treated in cognitive disorders such as AD, systemic administration by intravenous or subcutaneous routes is generally thought to result in a low migration rate of antibody from the blood to the brain, due to the presence of the blood-brain barrier, and therefore antibodies used for treatment of cognitive disorders are expected to have lower pharmacological effects compared to treatment for diseases involving other organs, and this has constituted a major problem.

The major symptoms in human cognitive disorders are memory impairment and cognitive function impairment, and since cognitive function is especially important for exhibiting memory-based judgment, communication and performance, the symptoms of cognitive disorders are of major importance. Motor function, on the other hand, while being a symptom found in chromosome 17-linked frontotemporal dementia with Parkinsonism (FTDP-17) and end-stage Alzheimer's disease, is not necessarily a major symptom exhibited in cognitive disorders. Consequently, the main issue to be considered for treating cognitive disorders is improvement of cognitive function. At the current time, however, there is no means of obtaining a therapeutic agent or prophylactic agent for cognitive disorders that exhibits a superior improvement on cognitive function, using suitable animal models for taupathy-associated cognitive function impairment which are necessary to solve the problem outlined above, nor does any therapeutic agent or prophylactic agent for cognitive disorders exist that exhibits a specific and superior effect against cognitive disorders.

WO2010/144711 discloses a peptide corresponding to residues 398-416 of tau wherein the Ser at positions 412 and 413 are phosphorylated. Also disclosed are antibodies that bind to the peptide.

US2002/0086009 discloses a number of peptides which partially overlap the region 410-421 of tau. These peptides are phosphorylated at positions 412 and/or 413 and used to generate antibodies for detecting Alzheimer's disease.

Boutanjagout et al 2011 discloses the use of the PHF1 antibody, which targets tau that is phosphorylated on serin amino acids 396 and/or 404, for the passive immunization of a Alzheimer's disease mouse model.

In light of these circumstances, therefore, a demand exists for a therapeutic agent or prophylactic agent with a powerful effect for improving cognitive function.

### Citation List

### Patent Literature

[PTL 1] US8012936
[PTL 2] WO2010/142423
[PTL 3] WO2010/144711

### [Non-patent literature]

[NPL 1] Kishimoto, T., Takahashi, S., STEP Series Seishinka, 2th Edition, P.103-104, Kaibashobo, 2008
[NPL 2] Asada, T., Igaku no Ayumi, supplementary volume, "Cognitive disorders", p.5-10, Ishiyaku Publishing, 2011
[NPL 3] Alistair Burns, John O'Brien and David Ames, Dementia. 3rd Edition, P.408-464, 2005
[NPL 4] Arai, T., Shinkei Naika, Vol.72, special number, (Suppl.6), P.46-51, 2010
[NPL 5] Wendy Noble et al., Expert Opin. Drug Discov., Vol.6, No.8, P.797-810, 2011
[NPL 6] Miguel Diaz-Hernandes et al., Journal of Biological Chemistry Vol.285, p.32539-32548, 2010
[NPL 7] Venessa Plouffe et al., PLoS ONE Vol.7, p36873, 2012
[NPL 8] Alistair Burns, John O'Brien and David Ames, Dementia. 3rd Edition, P.459, 2005

### DISCLOSURE OF THE INVENTION

### Problems to be Solved by the Invention

It is an object of the present invention to provide a therapeutic agent or prophylactic agent for cognitive disorders, focusing on tau phosphorylation in tauopathies.

It is another object of the invention to provide a therapeutic agent or prophylactic agent for cognitive disorders comprising as an active ingredient an antibody that participates in specific antigen-antibody reaction for tau phosphorylated on an amino acid residue corresponding to the vicinity of Ser413, or a peptide having the amino acid sequence in the vicinity of Ser413 and phosphorylated on at least one amino acid residue.

It is yet another object of the invention to provide a monoclonal antibody having a high cognitive function-improving effect, and a method of preparing an antibody that is even more suitable for treatment of cognitive disorder, such as a humanized antibody, based on analysis of the structure of the monoclonal antibody.

### Means for Solving the Problems

The present invention is as described below. The tau protein of the invention includes not only 4R2N, but all 6 types of isoforms. For convenience, the positions of the amino acid residues according to the invention are identified based on SEQ ID NO: 1, and for example, if the amino acid residue corresponding to Ser413 of SEQ ID NO: 1 is mentioned, this refers to the 413th serine of SEQ ID NO: 1 (4R2N), or the serine which is the 384th amino acid residue of SEQ ID NO: 2 (4R1N), the 355th of SEQ ID NO: 3 (4R0N), the 382nd of SEQ ID NO: 4 (3R2N), the 353rd of SEQ ID NO: 5 (3R1N) or the 324th of SEQ ID NO: 6 (3R0N).

In a first aspect, the invention provides a therapeutic agent or prophylactic agent for use in a method of treatment or prevention of a tauopathy, wherein the therapeutic agent or prophylactic agent comprises, as an active ingredient, a monoclonal antibody that binds specifically to a tau protein phosphorylated at the amino acid residue corresponding to Ser413 of SEQ ID NO: 1, wherein the binding is defined by affinity represented by an equilibrium dissociation constant (KD) of at most 1 x 10⁻⁶ M, and wherein said binding to the phosphorylated tau protein is at least 10 times stronger than the binding to a tau protein that has not been phosphorylated at said Ser413.

In one embodiment, the antibody comprises: a heavy chain variable region (VH) comprising a CDR-H1 having a sequence selected from SEQ ID NO: 7 or 8; a CDR-H2 having a sequence selected from SEQ ID NO: 9, 10, 11 or 12; and a CDR-H3 having the sequence represented by SEQ ID NO: 13; and a light chain variable region (VL) comprising a CDR-L1 having a sequence selected from SEQ ID NO: 14 or 15; a CDR-L2 having the sequence represented by SEQ ID NO: 16; and a CDR-L3 having the sequence represented by SEQ ID NO: 17.

In another embodiment, the antibody comprises: a VH comprising a CDR-H1 having the sequence represented by SEQ ID NO: 8; a CDR-H2 having a sequence at least 85% identical to the sequence represented by SEQ ID NO: 9; and a CDR-H3 having the sequence represented by SEQ ID NO: 13; and a VL comprising a CDR-L1 having a sequence at least 85% identical to the sequence represented by SEQ ID NO: 14; a CDR-L2 having the sequence represented by SEQ ID NO: 16; and a CDR-L3 having the sequence represented by SEQ ID NO: 17.

In another embodiment, the antibody comprises: a VH comprising a CDR-H1 having the sequence represented by SEQ ID NO: 8; a CDR-H2 having the sequence represented by SEQ ID NO: 9; and a CDR-H3 having the sequence represented by SEQ ID NO: 13; and a VL comprising a CDR-L1 having the sequence represented by SEQ ID NO: 14; a CDR-L2 having the sequence represented by SEQ ID NO: 16; and a CDR-L3 having the sequence represented by SEQ ID NO: 17.

In another embodiment, the antibody comprises: a VH having a sequence at least 85% identical to the sequence represented by SEQ ID NO: 20; and a VL having a sequence at least 85% identical to the sequence represented by SEQ ID NO: 26. In a further embodiment, the antibody comprises: a VH having the sequence represented by SEQ ID NO: 20; and a VL having the sequence represented by SEQ ID NO: 26.

In another embodiment, the antibody is a humanized antibody or a chimeric antibody.

In another embodiment, the antibody binding to the tau protein phosphorylated at the amino acid residue corresponding to Ser413 of SEQ ID NO: 1 is at least 30 times, or at least 100 times stronger than the binding to a tau protein that has not been phosphorylated at said Ser413. In another embodiment, the antibody binds to the tau protein phosphorylated at the amino acid residue corresponding to Ser413 of SEQ ID NO: 1 with an affinity represented by an equilibrium dissociation constant (KD) of at most 1x10⁻⁷M or at most 1x10⁻⁸M. In another embodiment, the binding affinity of the antibody is determined by a binding measurement using surface plasmon resonance.

In a second aspect, the invention provides a therapeutic agent or prophylactic agent for use in a method of treatment or prevention of a tauopathy containing, as an active ingredient, a peptide comprising a portion of tau protein, wherein said peptide comprises the amino acids 410-421 of SEQ ID NO: 1, wherein the amino acid residue corresponding to Ser413 is phosphorylated.

In one embodiment, the tauopathy is Alzheimer's disease, cortical-basal ganglia degeneration, progressive supranuclear palsy, Pick's disease, argyrophilic grain dementia (argyrophilic grain disease), Multiple system tauopathy with dementia (MSTD), chromosome 17-linked frontotemporal dementia with Parkinsonism (FTDP-17), neurofibrillary tangle dementia, diffuse neurofibrillary tangles with calcification (DNTC), white matter tauopathy with globular glial inclusions (WMT-GGI) or frontotemporal lobar degeneration with tau-positive inclusions (FTLD-tau).

The following items are disclosed herein:
(1) A therapeutic agent or prophylactic agent for cognitive disorders comprising, as an active ingredient, an antibody that participates in antigen-antibody reaction with tau protein that has been phosphorylated on at least one amino acid residue corresponding to positions 410 to 421 of the tau protein represented by SEQ ID NO: 1.
(2) The therapeutic agent or prophylactic agent for cognitive disorders according to (1), wherein the antibody is an antibody that participates in antigen-antibody reaction with phosphorylated tau protein characteristic of cognitive disorders.
(3) The therapeutic agent or prophylactic agent for cognitive disorders according to (1) or (2), wherein the antibody participates in antigen-antibody reaction with tau protein that is phosphorylated on one or more sites selected from among Ser412, Ser413, Thr414 and Ser416.
(4) The therapeutic agent or prophylactic agent for cognitive disorders according to any one of (1) to (3), wherein the antibody is an antibody which, in binding with tau protein, binds in competition with an antibody including VH consisting of the amino acid sequence listed as SEQ ID NO: 20 and VL consisting of the amino acid sequence listed as SEQ ID NO: 26.
(5) The therapeutic agent or prophylactic agent for cognitive disorders according to any one of (1) to (3), wherein the antibody is an antibody including VH consisting of the amino acid sequence listed as SEQ ID NO: 20 and VL consisting of the amino acid sequence listed as SEQ ID NO: 26.
(6) The therapeutic agent or prophylactic agent for cognitive disorders according to any one of (1) to (4), wherein the antibody is an antibody that participates in antigen-antibody reaction with tau protein that is phosphorylated on the amino acid residue corresponding to the Ser413 site.
(7) The therapeutic agent or prophylactic agent for cognitive disorders according to any one of (1) to (6), wherein the antibody is an antibody comprising a CDR sequence on the H chain represented by SEQ ID NOs: 7 to 13, a CDR sequence on the H chain represented by at least one of SEQ ID NOs: 7 to 13 or a CDR sequence on the H chain having at least 85% homology with at least one CDR sequence on the H chain represented by SEQ ID NOs: 7 to 13, and/or a CDR sequence on the L chain represented by SEQ ID NOs: 14 to 17, a CDR sequence on the L chain represented by at least one of SEQ ID NOs: 14 to 17 or a CDR sequence on the L chain having at least 85% homology with at least one CDR sequence on the L chain represented by SEQ ID NOs: 14 to 17.
(8) The therapeutic agent or prophylactic agent for cognitive disorders according to any one of (1) to (7), wherein the antibody is an antibody comprising an H chain variable region represented by any one of SEQ ID NOs: 18 to 24 or an H chain variable region containing a sequence having at least 85% homology with any one of SEQ ID NOs: 18 to 24, and/or an L chain variable region represented by any one of SEQ ID NOs: 25 to 30 or an L chain variable region containing a sequence having at least 85% homology with any one of SEQ ID NOs: 25 to 30.
(9) The therapeutic agent or prophylactic agent for cognitive disorders according to any one of (1) to (8), wherein the antibody is a humanized antibody or chimeric antibody.
(10) A therapeutic agent or prophylactic agent for cognitive disorders containing, as an active ingredient, a peptide that includes a sequence of at least 8 contiguous amino acids from the amino acid sequence consisting of the amino acid residues corresponding to amino acid numbers 410-421 of SEQ ID NO: 1, at least one of the amino acid residues in the peptide being phosphorylated.
(11) The therapeutic agent or prophylactic agent for cognitive disorders according to (10), wherein at least one of the phosphorylated amino acid residues in the peptide corresponds to amino acid residue Ser412, Ser413, Thr414 or Ser416 of SEQ ID NO: 1.
(12) The therapeutic agent or prophylactic agent for cognitive disorders according to (10) or (11), wherein the phosphorylated amino acid residues in the peptide include at least the amino acid residue corresponding to Ser413 of SEQ ID NO: 1.
(13) The therapeutic agent or prophylactic agent for cognitive disorders according to any one of (1) to (12), wherein the cognitive disorder is a tauopathy.
(14) The therapeutic agent or prophylactic agent for cognitive disorders according to (13), wherein the tauopathy is Alzheimer's disease, cortical-basal ganglia degeneration, progressive supranuclear palsy, Pick's disease, argyrophilic grain dementia (argyrophilic grain disease), Multiple system tauopathy with dementia (MSTD), chromosome 17-linked frontotemporal dementia with Parkinsonism (FTDP-17), neurofibrillary tangle dementia, diffuse neurofibrillary tangles with calcification (DNTC), white matter tauopathy with globular glial inclusions (WMT-GGI) or frontotemporal lobar degeneration with tau-positive inclusions (FTLD-tau).
(15) A monoclonal antibody that participates in antigen-antibody reaction with a peptide comprising a sequence of at least 8 contiguous amino acids from the amino acid sequence consisting of amino acid numbers 410-421 of SEQ ID NO: 1, the amino acid residue corresponding to Ser413 of SEQ ID NO: 1 in the peptide being phosphorylated.
(16) An antibody for phosphorylated tau protein, the antibody being one whose binding to antigen is competitive against an antibody including VH consisting of the amino acid sequence listed as SEQ ID NO: 20 and VL consisting of the amino acid sequence listed as SEQ ID NO: 26.
(17) An antibody for phosphorylated tau protein, the antibody being one including VH consisting of the amino acid sequence listed as SEQ ID NO: 20 and VL consisting of the amino acid sequence listed as SEQ ID NO: 26.
(18) A monoclonal antibody having a CDR sequence on the H chain represented by SEQ ID NOs: 7 to 13, a CDR sequence on the H chain represented by at least one of SEQ ID NOs: 7 to 13, or an H chain including in the CDR sequence having at least 85% homology with at least one CDR sequence on the H chain represented by SEQ ID NOs: 7 to 13, and/or a CDR sequence on the L chain represented by SEQ ID NOs: 14 to 17, a CDR sequence on the L chain represented by at least one of SEQ ID NOs: 14 to 17, or an L chain including in the CDR sequence having at least 85% homology with at least one CDR sequence on the L chain represented by SEQ ID NOs: 14 to 17.
(19) A monoclonal antibody comprising an H chain variable region represented by any one of SEQ ID NOs: 18 to 24 or an H chain variable region having at least 85% homology with any one of SEQ ID NOs: 18 to 24, and/or an L chain variable region represented by any one of SEQ ID NOs: 25 to 30 or an L chain variable region having at least 85% homology with any one of SEQ ID NOs: 25 to 30.
(20) The monoclonal antibody according to any one of (15) to (19), wherein the antibody is a humanized antibody or chimeric antibody.
(21) A peptide consisting of a sequence of at least 8 contiguous amino acids from among the amino acid sequence consisting of the amino acid residues corresponding to amino acid numbers 410-421 of SEQ ID NO: 1, at least one of the amino acid residues in the peptide being phosphorylated.
(22) The peptide according to (21), wherein at least one of the amino acid residues corresponding to amino acids Ser412, Ser413, Thr414 and Ser416 of SEQ ID NO: 1 is phosphorylated.
(23) The peptide according to (21) or (22), wherein the phosphorylated amino acid residue is the amino acid residue corresponding to Ser413 of SEQ ID NO: 1.

### Effect of the Invention

The present invention provides a therapeutic agent or prophylactic agent for cognitive disorders, containing as an active ingredient, an antibody that participates in antigen-antibody reaction specifically with phosphorylated tau at the amino acid residue corresponding to Ser413 of SEQ ID NO: 1 wherein the binding is defined by affinity represented by a KD of at most 1 x 10⁻⁶ M, and wherein said binding to the phosphorylated tau protein is at least 10 times stronger than the binding to a tau protein that has not been phosphorylated at said Ser413; or a peptide including the amino acids 410-421 of SEQ ID NO: 1, with the amino acid residue Ser413 being phosphorylated. Also provided herein is a monoclonal antibody having a high cognitive function-improving effect, and a method of preparing an antibody that is even more suitable for treatment of cognitive disorder, such as a humanized antibody, based on analysis of the structure of the monoclonal antibody.

### Brief Description of the Drawings

Fig. 1 is a listing of the initial portions of amino acid sequences of isoforms of human tau protein, aligned using ClustalW.
Fig. 2 is a listing of the latter portions of amino acid sequences of isoforms of human tau protein, aligned using ClustalW.
Fig. 3 is a graph showing specificity of rabbit polyclonal antibody for pSer413 peptide.
Fig. 4 is a graph showing the results of a Trial test in model mice administered pSer413-recognizing rabbit polyclonal antibody.
Fig. 5 is a graph showing the results of a Probe test in model mice administered pSer413-recognizing rabbit polyclonal antibody.
Fig. 6 is a line graph showing the results of an Open Field test in model mice administered pSer413-recognizing rabbit polyclonal antibody.
Fig. 7 is a bar graph showing the results of an Open Field test in model mice administered pSer413-recognizing rabbit polyclonal antibody.
Fig. 8 is a graph showing the results of a Trial test in model mice administered pSer413-recognizing mouse monoclonal antibody (Ta1505).
Fig. 9 is a graph showing the results of a Probe test in model mice administered pSer413-recognizing mouse monoclonal antibody (Ta1505).
Fig. 10 is a line graph showing the results of an Open Field test in model mice administered pSer413-recognizing mouse monoclonal antibody (Ta1505).
Fig. 11 is a bar graph showing the results of an Open Field test in model mice administered pSer413-recognizing mouse monoclonal antibody (Ta1505).
Fig. 12 is a graph showing the results of a Trial test in model mice administered pSer396-recognizing mouse monoclonal antibody (Ta9).
Fig. 13 is a graph showing the results of a Probe test in model mice administered pSer396-recognizing mouse monoclonal antibody (Ta9).
Fig. 14 is a line graph showing the results of an Open Field test in model mice administered pSer396-recognizing mouse monoclonal antibody (Ta9).
Fig. 15 is a bar graph showing the results of an Open Field test in model mice administered pSer396-recognizing mouse monoclonal antibody (Ta9).
Fig. 16 is a bar graph showing hippocampal synaptophysin levels in model mice administered Ta1505 antibody.
Fig. 17 is a diagram representing a gene fragment containing the tau gene.
Fig. 18 is a graph showing the results of a Water Maze Trial test in memory learning-impaired mice (Tau-Tg) administered Ta1505 antibody.
Fig. 19 is a graph showing the results of a Water Maze Probe test in memory learning-impaired mice (Tau-Tg) administered Ta1505 antibody.
Fig. 20 is a graph showing the results of a Water Maze Trial test in memory learning-impaired mice (Tau-Tg) administered Ta9 antibody.
Fig. 21 is a graph showing the results of a Water Maze Probe test in memory learning-impaired mice (Tau-Tg) administered Ta9 antibody.
Fig. 22 is a photograph showing immunohistostaining of hippocampus CA3 region and CA23 region with Ta1505 antibody in memory learning-impaired mice (Tau-Tg) administered control IgG (1mg/head) or Ta1505 antibody (1mg/head).
Fig. 23 is a photograph showing immunohistostaining of parahippocampal gyrus region with Ta1505 in memory learning-impaired mice (Tau-Tg) administered control IgG (1mg/head).
Fig. 24 is a photograph showing immunohistostaining of parahippocampal gyrus region with Ta1505 antibody in memory learning-impaired mice (Tau-Tg) administered Ta1505 antibody (1mg/head).
Fig. 25 is a photograph showing immunohistostaining of hippocampus CA3 region and CA23 region with AT8 in memory learning-impaired mice (Tau-Tg) administered control IgG (1mg/head) or Ta1505 antibody (1mg/head).
Fig. 26 is a photograph showing immunohistostaining of parahippocampal gyrus region with AT8 in memory learning-impaired mice (Tau-Tg) administered control IgG (1mg/head).
Fig. 27 is a photograph showing immunohistostaining of parahippocampal gyrus region with AT8 in memory learning-impaired mice (Tau-Tg) administered Ta1505 (1mg/head).
Fig. 28 is a graph showing quantity of G2, AT8, PHF1, and Ta1505 reactive tau in TBS soluble fraction of brain in memory learning-impaired mice (Tau-Tg) administered Ta1505 (1mg/head).
Fig. 29 is a graph showing quantity of G2, AT8, PHF1, and Ta1505 reactive tau in sarkosyl soluble fraction of brain in memory learning-impaired mice (Tau-Tg) administered Ta1505 (1mg/head).

### Best Mode for Carrying Out the Invention

The present inventors have prepared antibodies that participate in antigen-antibody reaction specifically with tau that has been phosphorylated at the amino acid residue corresponding to Ser413 of SEQ ID NO: 1, which is the site specifically phosphorylated in AD, have administered it to Tg mice exhibiting maturation-associated cognitive function impairment, and have found that cognitive function can be recovered to approximately the same level as a control group. On the other hand, adequate amelioration of cognitive function was not seen using, for comparison, a comparable concentration of antibody for tau that has been phosphorylated at the amino acid residue corresponding to Ser396 of SEQ ID NO: 1, which has higher affinity for equivalent antigen than the antibody of the invention. The portion in the vicinity of the amino acid residue corresponding to Ser413 of SEQ ID NO: 1 is a region for which no particular information is known in terms of the relationship between tau structure and function, and it was a completely unexpected result to find that an antibody that participates in antigen-antibody reaction specifically with this portion has such a powerful improving effect on cognitive function. Thus, the site in the vicinity of the amino acid residue corresponding to Ser413 of SEQ ID NO: 1, which has not hitherto been a focus of interest, has first been elucidated by the present invention as an important site for onset of cognitive function impairment in tauopathies, and the invention has thereupon been completed.

### [Anti-phosphorylated tau antibody]

Tau (protein), for the purpose of the invention, includes not only human tau protein as represented by SEQ ID NO: 1-6, but also genetic mutants thereof. As explained above under Background Art, more than 40 mutations are known in FTDP-17, a familial neurodegenerative disease associated with cognitive disorder, but the mutation sites are not necessarily limited thereto. Furthermore, proteins with mutations at amino acids in 1 to 50 locations, preferably 1 to 30 locations and more preferably 1 to 10 locations, as the number of mutations in SEQ ID NOs: 1-6, are also treated as tau for the purpose of the invention. In addition, proteins exhibiting at least 80% homology (Identity) with the human tau protein listed as SEQ ID NO: 1 according to the BLAST method (default conditions for NCBI PBLAST), and their isoforms, are also included. Such proteins also include tau of species other than humans, such as chimpanzee, rhesus monkey, horse, pig, dog, mouse, rabbit and rat, and can be used to prepare therapeutic agents or prophylactic agent targeting those tau proteins, for the purpose of ameliorating cognitive function in those animals.

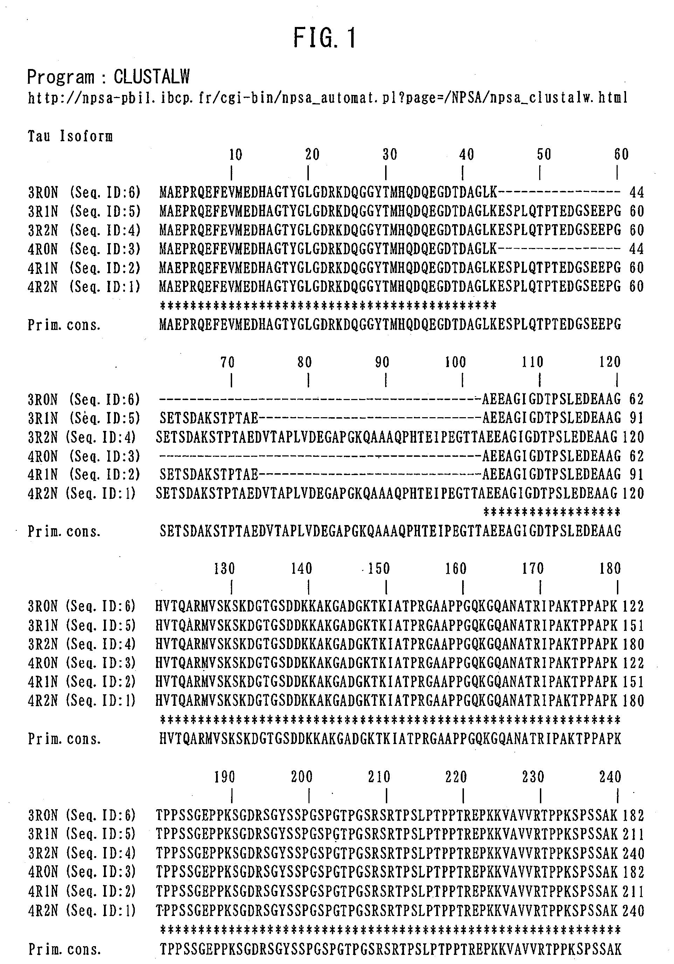

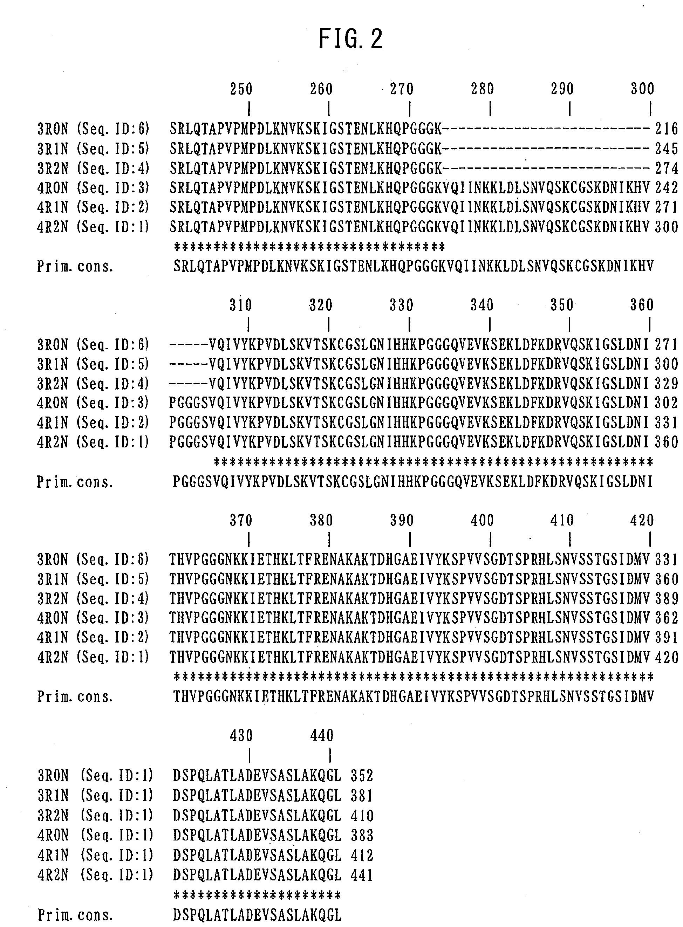

The amino acid numbers according to the invention, i.e. the positions of the amino acid residues, are designated based on the sequence listed as SEQ ID NO: 1, for convenience. Thus, for example, if the amino acid residue corresponding to Ser413 of SEQ ID NO: 1 is mentioned, this refers to the serine which is the 413th amino acid residue of SEQ ID NO: 1 (4R2N), the 384th of SEQ ID NO: 2 (4R1N), the 355th of SEQ ID NO: 3 (4R0N), the 382nd of SEQ ID NO: 4 (3R2N), the 353rd of SEQ ID NO: 5 (3R1N) or the 324th of SEQ ID NO: 6 (3R0N). Table 1 shows the positions of the amino acid residues that are in the same mutual positions, for each isoform. In Table 1, the positions of the amino acid residues for each isoform are shown corresponding to 410-421 of SEQ ID NO: 1, and the mutual positional relationships of the amino acid residues at other positions will be easily understood based on Fig. 1 and Fig. 2, for example.

**[Table 1]**

| Isoform | 4R2N | 4R1N | 4R0N | 3R2N | 3R1N | 3R0N |
|---|---|---|---|---|---|---|
| Seq.ID | Seq. ID:No.1 | Seq. ID:No.2 | Seq. ID:No.3 | Seq. ID:No.4 | Seq. ID:No.5 | Seq. ID:No.6 |
| Amino Asid Residue | Position of Amino Acid Residue | | | | | |
| Asn | 410 | 381 | 352 | 379 | 350 | 321 |
| Val | 411 | 382 | 353 | 380 | 351 | 322 |
| Ser | 412 | 383 | 354 | 381 | 352 | 323 |
| Ser | 413 | 384 | 355 | 382 | 353 | 324 |
| Thr | 414 | 385 | 356 | 383 | 354 | 325 |
| Gly | 415 | 386 | 357 | 384 | 355 | 326 |
| Ser | 416 | 387 | 358 | 385 | 356 | 327 |
| Ile | 417 | 388 | 359 | 386 | 357 | 328 |
| Asp | 418 | 389 | 360 | 387 | 358 | 329 |
| Met | 419 | 390 | 361 | 388 | 359 | 330 |
| Val | 420 | 391 | 362 | 389 | 360 | 331 |
| Asp | 421 | 392 | 363 | 390 | 361 | 332 |

The term "anti-phosphorylated tau antibody" refers to an antibody that participates in antigen-antibody reaction with tau that has been phosphorylated on an amino acid residue at one or more locations of the amino acid sequence of tau referred to above. The phosphorylated amino acid residues may be serine (Ser), threonine (Thr), tyrosine (Tyr), or the like. Also, the site on phosphorylated tau in which the anti-phosphorylated tau antibody of the invention participates in antigen-antibody reaction is the site that is specifically phosphorylated in neurodegenerative diseases such as AD. In addition, the site of phosphorylated tau where an anti-phosphorylated tau antibody participates in antigen-antibody reaction may be selected from among the amino acid residues corresponding to Ser412, Ser413, Thr414 and Ser416 of SEQ ID NO: 1. In particular cases, an antibody may participate in antigen-antibody reaction with tau that has been phosphorylated at an amino acid residue corresponding to Ser412 or Ser413 of SEQ ID NO: 1, or both sites. The antibody of the invention participates in antigen-antibody reaction with tau that has been phosphorylated at the site of the amino acid residue corresponding to Ser413 of SEQ ID NO: 1. Here, an amino acid residue corresponding to Ser412, Ser413, Thr414 or Ser416 of SEQ ID NO: 1 refers to the site corresponding to the amino acid number in human 4R2N tau (SEQ ID NO: 1), and as explained under Background Art, the corresponding site in isoforms, or the corresponding site in non-human homologs, is treated in the same manner regardless of the amino acid number assigned from that amino acid sequence. The corresponding sites in isoforms or homologs can be determined by a person skilled in the art through appropriate analysis by a Pairwise Sequence Alignment method such as the Needleman-Wunsch method or Smith-Waterman method, or by Multiple Sequence Alignment such as the ClustalW method or PRRP method. An example of an analysis method of a corresponding site is shown in Fig. 1 and Fig. 2, with the amino acid sequences (single-letter representation) of 6 different isoforms in humans aligned using ClustalW. As seen here, the structure in the vicinity of the amino acid residues corresponding to Ser412, Ser413, Thr414 and Ser416 of SEQ ID NO: 1 is conserved in the 6 isoforms, and it can be easily discerned which are the corresponding amino acids.

An anti-phosphorylated tau antibody that can be used in a therapeutic agent or prophylactic agent as provided herein is an antibody that participates in antigen-antibody reaction specifically with tau protein that has been phosphorylated on at least one amino acid residue present from position 410 to position 421 of the tau protein of SEQ ID NO: 1, preferably it is an antibody that participates in antigen-antibody reaction specifically with tau protein that has been phosphorylated at one or more sites selected from among the amino acid residues corresponding to Ser412, Ser413, Thr414 and Ser416 of SEQ ID NO: 1, more preferably it is an antibody that binds competitively against an antibody whose VH amino acid sequence is SEQ ID NO: 20 and whose VL amino acid sequence is SEQ ID NO: 26 (hereunder referred to as "1505 antibody"). An antibody of the invention participates in antigen-antibody reaction specifically with tau protein that has been phosphorylated at the Ser413 site. Tau protein that has been phosphorylated on at least one amino acid residue corresponding to positions 410 to 421 of SEQ ID NO: 1, tau protein that has been phosphorylated at one or more sites selected from among the amino acid residues corresponding to Ser412, Ser413, Thr414 and Ser416 of SEQ ID NO: 1, or tau protein that has been phosphorylated at the site of the amino acid residue corresponding to Ser413 of SEQ ID NO: 1, is tau protein that has been phosphorylated at the corresponding site including isotypes of human or other species, as mentioned above, but more preferably it is human tau protein, and even more preferably it is any of the 6 isoforms in humans.

In regard to Tau protein that has been phosphorylated on at least one amino acid residue corresponding to positions 410 to 421 of SEQ ID NO: 1, tau protein that has been phosphorylated at one or more sites selected from among the amino acid residues corresponding to Ser412, Ser413, Thr414 and Ser416 of SEQ ID NO: 1, or tau protein that has been phosphorylated at the site of the amino acid residue corresponding to Ser413 of SEQ ID NO: 1, these phosphorylated tau proteins include peptides which have complete homology with portions of the amino acid sequences of the amino acid residues corresponding to positions 410 to 421 of tau protein, or homology with at least 80% of the sequences, and are phosphorylated at these amino acid residues, and an antibody that participates in antigen-antibody reaction specifically with such a peptide is also an anti-phosphorylated tau antibody.

As disclosed herein "participates in antigen-antibody reaction" means binding with tau protein that has been phosphorylated on at least one amino acid residue corresponding to positions 410 to 421 of SEQ ID NO: 1, tau protein that has been phosphorylated at one or more sites selected from among the amino acid residues corresponding to Ser412, Ser413, Thr414 and Ser416 of SEQ ID NO: 1. According to the invention, "participates in antigen-antibody reaction" means binding with tau protein that has been phosphorylated at the site of the amino acid residue corresponding to Ser413 of SEQ ID NO: 1, with affinity represented by an equilibrium dissociation constant (KD) of at most 1 × 10⁻⁶ M, preferably binding with affinity represented by an equilibrium dissociation constant of at most 1 × 10⁻⁷ M, and even more preferably binding with affinity represented by an equilibrium dissociation constant of at most 1 × 10⁻⁸ M.

The term "specifically" means that the binding with tau protein that has been phosphorylated on at least one amino acid residue corresponding to positions 410 to 421 of SEQ ID NO: 1, tau protein that has been phosphorylated at one or more sites selected from among the amino acid residues corresponding to Ser412, Ser413, Thr414 and Ser416 of SEQ ID NO: 1, and/or tau protein that has been phosphorylated at the site of the amino acid residue corresponding to Ser413 of SEQ ID NO: 1, is in a state which is at least 10 times stronger, more preferably at least 30 times stronger and even more preferably at least 100 times stronger, than binding with the tau protein that has not been phosphorylated at that site (including peptides having complete homology with a portion of the amino acid sequence of the tau protein, or having homology with at least 80% of the sequence).

In addition, "binds competitively" with an antibody whose VH amino acid sequence is SEQ ID NO: 20 and whose VL amino acid sequence is SEQ ID NO: 26 (1505 antibody) means a phenomenon in which, when another antibody is copresent with the monoclonal antibody during binding to antigen, binding of the monoclonal antibody is inhibited, and generally speaking, this can be measured by measuring the amount of addition (concentration) at which the binding amount of the monoclonal antibody to antigen is reduced when a different antibody is added in varying amount (concentration) with respect to a fixed amount (concentration) of the monoclonal antibody, the degree of inhibition being expressed as the value IC₅₀ or Ki. An antibody that binds competitively with an antibody whose VH amino acid sequence is SEQ ID NO: 20 and whose VL sequence is SEQ ID NO: 26 (1505 antibody) is one that has an IC₅₀ value of lower than 1 µM, more preferably lower than 100 nM and even more preferably lower than 10 nM, when antigen-antibody binding has been detected using 10 nM of the monoclonal antibody.

The antibody-antigen binding of such an antibody with phosphorylated tau protein can be determined by appropriate binding measurement by a person skilled in the art using a solid phase or liquid phase system, with a method such as ELISA, EIA, surface plasmon resonance, FRET, LRET or the like, although there is no limitation to these. When measuring such antigen-antibody binding, the antibody and/or antigen (phosphorylated tau protein or tau protein) is labeled with an enzyme, fluorescent substance, luminescent substance, radioactive isotope or the like, and a measuring method suitable for the physical and/or chemical properties of the labeled substance is used to allow detection of the antigen-antibody reaction.

The anti-phosphorylated tau antibody of the invention also includes an antibody wherein the H chain variable region contains the amino acid sequences CDR-H1, CDR-H2 and CDR-H3, consisting of a combination of SEQ ID NO: 7 or 8 as the CDR-H1 amino acid sequence, any selected from among SEQ ID NOs: 9, 10, 11 and 12 as the CDRH-H2 amino acid sequence and SEQ ID NO: 13 as the CDRH-H3 amino acid sequence, and the L chain variable region contains the amino acid sequences CDR-L1, CDR-L2 and CDR-L3, consisting of a combination of SEQ ID NO: 14 or 15 as the CDR-L1 amino acid sequence, SEQ ID NO: 16 as the CDR-L2 amino acid sequence and SEQ ID NO: 17 as the CDR-L3 amino acid sequence. Preferably, it is an antibody wherein the set of CDR-H1, CDR-H2 and CDR-H3 in the H chain variable region is any selected from among the combinations: SEQ ID NO: 7, SEQ ID NO: 9 and SEQ ID NO: 13; SEQ ID NO: 8, SEQ ID NO: 9 and SEQ ID NO: 13; SEQ ID NO: 7, SEQ ID NO: 10 and SEQ ID NO: 13; SEQ ID NO: 8, SEQ ID NO: 12 and SEQ ID NO: 13; and SEQ ID NO: 7, SEQ ID NO: 11 and SEQ ID NO: 13, the set of CDR-L1, CDR-L2 and CDR-L3 in the L chain variable region is SEQ ID NO: 14, SEQ ID NO: 16 and SEQ ID NO: 17; or SEQ ID NO: 15, SEQ ID NO: 16 and SEQ ID NO: 17, and more preferably it is an antibody wherein the combination of the set of CDR-H1, CDR-H2 and CDR-H3 in the H chain variable region and the set of CDR-L1, CDR-L2 and CDR-L3 in the L chain variable region is selected from among the combinations: SEQ ID NO: 7, SEQ ID NO: 9, SEQ ID NO: 13, SEQ ID NO: 14, SEQ ID NO: 16 and SEQ ID NO: 17; SEQ ID NO: 8, SEQ ID NO: 9, SEQ ID NO: 13, SEQ ID NO: 14, SEQ ID NO: 16 and SEQ ID NO: 17; SEQ ID NO: 7, SEQ ID NO: 10, SEQ ID NO: 13, SEQ ID NO: 14, SEQ ID NO: 16 and SEQ ID NO: 17; SEQ ID NO: 8, SEQ ID NO: 12, SEQ ID NO: 13, SEQ ID NO: 14, SEQ ID NO: 16 and SEQ ID NO: 17; and SEQ ID NO: 7, SEQ ID NO: 11, SEQ ID NO: 13, SEQ ID NO: 15, SEQ ID NO: 16 and SEQ ID NO: 17. The antibody of the invention also includes those wherein at least one of the corresponding CDR sequences among the amino acid sequences of CDR-H1, CDR-H2, CDR-H3, CDR-L1, CDR-L2 and CDR-L3 is a sequence exhibiting at least 85% homology (identity) and preferably at least 90% homology, with any of SEQ ID NO: 7 to 17, according to the BLAST method (default conditions for NCBI PBLAST).

The method for identifying the sequence of CDR-H1, CDR-H2, CDR-H3, CDR-L1, CDR-L2 or CDR-L3 in the antibody may be, for example, the method of Kabat or the method of Chothia, and a person skilled in the art can identify the sequence of the portion corresponding to each CDR, the methods of Kabat (Kabat, E.A. and Wu, T.T., J. Immunol., 147, 1709-1719, 1991) and Chothia (Al-Lazikani, B., Lesk, A.M. and Chothia, C., J. Mol. Biol., 273,927-948, 1997) being common technical knowledge for those skilled in the relevant field, and their summaries may be found on the web site of Dr. Andrew C.R. Martin's Group (http://www.bioinf.org.uk/abs/), for example.

A different form of the antibody of the invention may be an antibody wherein the H chain variable region (VH) amino acid sequence is one selected from among SEQ ID NOs: 18 to 24 and the L chain variable region (VL) amino acid sequence is one selected from among SEQ ID NOs: 25 to 30, and preferably an antibody wherein the combination of VH and VL amino acid sequences is one selected from among the combinations: SEQ ID NO: 18 and SEQ ID NO: 25, SEQ ID NO: 19 and SEQ ID NO: 26, SEQ ID NO: 20 and SEQ ID NO: 26, SEQ ID NO: 21 and SEQ ID NO: 27, SEQ ID NO: 22 and SEQ ID NO: 28, SEQ ID NO: 23 and SEQ ID NO: 29, and SEQ ID NO: 24 and SEQ ID NO: 30. The antibody of the invention includes those wherein at least one from the VH and VL amino acid sequences is any VH amino acid sequence listed as SEQ ID NOs: 18 to 24 or any VL amino acid sequence listed as SEQ ID NOs: 25 to 30, and sequences exhibiting at least 85% homology (identity) and preferably at least 90% homology with SEQ ID NOs: 18 to 30 based on the BLAST method (default conditions for NCBI PBLAST).

The amino acid sequences of the constant regions in such antibodies are selected from among the mammalian subtypes IgG, IgA, IgM, IgE and IgD, or their variants.

A person skilled in the art can design recombinant antibodies suitable for administration toward treatment of the species of interest, such as humanized antibodies, based on information for the amino acid sequences of CDR-H1, CDR-H2 and CDR-H3 and/or the nucleotide sequences of genes coding for them, or the amino acid sequences of CDR-L1, CDR-L2 and CDR-L3 and/or the nucleotide sequences of genes coding for them, and a person skilled in the art can also design chimeric antibodies according to the purpose, based on information for the amino acid sequence of the H chain variable region and/or the nucleotide sequence of a gene coding for it, or the amino acid sequence of the L chain variable region and/or the nucleotide sequence of a gene coding for it. Furthermore, a person skilled in the art can appropriately use known technology for creation of low-molecular antibodies or scaffold antibodies, based on information for the amino acid sequences of CDR-H1, CDR-H2 and CDR-H3 and/or the nucleotide sequences of genes coding for them, or the amino acid sequences of CDR-L1, CDR-L2 and CDR-L3 and/or the nucleotide sequences of genes coding for them, or based on information for the amino acid sequence of the H chain variable region and/or the nucleotide sequence of a gene coding for it, or the amino acid sequence of the L chain variable region and/or the nucleotide sequence of a gene coding for it.

The antibody of the invention may be an antibody composed of two H chains and two L chains, or it may be an antibody composed of only two H chains. Also, the antibody of the invention may be an antibody fragment, examples of antibody fragments including F(ab')2, Fab and Fv structures.

The antibody of the invention can be obtained using techniques known to those skilled in the art. The antibody of the invention is a monoclonal antibody. Antibodies further disclosed herein may be monoclonal or polyclonal antibodies (Milstein et al., Nature (England), October 6, 1983, Vol.305, No. 55934, p.537-540). For example, polyclonal antibody can be obtained using as antigen tau protein that has been phosphorylated on at least one amino acid residue corresponding to positions 410 to 421 of SEQ ID NO: 1, tau protein that has been phosphorylated at one or more sites selected from among Ser412, Ser413, Thr414 and Ser416 of SEQ ID NO: 1, tau protein that has been phosphorylated at the Ser413 site of SEQ ID NO: 1, or a peptide having complete homology with at least a portion of the amino acid sequence from positions 410 to 421 of tau protein of SEQ ID NO: 1, or homology with at least 80% of the sequence, and that has been phosphorylated at these amino acid residues, to sensitize a mammal, and the antibody recovered from the serum of the animal. When the peptide is to be used as antigen, the antigen may be used in a form conjugated with a carrier protein such as BSA or KLH, or with polylysine. Specific examples of peptides to be used as antigens include the peptides of SEQ ID NO: 31 or 32 that have been phosphorylated at the site corresponding to Ser413 of SEQ ID NO: 1, as described in Examples 1 and 2, but there is no limitation to these. For a monoclonal antibody according to the invention, a hybridoma established by using immunocytes from a mammal sensitized with the antigen and fusing them with myeloma cells or the like, may be cloned and the antibody recovered from the culture. A method for obtaining such monoclonal antibodies is described in Example 2, and the monoclonal antibodies obtained thereby may be monoclonal antibodies containing the VH amino acid sequences of SEQ ID NO: 18 to 24 and the VL sequences of SEQ ID NO: 25 to 30 (Ta1501, 1502, 1505-1509), but there is no limitation thereto.

For the obtained monoclonal antibody, it is possible to obtain a nucleic acid molecule having a gene sequence coding for the amino acids of the antibody protein, and it is possible to prepare the antibody by genetic engineering techniques using such a nucleic acid molecule. Introducing modifications to increase the bindability or specificity of an antibody, using genetic information of the antibody, such as information for the H chain, L chain or their variable regions or CDR sequences, and preparing antibodies having a structure suitable for use in treatment by modifying an antibody of an animal such as a mouse to a human-type antibody, are techniques that are well known to those skilled in the art. Furthermore, by using non-human transgenic animals in which a human antibody gene has been transferred, as the animals for sensitization with antigen, it is possible to obtain human-type monoclonal antibodies. In addition, techniques in which a phage library expressing the antigen-binding region of a human antibody or a portion thereof (human antibody phage display) is used to obtain antibody specifically binding with the corresponding antigen, or a phage clone comprising a specific amino acid sequence, human antibodies are prepared from that information, can be appropriately carried out by a person skilled in the art, as a method that does not require sensitization of animals.(See Taketo Tanaka et al., Keio J. Med., Vol.60, p.37-46, for example).

Moreover, as the aforementioned method of producing monoclonal antibodies, a hybridoma producing the antibody to be obtained may be cultured and the antibody purified and obtained by common methods from the resulting culture supernatant. As a different production method, a gene coding for an antibody, and more specifically a gene coding for the immunoglobulin heavy chain and/or light chain, may be obtained from a hybridoma that produces the antibody of interest, or from a phage clone or the like obtained by a human antibody phage display, and a vector for expression of the gene prepared and transferred into host cells (mammalian cells, insect cells, microbes or the like) to produce the antibody. During this procedure, a person skilled in the art may carry out publicly known techniques for gene modification of the gene coding for the immunoglobulin heavy chain and/or light chain, for introduction of desired traits, or may create a human-type antibody, antibody chimera protein, low-molecular antibody or scaffold antibody, using structural information for the variable region or CDR of the immunoglobulin heavy chain and/or light chain. In addition, in order to improve the antibody performance or avoid side-effects, techniques well known to those skilled in the art may be appropriately employed for introducing modifications into the structure of the constant region of the antibody, or modifying its sugar chain portions.

### [Phosphorylated peptide with tau-derived amino acid sequence]

The invention provides peptides that contain portions of the amino acid sequence of tau between the amino acids 410 and 421 and that have been phosphorylated at the amino acid residue Ser413. A "phosphorylated" amino acid residue is one that is ester bonded to a phosphate group on a side chain of the amino acid residue, typical phosphorylated amino acid residues being serine, threonine and tyrosine. Phosphorylated peptides may be peptides with a length of at least 8 amino acids containing at least 3 contiguous amino acids among the amino acid sequence consisting of the amino acid residues corresponding to amino acid numbers 410-421 of SEQ ID NO: 1, preferably a peptide with a length of at least 8 amino acids containing at least 5 contiguous amino acids, and more preferably a peptide with a length of at least 8 amino acids containing at least 8 contiguous amino acids. The phosphorylated amino acid residues in the phosphorylated peptides may be any of the amino acid residues corresponding to amino acid numbers 410-421 of SEQ ID NO: 1, preferably any of the amino acid residues corresponding to amino acids Ser412, Ser413, Thr414 and Ser416 of SEQ ID NO: 1. The phosphorylated amino acid residue in the phosphorlylated peptides of the invention is the amino acid residue corresponding to Ser413 of SEQ ID NO: 1. A typical example of such a phosphorylated peptide is the phosphorylated peptide used for antibody preparation in Example 1 (SEQ ID NO: 31), but there is no limitation to this one.

A phosphorylated peptide having a tau-derived amino acid sequence according to the invention may be used in a therapeutic agent or prophylactic agent for cognitive disorders that comprises antigen for preparation of anti-phosphorylated tau antibody according to the invention, or the peptide itself. The phosphorylated peptide may also be modified with substances providing other functions, according to the purpose, at the N-terminus and/or C terminus of the sequence. For example, it may have a methionine residue, acetyl or pyroglutamic acid added to the N-terminus of the phosphorylated peptide, or it may be modified with a fluorescent substance or the like. Substances used to modify the N-terminus and/or C-terminus of the phosphorylated peptide may also be peptides or proteins, examples of which include peptides with amino acid sequences of appropriate tag sequences (typically histidine tag or FLAG tag), recognition sequence of T cell receptors (TCR) or major histocompatibility antigens (MHC), and viral or bacterial proteins, or peptides with sequences derived therefrom, added to the N-terminus or C-terminus. The phosphorylated peptide of the invention also includes those modified with compounds or peptides at one or more amino acid residues other than at the N-terminus or C-terminus. Such phosphorylated peptide modification can be accomplished using methods known to those skilled in the art, such as the method described by Hermanson et al. (Bioconjugate techniques, USA, 1996, Academic Press).

The phosphorylated peptide of the invention can be produced by a person skilled in the art using appropriate synthesis or genetic engineering methods. Examples of methods for producing phosphorylated peptides by synthesis include Boc methods (Wakamiya T. et al., Chemistry Letters, Vol.22 P.1401, 1993), Fmoc methods (PERICH, J. W., International Journal of Peptide and Protein Research, vol.40, P.134-140, 1992), and the method described in Japanese Patent No. 3587390, although other appropriate methods may be implemented by a person skilled in the art. Also, production methods by genetic engineering may involve preparation of genetic material (DNA, RNA) having a nucleotide sequence coding for the peptide to be produced or its precursor, and insert into an appropriate expression vector with addition of a promoter sequence and the like, for introduction into a suitable host for expression, or production using a cell-free protein synthesis system. In this case, the phosphorylated peptide that has been phosphorylated at the site of interest can be produced by appropriate phosphorylation reaction in a host by a kinase that is in the host or induced by genetically engineered expression, or the peptide of interest may be collected first and then reacted with kinase or the like *in vitro* for phosphorylation reaction. For such *in vitro* phosphorylation reaction, the enzyme used may be one whose function in tau phosphorylation reaction for peptides of interest is known, such as GSK3 or CDK5. In order to obtain peptides with the amino acid residues of interest phosphorylated, among the phosphorylated peptides that have been phosphorylated in this manner in a host or *in vitro,* there may be used methods for recovery of phosphorylated peptides specifically bound to antibody in antibody-antigen reaction using the anti-phosphorylated tau antibodies mentioned above.

### [Therapeutic agent or prophylactic agent for cognitive disorders comprising anti-phosphorylated tau antibody or phosphorylated tau peptide]

The term "cognitive disorders" for humans means a condition of impairment in intellectual function that has been developed or acquired, and it is considered a type of intellectual disturbance (Kamijima, K., Niwa, S., Nankodo's Essential Well - Advanced Series, New Seishin Igaku, p.69-70, 2008), and in a wider sense they are considered diseases exhibiting intellectual disturbance and/or memory impairment. In neurodegenerative diseases such as AD, "neurodegeneration" can be ascertained by confirming the presence of neurofibrillary tangles (NFT) by postmortem histological analysis, but a physician may conduct diagnosis of neurodegenerative disease by the Hasegawa Dementia Scale - Revised (HDS-R) or Mini-Mental State Examination (MMSE), based on interrogation as part of neuropsychological examination, by Clinical Dementia Rating (CDR) or Functional Assessment Staging (FAST) based on observation, by increase in the abundance of tau or phosphorylated tau or increase in the abundance of Aβ in cerebrospinal fluid, as biochemical examination, or based on information obtained from cranial CT, cranial MRI, SPECT or PET, as imaging examination, to diagnose cognitive disorder and specifically neurodegenerative disease. Thus, the therapeutic agent or prophylactic agent for cognitive disorders of the invention is administered to a patient diagnosed with cognitive disorder by a physician, and it has an improving effect in comparison to before administration, for at least one item of diagnosis of neurodegenerative disease, or an effect of inhibiting progression of symptoms or maintaining or recovering the state before administration.

Patients to be administered the therapeutic agent or prophylactic agent for cognitive disorders of the invention are patients with cognitive disorders, and especially patients with tauopathies, which include patients with Alzheimer's disease (AD), cortical-basal ganglia degeneration (CBD or CBS), progressive supranuclear palsy, Pick's disease, argyrophilic grain dementia (argyrophilic grain disease), Multiple system tauopathy with dementia (MSTD), chromosome 17-linked frontotemporal dementia with Parkinsonism (FTDP-17), neurofibrillary tangle dementia, diffuse neurofibrillary tangles with calcification (DNTC), white matter tauopathy with globular glial inclusions (WMT-GGI), frontotemporal lobar degeneration with tau-positive inclusions (FTLD-tau), Economo's postencephalitic Parkinson's disease, subacute sclerosing panencephalitis or boxer's encephalopathy. Therefore, the therapeutic agent for cognitive disorders of the invention is a therapeutic agent or prophylactic agent for tauopathies, and from a different viewpoint, it may be considered to be a therapeutic agent or prophylactic agent for Alzheimer's disease (AD), cortical-basal ganglia degeneration (CBD or CBS), progressive supranuclear palsy, Pick's disease, argyrophilic grain dementia (argyrophilic grain disease), Multiple system tauopathy with dementia (MSTD), chromosome 17-linked frontotemporal dementia with Parkinsonism (FTDP-17), neurofibrillary tangle dementia, diffuse neurofibrillary tangles with calcification (DNTC), white matter tauopathy with globular glial inclusions (WMT-GGI), frontotemporal lobar degeneration with tau-positive inclusions (FTLD-tau), Economo's postencephalitic Parkinson's disease, subacute sclerosing panencephalitis or boxer's encephalopathy.

The therapeutic agent or prophylactic agent for cognitive disorders of the invention may also be considered to have an effect of improving cognitive function or inhibiting loss of or maintaining cognitive function in non-human animals. Such non-human animals include animals such as chimpanzees, rhesus monkeys, horses, pigs, dogs, mice, rabbits, rats, cats and the like expressing tau with high homology for human tau, with no limitation to these.

### [Animal models for cognitive disorder]

Animals for research on the therapeutic agent or prophylactic agent for cognitive disorders of the invention include animal models for cognitive disorders, among which animal models of tauopathies may be mentioned in particular. Animal models for tauopathies are animals expressing normal-type or gene mutant tau in the brain, and particularly animal models exhibiting impairment in cognitive function. Such animals expressing normal-type or gene mutant tau in the brain can be prepared by genetic engineering, a typical example being transgenic mice (Tg mice). Animal models such as Tg mice that express gene mutant tau are useful as models of genetic familial tauopathies, but for examination of the effect of a therapeutic agent or prophylactic agent for sporadic tauopathies that constitute the majority of cases among humans, preferably an effect is exhibited in Tg mice expressing normal-type tau. Most suitable as Tg mice expressing normal-type tau are mice prepared in the production examples of the present invention, but there may also be used the Tg mice reported by Kambe et al. (Neurobiology of Disease, Vol.42, P.404-414, 2011) and Kimura et al. (The EMBO J. vol.26. P.5143-5152, 2007). However, although cognitive function impairment is seen in the mice of Kambe et al. and Kimura et al., it appears after 14 months of age and 20 months of age, respectively, and therefore the onset is well into senescence and aging effects may also be contributing factors, while the effects and labor of long-term breeding are also issues. In contrast, the mice prepared in the production examples of the present invention express human normal-type tau and exhibit onset of cognitive function impairment at the relatively early stage of about 6 months of age, and they are therefore most suitable as cognitive disorder models for which factors such as aging can be excluded, and using such models allows more accurate evaluation of therapeutic agents or prophylactic agent for cognitive disorders that have improving effects on cognitive function.

The preferred method of examining the effect of a therapeutic agent or prophylactic agent for cognitive disorders according to the invention in an animal model is a method of testing cognitive function, such as a memory learning test. Such a method may be a Morris water maze test, a step-through learning test or a novel object recognition test, but preferably it is a combination of behavioral measurement tests such as an Open Field Test, in order to take into account the conditions of behavior quantity and animal anxiety.

As methods for examining the effect of a therapeutic agent or prophylactic agent for cognitive disorders according to the invention, it is possible to use methods of examining the levels of tau protein or phosphorylated tau in brain tissue, during administration to an animal model of the cognitive disorder. In AD and other neurodegenerative diseases, expression levels of tau protein or increased abnormal phosphorylated tau are associated with pathology (Khalid Iqbal et al., Curr. Alzheimer Res., Vol.7, p.654-664, 2010). It is also well known that reducing tau expression and abnormal phosphorylated tau levels in some pathological model animals produces improvement in cognitive function and motor function (K. Santa Cruz et al., Science, 30, Vol.9, p.476-481, 2005; Sylvie Le Corre et al., Proc. Nat. Acad. Sci. USA, 10, Vol.3, p.9673-9678, 2006). As a method of examining changes in tau protein or phosphorylated tau, this can be accomplished by a method such as Western blotting using a brain tissue homogenate, as described in the examples, but a person skilled in the art can select another appropriate method such as ELISA (Xiyun Chai et al., J. Biol. Chem., Vol.286, p.34457-34467, 2011) or an immunohistochemical method (David J. Irwin et al., BRAIN, Vol.135, p.807-818, 2012).

The effect of a therapeutic agent or prophylactic agent for cognitive disorders of the invention in an animal model may be used as pharmacological effect data for development of a therapeutic agent or prophylactic agent in humans.

### [Therapeutic agent or prophylactic agent for cognitive disorders]

One form of the therapeutic agent or prophylactic agent for cognitive disorders provided herein is one comprising anti-phosphorylated tau antibody, and preferably an antibody that participates in antigen-antibody reaction specifically with tau protein that has been phosphorylated on at least one amino acid residue corresponding to positions 410 to 421 of SEQ ID NO: 1 in tau protein, more preferably an antibody that participates in antigen-antibody reaction with tau protein that has been phosphorylated at one or more sites selected from among the amino acid residues corresponding to Ser412, Ser413, Thr414 and Ser416 of SEQ ID NO: 1, more preferably an antibody that binds competitively against an antibody whose VH amino acid sequence is SEQ ID NO: 20 and whose VL amino acid sequence is SEQ ID NO: 26 (1505). One form of the therapeutic agent or prophylactic agent for cognitive disorders of the invention is one comprising an anti phosphorylated tau antibody that participates in antigen-antibody reaction with tau protein that has been phosphorylated at the site of the amino acid residue corresponding to Ser413 of SEQ ID NO: 1. Such antibodies were explained above under the heading [Anti-phosphorylated tau antibody].

Another form of the therapeutic agent or prophylactic agent for cognitive disorders of the invention is one containing a peptide that includes a portion of the tau sequence between amino acid residues 410 and 421 and has been phosphorylated at the amino acid residues Ser413. A phosphorylated peptide may be is a peptide with a length of at least 8 amino acids containing at least 3 contiguous amino acids among the amino acid sequence consisting of the amino acid residues corresponding to amino acid numbers 410-421 of SEQ ID NO: 1, preferably a peptide with a length of at least 8 amino acids containing at least 5 contiguous amino acids, and more preferably a peptide with a length of at least 8 amino acids containing at least 8 contiguous amino acids. Also, the phosphorylated amino acid residues in the phosphorylated peptides may be any of the amino acid residues corresponding to amino acid numbers 410-421 of SEQ ID NO: 1, preferably any of the amino acid residues corresponding to amino acids Ser412, Ser413, Thr414 and Ser416 of SEQ ID NO: 1. The therapeutic agent or prophylactic agent for cognitive disorders of the invention is one containing a peptide that includes a portion of the tau sequence between amino acids 410 and 421 and has been phosphorylated at the amino acid residue corresponding to Ser413 of SEQ ID NO: 1. Such peptides were explained above under the heading [Phosphorylated peptide with tau-derived amino acid sequence].

The therapeutic agent or prophylactic agent for cognitive disorders of the invention may also contain pharmaceutically acceptable additives. Formulations using pharmaceutically acceptable additives can be prepared by the method described in "Remington: The Science and Practice of Pharmacy, 20th Edition, University of the Sciences in Philadelphia, Williams & Wilkins, December 15, 2000". One form for such a therapeutic agent or prophylactic agent is a liquid drug prepared by dissolution, suspension or emulsification in an aseptic aqueous solution or oily solution. Solvents for this include distilled water for injection and physiological saline, for aqueous solutions, with addition of an osmoregulating agent (for example, D-glucose, D-sorbitol, D-mannitol, sodium chloride and the like), often used in combination with a suitable dissolving aid such as an alcohol (for example, ethanol), a polyalcohol (for example, propylene glycol or polyethylene glycol), or a nonionic surfactant (for example, polysorbate 80 or polyoxyethylene hydrogenated castor oil 50). Oily solutions are also sometimes used as solvents, examples of such oily solutions including sesame oil and soybean oil, which are often used in combination with benzyl benzoate, benzyl alcohol or the like as dissolving aids. In such liquid drugs, there are often used appropriate additives such as buffering agents (for example, phosphate buffering agents and acetate buffering agents), soothing agents (for example, benzalkonium chloride and procaine hydrochloride), stabilizers (for example, human serum albumin and polyethylene glycol), preservatives (for example, ascorbic acid, erythorbic acid, and their salts), coloring agents (for example, copper chlorophyll β-carotene, Red #2 and Blue #1), antiseptic agents (for example, paraoxybenzoic acid esters, phenol, benzethonium chloride and benzalkonium chloride), thickeners (for example, hydroxypropyl cellulose, carboxymethyl cellulose, and their salts), stabilizers (for example, human serum albumin mannitol and sorbitol), and odor correctives (for example, menthol and citrus aromas). Different forms of therapeutic agents or prophylactic agent include solid formulations such as powders, tablets, granules, capsules, pills, suppositories, lozenges and the like. For a solid formulation to be administered in oral form, there may be used additives such as excipients (for example, crystalline cellulose, lactose and starch), lubricants (for example, magnesium stearate and talc), binders (hydroxypropyl cellulose, hydroxypropyl methyl cellulose, macrogol and the like), and disintegrators (for example, starch and carboxymethyl cellulose calcium). If necessary, additives such as antiseptic agents (for example, benzyl alcohol, chlorobutanol, methyl paraoxybenzoate and propyl paraoxybenzoate), antioxidants, coloring agents, sweeteners and the like may be used. Other alternative forms include therapeutic agents or prophylactic agent for application onto mucous membranes, such formulations often containing additives such as pressure-sensitive adhesives, pressure-sensitive enhancers, viscosity regulators, thickening agents and the like (for example, mucin, agar, gelatin, pectin, carrageenan, sodium alginate, locust bean gum, xanthan gum, tragacanth gum, gum arabic, chitosan, pullulan, waxy starch, sucralfate, cellulose and its derivatives (such as hydroxypropyl methyl cellulose), polyglycerol fatty acid esters, acrylic acid-alkyl (meth)acrylate copolymers, or their salts and polyglycerol fatty acid esters), primarily for the purpose of imparting mucosal adsorption or retention properties. However, the form, solvent and additives for the therapeutic agent or prophylactic agent to be administered to the body are not limited to these, and appropriately selection may be made by a person skilled in the art.

The therapeutic or prophylactic agent for cognitive disorders according to the invention also encompasses the concept of a therapeutic agent or prophylactic agent comprising existing substances with effects of inhibiting development of cognitive disorder, in addition to the aforementioned anti-phosphorylated tau antibody or phosphorylated peptide. Also, the therapeutic or prophylactic agent for cognitive disorders according to the invention may be part of a kit for combined use of a therapeutic agent or prophylactic agent containing the anti-phosphorylation antibody or phosphorylated peptide and a therapeutic agent or prophylactic agent containing an existing substance with effects of inhibiting development of cognitive disorder. Examples of substances that inhibit development of cognitive disorder include, but are not limited to, donepezil, galantamine, memantine and rivastigmine. The dosage of the substance with an effect of inhibiting development of cognitive disorder that is to be added, or the therapeutic agent or prophylactic agent containing the substance with an effect of inhibiting development of cognitive disorder, may be a commonly employed dosage for treatment, which may be varied according to the conditions.

Also, while the results of the examples demonstrate that the antibody to be used for carrying out the invention exhibits a drug effect by acting on the brain through the blood-brain barrier even when administered peripherally by intraperitoneal administration, it is possible to prepare a formulation that efficiently supplies an anti-phosphorylated tau antibody, which can also be used as a cognitive disorder therapeutic or prophylactic agent of the invention, to brain tissue, and such formulations are also encompassed by the therapeutic or prophylactic agent for cognitive disorders according to the invention. Methods for efficiently supplying antibodies or peptides to brain tissue through the blood-brain barrier are known, such as methods of adding targeting substances or methods of encapsulating in liposomes or nanoparticles. Substances to be used for targeting include those that undergo total or partial change in charge characteristics by binding with antibody, or peptides, proteins or other compounds having a property of binding with a specific receptor or transporter. Examples of peptides, proteins or other compounds having a property of binding with a specific receptor or membrane protein include ligands that bind to receptor ligands or membrane proteins, and their analogs, and antibodies, agonist compounds/antagonist compounds/allosteric modulators that bind to receptor ligands or membrane proteins, and their analog compounds. Examples of receptor ligands or membrane proteins as targets for a peptide, protein or other compound having the property of binding to a specific receptor or transporter include transferrin receptor (TfR), insulin receptor (IR), insulin-like growth factor receptor (IGFR), LDL receptor-related protein (LRP) and diphtheria toxin receptor (HB-EGF), with no particular limitation to these (Angela R. Jones et al., Pharm. Res., Vol.24, p.1759-1771, 2007). A substance for targeting may be chemically added to the antibody to be used for the therapeutic or prophylactic agent for cognitive disorders according to the invention, the method being one that can be appropriately carried out by a person skilled in the art with reference to a known method such as described in, for example, Hermanson et al., Bioconjugate techniques, USA 1996, Academic Press. The substance for targeting may also be bound to liposomes or nanoparticles encapsulating the antibody or peptide (Sonu Bhaskar et al., Particle and Fibre Toxicology, Vol.7, No.3, 2010). In addition, when the substance for targeting is a peptide or protein, it can be produced as an appropriate fusion protein by a person skilled in the art using genetic engineering techniques, either by producing a fusion peptide by peptide chemical synthesis, or by combining a nucleic acid comprising a nucleotide sequence coding for the amino acid sequence of a peptide or protein with a nucleic acid comprising a nucleotide sequence coding for the amino acid sequence for the antibody or peptide to be used.

An agent for treatment or prevention according to the invention may be administered orally or parenterally, for the purpose of improving symptoms. For oral administration, a dosage form such as granules, powder, tablets, capsules, liquid drug, syrup, emulsion, suspending agent or elixir may be selected. For parenteral administration, a transnasal agent may be prepared, and a liquid drug, suspension or solid formulation may be selected. An injection may be prepared as a different form of parenteral administration, the injection being selected as hypodermic injection, intravenous injection, dropping injection, intramuscular injection, intracerebroventricular injection or intraperitoneal injection. Other formulations used for parenteral administration include suppositories, sublingual agents, percutaneous agents and transmucosal administration agents other than transnasal agents. In addition, intravascular local administration is possible by a mode of addition or coating onto a stent or intravascular obturator.

The dose for an agent for treatment or prevention according to the invention will differ depending on the patient age, gender, body weight and symptoms, the therapeutic effect, the method of administration, the treatment time, or the types of active ingredients in the medical composition, but normally it may be administered in the range of 0.1 mg to 1 g and preferably in the range of 0.5 mg to 200 mg of active compound per administration for adults. However, since the dose will vary depending on a variety of conditions, lower doses than those mentioned above may be sufficient, or doses exceeding these ranges may be necessary.

The agent for treatment or prevention according to the invention can exhibit an effect within a short administration period.

### [Examples]

The present invention will now be explained in greater detail by examples, with the understanding that the examples are not limitative on the invention in any way. The experiments were conducted with the approval of the Animal Experiment Committee at Osaka City University, Abeno Campus.

### [Example 1] Preparation of rabbit polyclonal antibody for pSer413 peptide

The antigen used to prepare antibody for pSer413 peptide was a synthetic peptide (SEQ ID NO: 31, synthesized by Medical & Biological Laboratories Co., Ltd. [MBL]) having Cys attached at the C-terminal site of the amino acid sequence corresponding to the region from the 410th Asn to the 416th Ser of the amino acid sequence of SEQ ID NO: 1 for human tau protein, phosphorylated at Ser corresponding to position 413 (this peptide will hereunder be referred to as pSer413(S) peptide).

### pSer413(S) peptide: N-AsnValSer(pSer)ThrGlySerCys-C (SEQ ID NO: 31)

Following synthesis, the pSer413(S) peptide was purified with HPLC and covalently bonded with KLH (Keyhole Limpet Hemocyanin). The obtained conjugate was used for immunization with 0.1 mg per dose per rabbit. For the first immunization, 0.2 mL of conjugate solution (conjugate concentration: 0.5 mg/mL) was mixed with an equal amount of Freund's complete adjuvant, and the dorsal area of a shaved Japanese white rabbit was intradermally injected at 8 locations with 50 µL each. For the second and subsequent immunizations, Freund's incomplete adjuvant was used for two more similar immunizations every 2 weeks. At 2 weeks after the final immunization, a blood sample was taken and the antibody titer of the serum was confirmed by ELISA using immunized peptide conjugate, and the animal was sacrificed after 1 week and the whole blood was taken. Serum was prepared from the obtained blood, and antibodies were purified from the serum using an affinity column of pSer413(Af) peptide (SEQ ID NO: 32).

The serum titer was confirmed by the following method. The pSer413(S) peptide was diluted to 5 µg/mL with PBS, and then dispensed in a plate at 100 µL/well and allowed to stand overnight at 4°C. After removing the solution, blocking buffer (MBL Co.) was dispensed at 250µL/well, and the plate was allowed to stand overnight at 4°C. The dilution series for the pre-immunization rabbit serum and post-immunization rabbit serum was 100, 500, 2,500, 12,500 and 62,500 folds, with a PBS-diluted sample added at 100 µL/well, and reaction was conducted at 25°C for 60 minutes. After rinsing, anti-rabbit IgG-peroxidase labeling (product of MBL) diluted 8,000-fold with Buffer (MBL) was added at 100 µL/well, and reaction was conducted at 25°C for 60 minutes. After rinsing, coloring solution (MBL) was added at 100 µL/well for coloration for 3 to 10 minutes, and then 2N sulfuric acid was added at 100 µL/well to terminate the reaction. After the reaction was terminated, the absorbance was measured at a measuring wavelength of 450 nm and a reference wavelength of 620 nm.

The antibody reactivity of the purified antibody was confirmed by the following method. The pSer413(L) peptide [pSer413(S) peptide (underlined) further extended in the N-terminal and C-terminal directions (SEQ ID NO: 33, synthesized by Biosynthesis Co.): N-ProArgHisLeuSerAsnValSer(pSer)ThrGlySerIleAspMetValAsp-C] or NonP(L) peptide having the same amino acid sequence but not phosphorylated at the site corresponding to Ser413 (SEQ ID NO: 34, synthesized by Biosynthesis Co.) was diluted to 1 µg/mL with PBS and then dispensed into a plate at 50 µL/well, and allowed to stand overnight at 4°C. After removing the solution, blocking buffer (3% BSA-PBS) was dispensed at 250 µL/well, and the mixture was allowed to further stand at 37°C for 1 hour or longer. The purified antibody diluted with PBS and serialized was added at 50 µL/well, and reaction was conducted at 25°C for 90 minutes. After rinsing, goat anti-rabbit IgG-alkaline phosphatase labeling (Bioscience) was diluted 2,000-fold with dilution buffer (3% BSA-PBS) and added at 50 µL/well, and reaction was conducted at 25°C for 60 minutes. After rinsing, coloring solution (2.5 mM MgCl₂-containing 0.1 M diethanolamine buffer solution, with 1 mg/mL PNPP [para-nitrophenyl phosphate] added to pH 9.8) was added at 100 µL/well for coloration for 30 minutes, and the absorbance was measured at a measuring wavelength of 405 nm and a reference wavelength of 550 nm.

### <Results>

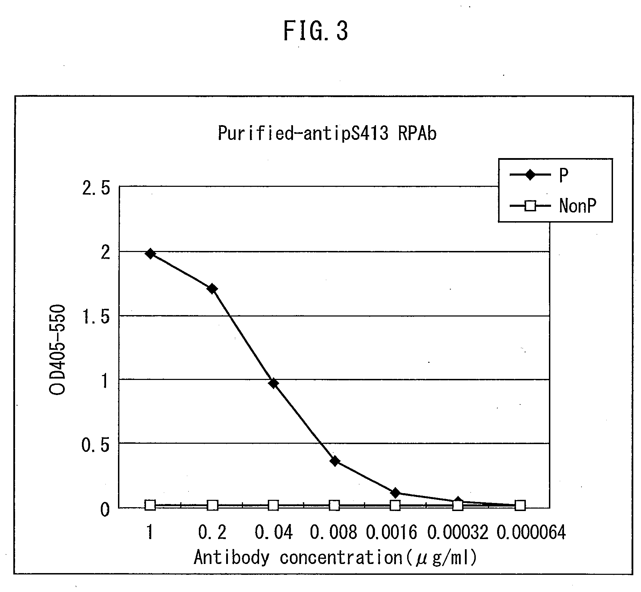

As shown in Fig. 3, the purified antibody had high specificity for pSer413(L) peptide, while virtually no reaction was observed with nonP(L) peptide. Therefore, the antibody is an antibody that participates in antigen-antibody reaction specifically with pSer413(L) (throughout the present specification, this may also be referred to as "pSer413-labeled rabbit polyclonal antibody" or "pS413AB").

### [Example 2] Preparation of monoclonal antibody for pSer413 peptide (Ta15 Series)

The antigen used was synthetic peptide pSer413(Im) (SEQ ID NO: 35), having GlyCysSerGly attached at the N-terminus of the sequence corresponding to the region from the 403rd Thr to the 423rd Pro, phosphorylated on the amino acid residue corresponding to Ser at position 413 of the amino acid sequence of human tau protein represented by SEQ ID NO: 1. Following synthesis, the pSer413(Im) peptide was purified with HPLC and covalently bonded with KLH (Keyhole Limpet Hemocyanin). The obtained conjugate was used for immunization with 0.04 mg per dose per mouse. The immunization was conducted by intraperitoneal injection into 10 mice at 200 µL each of a mixture of 0.4 ml of conjugate solution (1.1 mg/ml in terms of peptide), 0.7 mL of saline and 1.1 mL of Freund's complete adjuvant. Three more similar immunizations (with the same immunization location and antigen dose, using Freund's incomplete adjuvant) were conducted every 2 weeks. One month after the final immunization, 100 µL of a 0.5 mg/mL antigen solution (dissolved in saline) in terms of peptide was injected into the caudal vein of the one mouse among the ten that had increased serum antibody titer for the antigen, and on the third day the animals were sacrificed and the spleens recovered. Two 18G injection needles were used to break up the spleens, and then the broken-up spleens were gently mashed with a rubber stopper surface. The mashed splenocytes were suspended in approximately 10 mL of cold RPMI 1640 medium, the supernatant was filtered with a 40 µm cell strainer, and the filtrate was collected in a 50 mL tube. The spleen cell debris was further mashed with a rubber stopper surface, and similarly suspended in cold RPMI 1640 medium, filtered, and the filtrate collected. Cold RPMI 1640 medium (or cold PBS) was added to a final volume of 40 mL for the spleen cell suspension. The lymphocyte concentration in the suspension was measured with a hemacytometer, and lymphocytes in a final amount corresponding to 2 × 10⁷ cells were transferred to a 50 mL tube. To this there was added an equivalent of 4 × 10⁷ cells of the mouse myeloma cell line P3X63Ag8 ·U1 (P3·U1) in the logarithmic growth stage, that had been cultured in culture solution B (RPMI 1640 + 10% fetal bovine serum + 2 mM glutamine + 100 µg/mL streptomycin + 100 unit/mL penicillin), and after centrifugation at 1500 rpm for 5 minutes, the supernatant was discarded. The cell pellet was thoroughly dispersed by tapping the tube. To this there was added 0.5 mL of a polyethylene glycol solution (composed of RPMI 1640 (2.3 mL) + polyethylene glycol 2000 (1.4 mL) + dimethyl sulfoxide (0.3 mL), hereunder abbreviated as "PEG solution"), and the cells were gently suspended. After 1 minute, 0.5 mL of PEG solution was slowly added dropwise over a period of 1 minute, 1.0 mL of RPMI 1640 was slowly added dropwise over a period of 1 minute, and then 2 mL of RPMI 1640 was slowly added dropwise over a period of 2 minutes. Next, 4 mL of HAT/GIT culture solution (GIT medium [Nihon Pharmaceutical Co., Ltd.] + 5% fetal bovine serum + 100 µg/mL streptomycin + 100 unit/mL penicillin + 95 µM hypoxanthine + 0.4 µM aminopterin + 16 µM thymidine) was added dropwise over a period of 2 minutes, and then 4 mL of HAT/GIT culture solution was added dropwise over a period of 2 minutes. Finally, HAT/GIT culture solution was added to obtain 40 to 50 mL of cell suspension. After incubation in a thermostatic bath at 37°C for 30 minutes, the suspension was seeded onto 7 culture plates (96 wells). The culture plates used were 96-well plates (feeder plates) on which mouse (ICR) abdominal cavity macrophages (feeder cells) had been cultured for several days (> 1 × 10⁵/well). The plates were then cultured for 7 to 10 days at 37°C, 5% CO₂.

Half of the culture solution was replaced with fresh HT culture solution (HAT/GIT culture solution without aminopterin) once every week, and hybridomas were obtained.

Antibody screening was carried out by the following method. The pSer413(L) peptide was diluted to 1 µg/mL with PBS, and then dispensed in a 96-well plate at 50 µL/well and allowed to stand overnight at 4°C. After removing the solution, blocking buffer (3% BSA-PBS) was dispensed at 250 µL/well, and the mixture was left to stand at room temperature for 1 hour or longer. The blocking buffer (3% BSA-PBS) was aspirated, the hybridoma supernatant was added at 30-50 µL/well, and reaction was conducted at 25°C for 60 minutes. After rinsing with phosphate-buffered saline containing 0.05% Tween20 (PBS-Tween), secondary antibody (a solution containing alkaline phosphatase-labeled goat anti-mouse IgG-Fc antibody [SouthernBiotech], diluted 2000-fold with blocking buffer) was added at 100 µL/well, and reaction was conducted at 25°C for 60 minutes. After rinsing, substrate solution (2.5 mM MgCl₂-containing 0.1 M diethanolamine buffer, with 0.7 to 1.2 mg/mL PNPP [para-nitrophenyl phosphate] added to pH 9.8) was added at 100 µL/well for coloration at 25°C for 60 minutes, after which the absorbance was measured at a measuring wavelength of 405 nm.

In this screening, cells were recovered from the positive wells and counted with a hemacytometer, and then seeded onto a 96-well plate (the previous feeder plate) at 1-3 cells/well, for cloning by the limiting dilution method (Ta1501, Ta1502, Ta1505-1509). For subsequent analysis, the clones were mass cultured and the antibody was purified with a protein G column. Ta1505 is the same as Ta1505-2. The isotypes were determined using a kit by AbD Serotec.

The purified antibody was reacted with an ELISA plate coated with partial peptide corresponding to the different phosphorylation sites of tau, to determine the phosphate group specificity. The method was as follows.

A 10% DMSO solution of each tau peptide (pS46 [SEQ ID NO: 36], pS199 [SEQ ID NO: 37], pS202 [SEQ ID NO: 38], pT212 [SEQ ID NO: 39], pS214 [SEQ ID NO: 40], pT212/pS214 [SEQ ID NO: 41], pT217 [SEQ ID NO: 42], pS413 [SEQ ID NO: 33], non pS413 [SEQ ID NO: 34]), or a PBS solution of BSA-conjugated peptide (pS400 [SEQ ID NO: 43]-BSA, pS412 [SEQ ID NO: 44]-BSA) was diluted to 1 µg/mL with PBS (pH 7) and added to a 96-well plate (MaxiSorp: Nunc) at 50 µL/well, and then incubated overnight at 4°C for immobilization. Next, the peptide solution was removed and rinsed 3 times with TBS containing 0.05% Tween20, after which 3% BSA/PBS was added at 280 µL/well and blocking was performed at 37°C for 1 hour.

The blocking solution was aspirated off, rinsing was performed 3 times with TBS containing 0.05% Tween20, and then 1 µg/mL of each Ta15 Series monoclonal antibody solution in 3% BSA-PBS was added at 50 µL/well, and reaction was conducted at room temperature for 1 hour. After rinsing 3 times with TBS containing 0.05% Tween 20, 50 µL of alkaline phosphatase-labeled goat anti-mouse IgG antibody (ThermoScience) diluted 2000-fold with PBS containing 3% BSA was added, and reaction was conducted at room temperature for 1 hour. After rinsing 3 times with TBS containing 0.05% Tween 20, 100 µL of a 1 mg/mL PNPP (para-nitrophenyl phosphate) solution dissolved in 0.1 M diethanolamine (pH 10) was added, reaction was conducted at room temperature for 1 hour, and the absorbance at 405 nm was measured. The reactivity evaluation results of each antibody for each peptide are shown in Table 2. The reactivity was evaluated on a 3-level scale.
+: Reactivity, -: No reactivity, ±: Slight but very weak reactivity

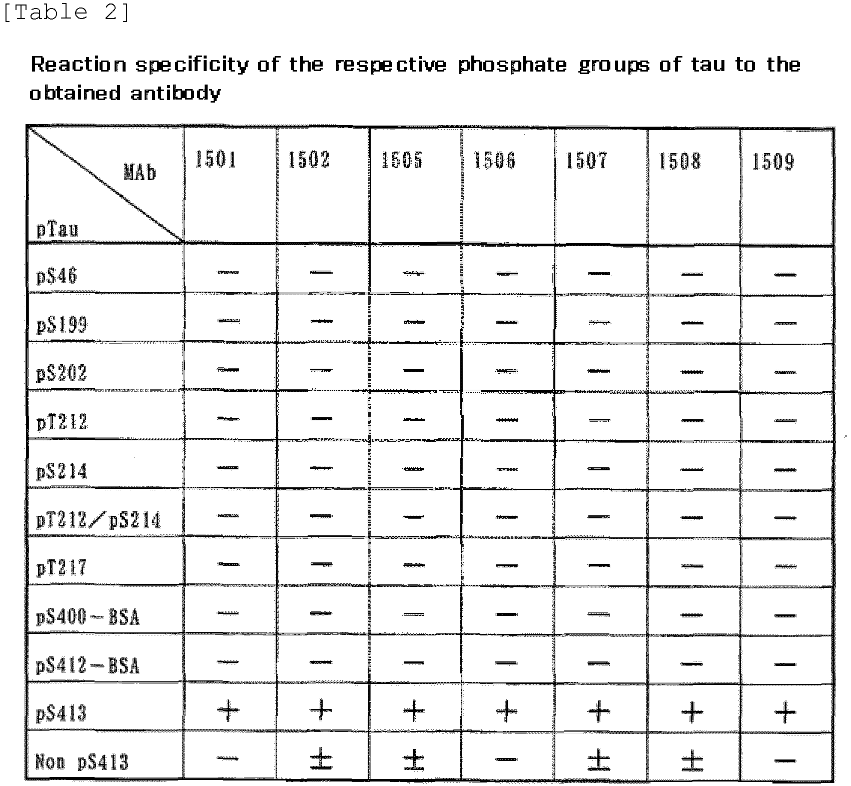

These antibodies (hereunder referred to as "Ta15 Series") were reacted only with pSer413 among the examined peptides, and the phosphate group specificity was extremely high.

### [Example 3] Measurement of Ta15 Series antibody binding affinity

In order to evaluate the binding affinity between the Ta15 Series antibodies and antigen peptide (pSer413 peptide (Im): see Example 2), a Biacore Surface Plasmon Resonance (SPR) system (BIACORE3000, code# BR-1100-45, by GE Healthcare, Japan) and each Biacore product were used for measurement according to the manufacturer's manual. One method of measurement used was a method of immobilizing (code# BR-1006-33) anti-mouse antibody (code# BR-1008-38) on a CM5 chip (carboxymethyldextran layer-formed chip, code# BR-1100-14) by covalent bonding through amine coupling reaction, binding the Ta15 Series antibody to the immobilized anti-mouse antibody, and measuring the binding behavior of the antigen peptide to the bound Ta15 Series antibody.

The specifically-binding reaction mixture was used with HBS-EP buffer (code# BR-T-1001-88), and the CM5 chip was activated by a mixed solution of N-ethyl-N'-(3-dimethylaminopropyl)-carbodiimide hydrochloride (EDC) and N-hydroxysuccinimide (NHS). A solution of anti-mouse antibody diluted to 0.001 mg/mL with 10 mM sodium acetate buffer at pH 5.0 was reacted with 4 flow cells on the chip, and after covalently bonding the anti-mouse antibody to the CM5 chip, it was subjected to blocking with ethanolamine. Of the 4 flow cells immobilizing anti-mouse antibody, one trapped negative antibody (mouse IgG2a isotype control, code# MAB003, by R&D Systems) while the other flow cells trapped Ta15 Series antibody, at a density of about 1000 RU each. HBS-EP buffer (0.01 M HEPES pH 7.4, 0.15 M NaCl, 3 mM EDTA, 0.005% v/v Surfactant P20) with added 1 M NaCl was reacted for 3 minutes and the nonspecific adsorbed antibody was removed, and then HBS-EP buffer was reacted for 10 minutes at a flow rate of 50 µL/min to stabilize the baseline. The peptide was serially diluted with HBS-EP buffer from a concentration of 100 pM to 5 mM (near the estimated binding dissociation constant [KD]), the obtained peptide solutions (5 concentrations) were reacted for between 4 minutes and 6 minutes (uniform) at intervals of 6 minutes each continuously from the low concentration end, and the binding behaviors were measured.

As the specific binding behavior data for the antigen peptide, the binding behavior data was obtained for a non-phosphorylated peptide (non pSer413 peptide) as a negative peptide, and this was subtracted from the binding behavior data for the antigen peptide, in order to eliminate the effects of noise produced by the procedure. No binding of non-phosphorylated peptide to antibody was observed at the measuring concentrations. The specific binding behavior data was fitted with Single Cycle Kinetics 1:1, Binding with drift model, using Biacore analysis software (BIA evaluation: Single Cycle Kinetics Analysis, code# AP-4000-01), and the kinetic association rate (Ka) and dissociation rate (Kd) were simultaneously obtained (Karlsson, R., Katsamba, P. S., Nordin, H., Pol, E. and Myszka, D. G. (2006). "Analyzing a kinetic titration series using affinity biosensors." Anal.Biochem. 349(1): 136-47). The equilibrium dissociation constant (KD) value, as the affinity measurement for the Ta15 Series antibodies, was calculated as Kd/Ka.

### <Results>

**[Table 3]**

| | Ka (Ms⁻¹) | Kd (s⁻¹) | KD (M) |
|---|---|---|---|
| 1501 | 5. 64x10⁴ | 1. 05x10⁻³ | 1. 86x10⁻⁸ |
| 1502 | 1. 12x10⁵ | 4. 70x10⁻⁴ | 4. 20x10⁻⁹ |
| 1505 | 1.29x10⁵ | 4.99x10⁻⁴ | 3.87x10⁻⁹ |
| 1506 | 1. 99x10⁵ | 1. 94x10⁻³ | 9.75x10⁻⁹ |
| 1507 | 1. 27x10⁵ | 5.33x10⁻⁴ | 4.20x10⁻⁹ |
| 1508 | 1.45x10⁵ | 9. 80x10⁻⁴ | 6.76x10⁻⁹ |
| 1509 | 8.71x10⁴ | 1.25x10⁻³ | 1. 44x10⁻⁸ |

Of the Ta15 Series antibodies, the Ta1505 antibody with the strongest affinity for the antigen peptide was used in a behavioral test using memory impaired mice.

### [Reference Example 4] Preparation of monoclonal antibody recognizing pSer396 and/or pSer404 peptide

As antigen there was used a synthetic peptide (SEQ ID NO: 47, synthesized by Biosynthesis Co.) having GlyCys attached to the N-terminal site of the sequence from the 379th Arg to the 408th Leu and phosphorylated on the amino acid residues corresponding to Ser at positions 396 and 404 of the amino acid sequence of human tau protein represented by SEQ ID NO: 1 (hereunder, this peptide will be referred to have "pSer396/pSer404 peptide").

### pSer396/pSer404 peptide: N-GlyCys-ArgGluAsnAlaLysAlaLysThrAspHisGlyAlaGluIleValTyrLys(pSer)ProV alValSerGlyAspThr(pSer)ProArgHisLeu-C (SEQ ID NO: 47)

After synthesis of the pSer396/pSer404 peptide, it was purified by HPLC and covalently bonded to maleimide activated KLH (Keyhole Limpet Hemocyanin)(Thermo Scientific). The obtained conjugate was used for immunization of Balb/c mice with approximately 0.04 mg per dose per mouse. The immunization was conducted by intraperitoneal injection into 4 mice at 100 µL each of a mixture of 0.3 ml of conjugate solution (0.77 mg/ml in terms of peptide) and 0.3 mL of Freund's complete adjuvant. Two of the mice were immunized by intraperitoneal immunization (with the same antigen amount, using Freund's incomplete adjuvant) and foot sole immunization (antigen + Titer Max adjuvant), while the other two were immunized only by intraperitoneal immunization, and this was repeated twice at 2 week intervals. The mice immunized by intraperitoneal immunization and foot sole immunization, which had increased serum antibody titers for antigen, were injected with antigen solution (dissolved in saline) through the caudal vein 15 days after the final immunization, and after 3 days the animals were sacrificed and the spleens extracted. Two 18G injection needles were used to break up the spleens, and then the broken-up spleens were gently mashed with a rubber stopper surface. The mashed splenocytes were suspended in approximately 10 mL of cold RPMI 1640 medium, the supernatant was filtered with a 40 µm cell strainer, and the filtrate was collected in a 50 mL tube. The spleen cell debris was further mashed with a rubber stopper surface, and similarly suspended in cold RPMI 1640 medium, filtered, and the filtrate collected. Cold RPMI 1640 medium (or cold PBS) was added to a final volume of 40 mL for the spleen cell suspension. The lymphocyte concentration in the suspension was measured with a hemacytometer, and lymphocytes in a final amount corresponding to 2 × 10⁷ cells were transferred to a 50 mL tube. To this there was added an equivalent of 4 × 10⁷ cells of mouse myeloma cell line P3 ·U1 in the logarithmic growth stage, that had been cultured in culture solution B (RPMI 1640 + 10% fetal bovine serum + 2 mM glutamine + 100 µg/mL streptomycin + 100 unit/mL penicillin), and after centrifugation at 1500 rpm for 5 minutes, the supernatant was discarded. The cell pellet was thoroughly dispersed by tapping the test tube. A 0.5 mL PEG solution was added thereto and the cells were gently suspended. After 1 minute, 0.5 mL of PEG solution was slowly added dropwise over a period of 1 minute, 1.0 mL of RPMI 1640 was slowly added dropwise over a period of 1 minute, and then 2 mL of RPMI 1640 was slowly added dropwise over a period of 2 minutes. Next, 4 mL of HAT/GIT culture solution (GIT medium [Nihon Pharmaceutical Co., Ltd.] + 5% fetal bovine serum + 100 µg/mL streptomycin + 100 unit/mL penicillin + 95 µM hypoxanthine + 0.4 µM aminopterin + 16 µM thymidine) was added dropwise over a period of 2 minutes, and then 4 mL of HAT/GIT culture solution was added dropwise over a period of 2 minutes. Finally, HAT/GIT culture solution was added to obtain 40 to 50 mL of cell suspension. After incubation in a thermostatic bath at 37°C for 30 minutes, the suspension was seeded onto 7 culture plates (96-well). The culture plates used were 96-well plates (feeder plates) on which mouse (ICR) abdominal cavity macrophages (feeder cells) had been cultured for several days (> 1 × 10⁵/well). The plates were then cultured for 7 to 10 days at 37°C, 5% CO₂.

Half of the culture solution was replaced with fresh HT culture solution (HAT/GIT culture solution without aminopterin) once every week, and hybridomas were obtained.

Monoclonal antibody screening was carried out in the following manner. The screening antigens used were pSer396/pSer404 peptide-BSA, having BSA conjugated to the N-terminal Cys of pSer396/pSer404 peptide (N-GlyCys-ArgGluAsnAlaLysAlaLysThrAspHisGlyAlaGluIleValTyrLys(pSer)ProV alValSerGlyAspThr(pSer)ProArgHisLeu-C) (SEQ ID NO: 47), and non-phosphorylated peptide-BSA, having BSA conjugated to the N-terminal Cys of a non-phosphorylated peptide (N-GlyCys-ArgGluAsnAlaLysAlaLysThrAspHisGlyAlaGluIleValTyrLysSerProValV alSerGlyAspThrSerProArgHisLeu-C) (SEQ ID NO: 48). The non-phosphorylated peptide-BSA or pSer396/pSer404 peptide-BSA was diluted to 1 µg/mL with PBS, and then dispensed in a 96-well plate at 50 µL/well and allowed to stand overnight at 4°C. After removing the solution, blocking buffer (3% BSA-PBS) was dispensed at 250 µL/well, and the mixture was left to stand at room temperature for 1 hour or longer. The blocking buffer (3% BSA-PBS) was aspirated, the hybridoma supernatant was added at 30-50 µL/well, and reaction was conducted at 25°C for 60 minutes. After rinsing with phosphate-buffered saline containing 0.05% Tween20 (PBS-Tween), a detection reagent (a solution containing added alkaline phosphatase-labeled Protein A, diluted 2000-fold with blocking buffer) was added at 100 µL/well, and reaction was conducted at 25°C for 60 minutes. After rinsing, substrate solution (2.5 mM MgCl₂-containing 0.1 M diethanolamine buffer, with 0.7 to 1.2 mg/mL PNPP [para-nitrophenyl phosphate] added to pH 9.8) was added at 100 µL/well for coloration at 25°C for 60 minutes, after which the absorbance was measured at a measuring wavelength of 405 nm.

The cells were recovered from the wells that had low reactivity with non-phosphorylated peptide-BSA and high reactivity with pSer396/pSer404 peptide-BSA, and were seeded in a 96-well plate (the previous feeder plate) at 1-3 cells/well, for cloning by the limiting dilution method. Clones producing Ta9 antibody (IgG3/κ) were selected from among these. For subsequent analysis, the clones were mass cultured and the antibody was purified with a protein G column.

Upon measuring the KD value of Ta9 antibody for pSer396/pSer404 peptide by the method described in Example 3, it was found to be 1.08 × 10⁻¹⁰, with affinity for peptide that was higher than the Ta15 Series antibodies.

### [Example 5] Behavioral test: Effect of obtained antibodies on memory learning-impaired mice

The effects of administering the following three antibodies on memory learning-impaired mice (Tau-Tg) were examined.
(1) pSer413-recognizing rabbit polyclonal antibody: Tau-Tg (line 609) mice or non-Tg mice (normal control), 9-11 months of age, n = 9-10/group
(2) Ta1505 (pSer413-recognizing monoclonal antibody): Tau-Tg (line 784) mice or non-Tg mice, 14 months of age, n = 9-10/group
(3) Ta9 (pSer396-recognizing monoclonal antibody): Tau-Tg (line 609) mice or non-Tg mice, 14 months of age, n = 9-10/group

For the experiment there were used male hetero mutant Tau-Tg mice (line 609 or line 784), and non-Tg littermates, 9-14 months of age. The groups were divided so that there was no difference in average weight between the groups. The hetero mutant Tau-Tg mice were administered antibody diluted with PBS or citrate buffer (pH 5), once per week, for a total of 5 times, into the abdominal cavity at 1 mg per mouse per administration. As a negative control group, the buffer used to prepare the antibody, or mouse IgG monoclonal antibody with no reactivity for tau, was administered into the abdominal cavity at the same dosage. As a positive control, non-Tg littermates were administered the buffer used to prepare the antibody, into the abdominal cavity at the same dosage.

The structures for groups (1) to (3) are shown below.

### (1) <Mice used>: Mutant Tau-Tg (line 609), 9 to 11 months of age.

### <Group structure>

Evaluation antibody group: 1.6 mg/mL of anti-tau pSer413 rabbit polyclonal antibody in PBS (n = 10)
Control antibody group: PBS (n = 9)
Non-Tg group: PBS (n = 9)

### (2) <Mice used>: Mutant Tau-Tg (line 784), 14 months of age.

### <Group structure>

Evaluation antibody group: 3.84 mg/ml of anti-tau pSer413 mouse monoclonal antibody Ta1505 in 0.1 M citrate buffer (pH 5) (n = 10)
Control antibody group: 4.28 mg/ml of *anti-Pseudomonas aeruginosa* mouse monoclonal antibody 4C10F4 in 0.1 M citrate buffer (pH 5) (n = 9)
Non-Tg group: 0.1 M citrate buffer (pH 5) (n = 9)

### (3) <Mice used>: Mutant Tau-Tg (line 609), 14 months of age.

### <Group structure>

Evaluation antibody group: 2.66 mg/ml of anti-tau pSer396 mouse monoclonal antibody Ta9 in 0.02 M citrate buffer (pH 6) (n = 10)
Control antibody group: 4.50 mg/ml of *anti-Pseudomonas aeruginosa* monoclonal antibody 6F11 in 0.02 M citrate buffer (pH 6) (n = 9)
Non-Tg group: 0.02 M citrate buffer (pH 6) (n = 9)

From Monday of the week following the final administration, a spatial reference memory test was conducted using a Morris water maze (water maze test). On the day following completion of the water maze test, an Open Field apparatus was used to measure the active movement quantity of the mice (Open Field test) .

### <Water maze test: Same for groups (1) to (3)>

Preparation: A black pool with an inner diameter of 100 cm and a height of 45 cm was filled with water to a depth of 16 cm. The water temperature was adjusted to 21-23 degrees, maintaining colorless transparency without coloration with titanium oxide or the like. No chlorine was added, and after every trial day the feces were removed and approximately 10 L of water was replaced.

Trial (Acquisition) test: A 15 cm-high transparent platform was immersed at a position 20 cm from the wall (30 cm from the center). With partitioning into 4 quadrants including the location where the platform was submersed, the mice were introduced randomly from one of the 3 quadrants without the platform. The limit for each test was 60 seconds, with 5 tests being conducted per day. The interval between tests was approximately 5 minutes. The "escape time" to reaching the platform was recorded as data. Mice that did not escape within 60 seconds were directed to the platform by the operator's hand, and the escape time was treated as 60 seconds. The mouse on the platform was removed from the pool after 10 seconds and allowed to behave freely until the next test, drying the body. The results for each mouse on each day were recorded as the average number of seconds for the escape times of the 5 tests.

The acquisition test was completed when the results for the control group (nonTg) stabilized, and a probe test was conducted on the following day.

Probe test: On the day following the last day, the platform was removed from the pool and the free swimming was photographed with a video camera for 60 seconds. The photographed video was observed, and the time during which the freely swimming mice swam in the quadrant that had contained the platform (target quadrant) among the 4 quadrants, was measured and expressed as a percentage of the 60 seconds. During this time, the swimming up to 30 seconds after introduction into the pool was also analyzed.

The significant test for the trial (acquisition) test was done by repeated measure and Fisher's PLSD, and significant test for the probe test was done by Fisher's PLSD.

### <Open Field test: Same for groups (1) to (3)>

Apparatus: A 5 mm-thick transparent acrylic board was used to create a 30 × 30 × 30 cm square box, and it was stored in a soundproof environment. A 40W incandescent lamp was placed over the storage environment and the floor face was irradiated with an illuminance of 110 lux.

Behavior quantity: (i) Infrared beams were set on the outside of the sides of the acrylic box at locations 1.5 cm from the floor surface, at a spacing of 10 cm. When the mouse continuously blocked 2 beams the locomotion was detected, and was counted.
(ii) Infrared beam surfaces were provided on locations of two opposite sides, 3.5 cm from the floor surface. When the mouse blocked a portion of the beam surface, rearing was detected and counted.

Test: Each mouse was subjected to a single 20-minute session. The mouse was introduced into the center section of the acrylic box and, upon rapidly closing the door to create a soundproof environment, the session was initiated.

The first 10 minutes of the session were free activity under light conditions, while for the last 10 minutes, illumination was ceased for free activity under dark conditions.

Analysis: The behavior quantity of each mouse each minute was indicated on a line graph, for locomotion and rearing.

Normal visual perception was defined as reaction to the change in environment to dark conditions 10 minutes after the start of the session, whereby the mouse exhibited a change in behavior quantity.

The each behavior quantity under light and dark conditions, and the total behavior quantity during 20 minutes, were represented in a bar graph.

A significant test was conducted between the groups, for total behavior quantity of locomotion and rearing.

Locomotion is a major spontaneous behavior quantity while rearing is a major exploratory behavior, but in fact both indicators are mutually influential (example: even when locomotion appears to be reduced, if rearing is increased during that time then the behavior quantity cannot be said to be reduced).

### <Immunohistochemistry test: only for group (2)>

Upon completion of the behavioral test, 5 mice were selected from each group and were perfusion-fixed with formalin. After embedding in paraffin, a thin slice was prepared from the brain using a microtome and treated 4 times with xylene and ethanol every 10 minutes, as deparaffinization treatment. It was then boiled for 30 minutes in citric acid buffer (pH 6) (antigen activating treatment) and returned to room temperature, after which it was rinsed twice with Tris buffer-physiological saline (TS) for 10 minutes. After blocking for 60 minutes at room temperature with TS containing 20% bovine serum, mouse antihuman synaptophysin antibody (SVP-38 diluted 200-fold with TS containing 10% bovine serum, by Sigma) was mounted and treatment was conducted overnight at 4°C. After rinsing twice with TS for 10 minutes, FITC-labeled anti-mouse IgG antibody (the antibody diluted 20-fold with TS containing 10% bovine serum, by Vector) was mounted, and treatment was conducted at room temperature for 60 minutes. After rinsing twice with TS for 10 minutes, VECTASHIELD (Vector) was mounted, and observation was made with a microscope. The fluorescence intensity was quantified and digitized with NIH imageJ, and expressed in arbitrary units.

### <Results>

### 1. Effects of each antibody administration on memory impairment

### (1) pSer413 rabbit polyclonal antibody

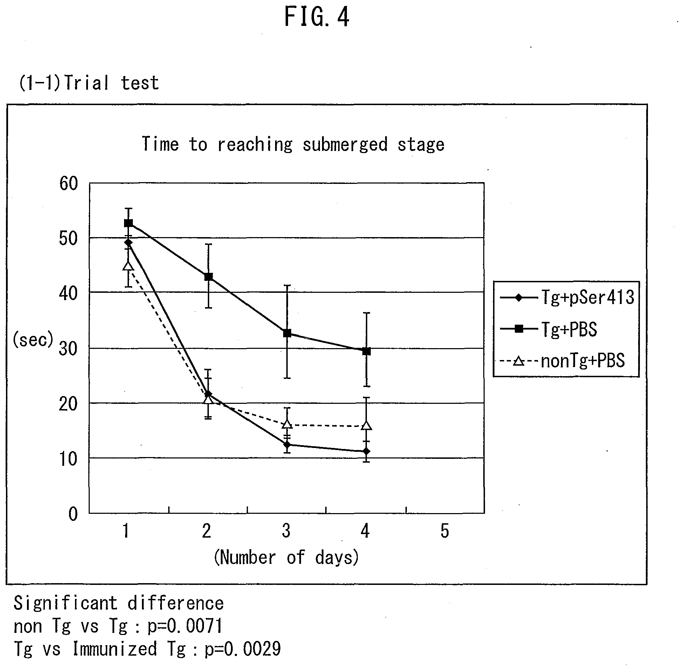

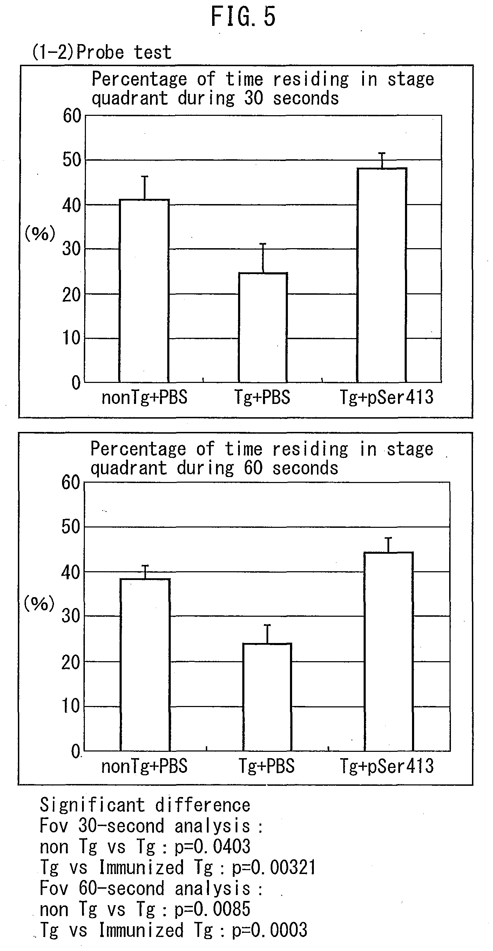

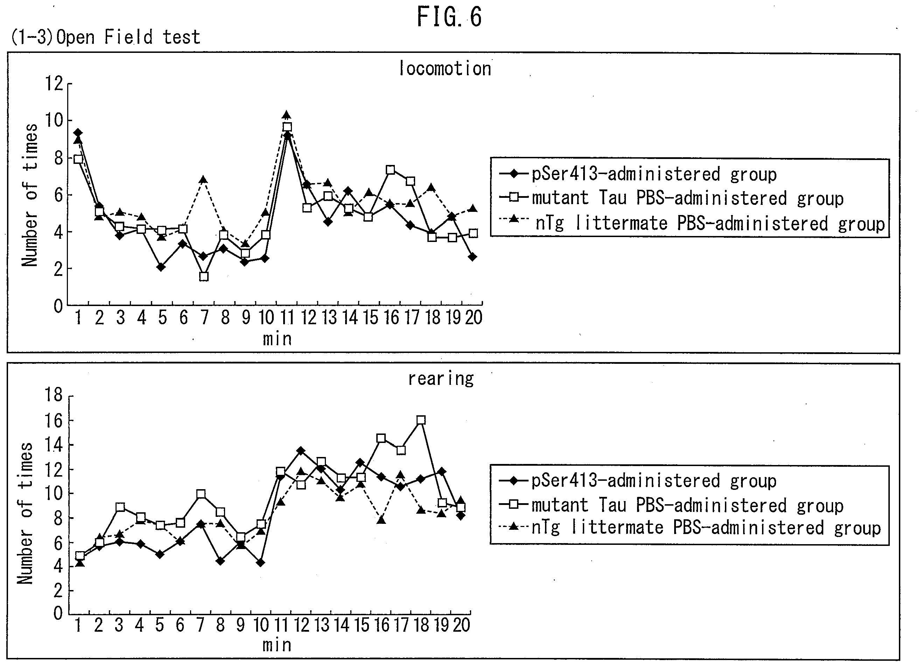

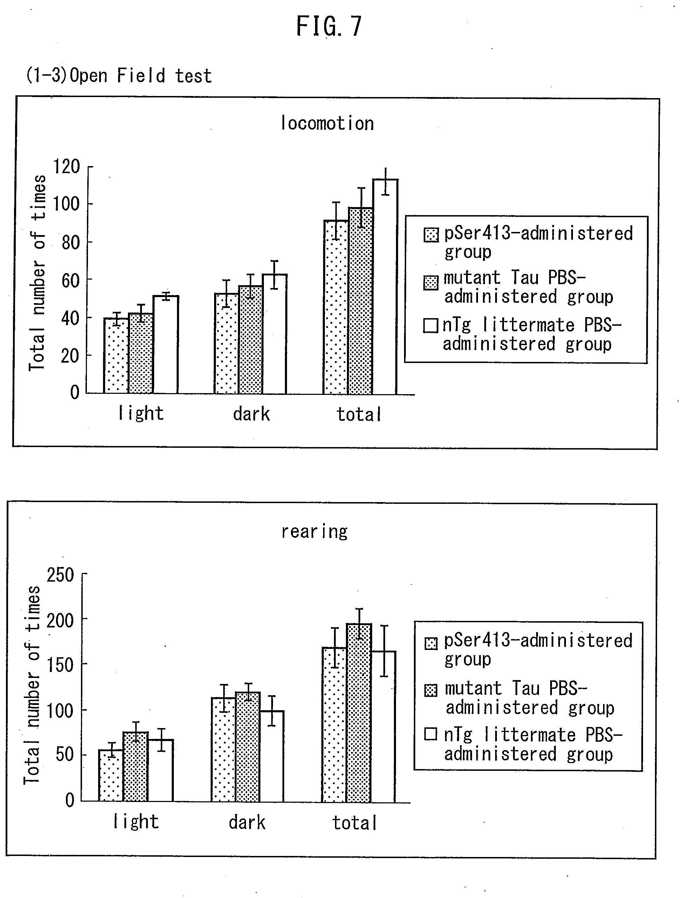

The results for (1-1) to (1-3) are shown in Fig. 4 to Fig. 7. To summarize, passive immunization with anti-pSer413 polyclonal antibody notably improved memory impairment in the model mice (Tau-Tg) to the same level as non-Tg. In (1-3), no significant difference in active movement and visual perception was seen between the groups.

### (2) pSer413 mouse monoclonal antibody (1505 antibody)

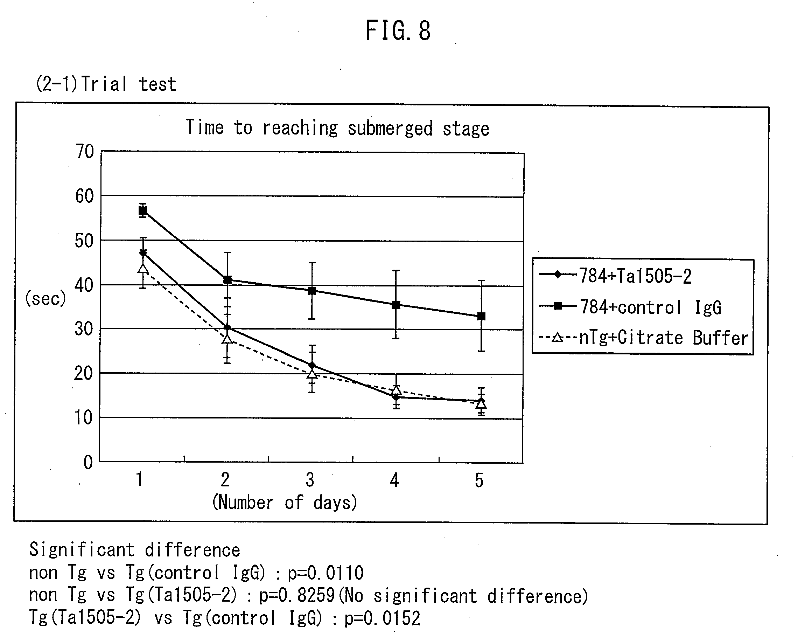

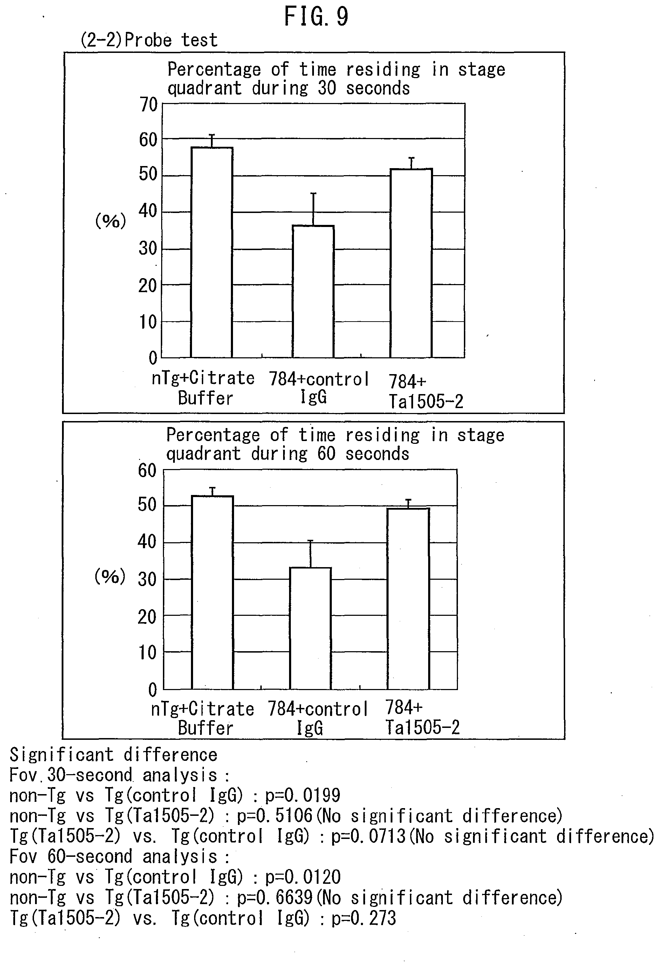

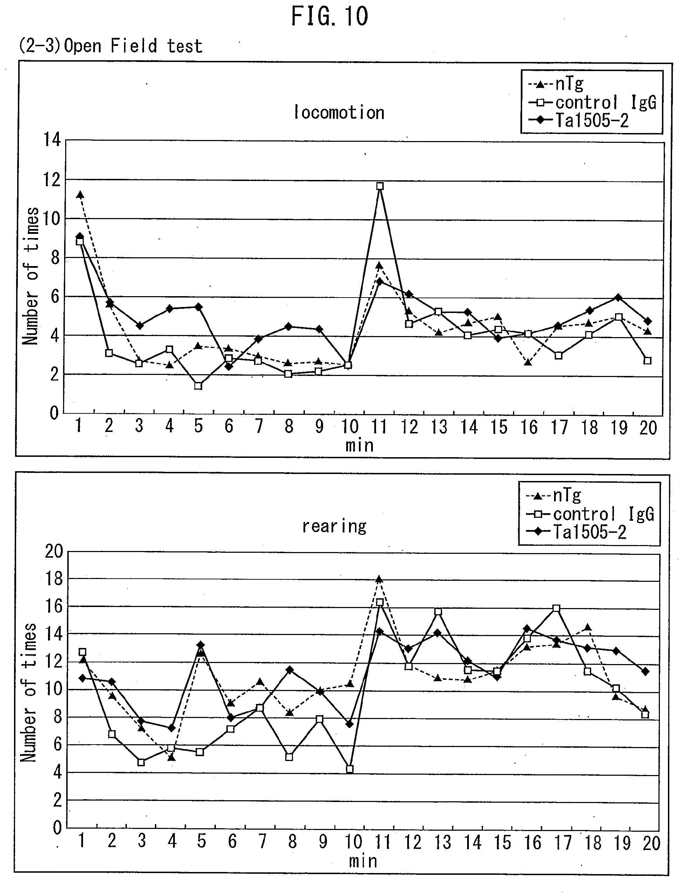

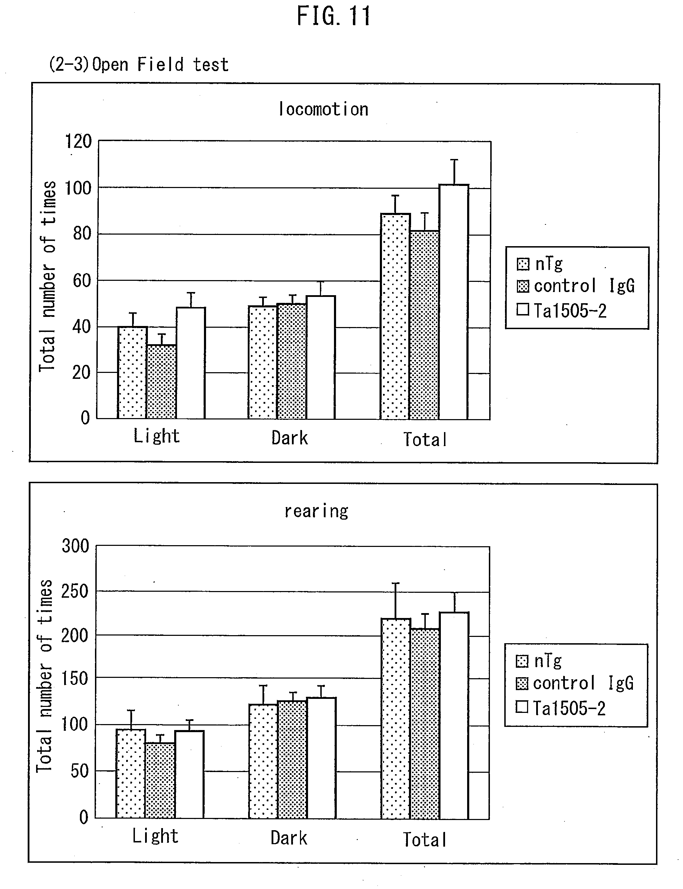

The results for (2-1) to (2-3) are shown in Fig. 8 to Fig. 11. To summarize, passive immunization with anti-pSer413 monoclonal antibody notably improved memory impairment in the model mice to the same level as non-Tg. In (2-3), no significant difference in active movement and visual perception was seen between the groups.

Based on the results of (2-1) and (2-2), the pSer413 epitope was concluded to be a satisfactory epitope as a target of passive immunization therapy, for both polyclonal antibodies and monoclonal antibodies.

### (3) pSer396 mouse monoclonal antibody (Ta9 antibody)

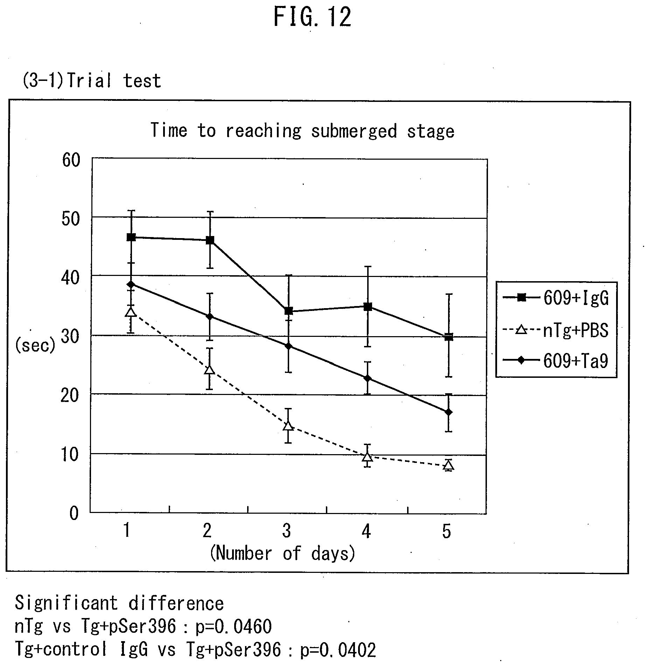

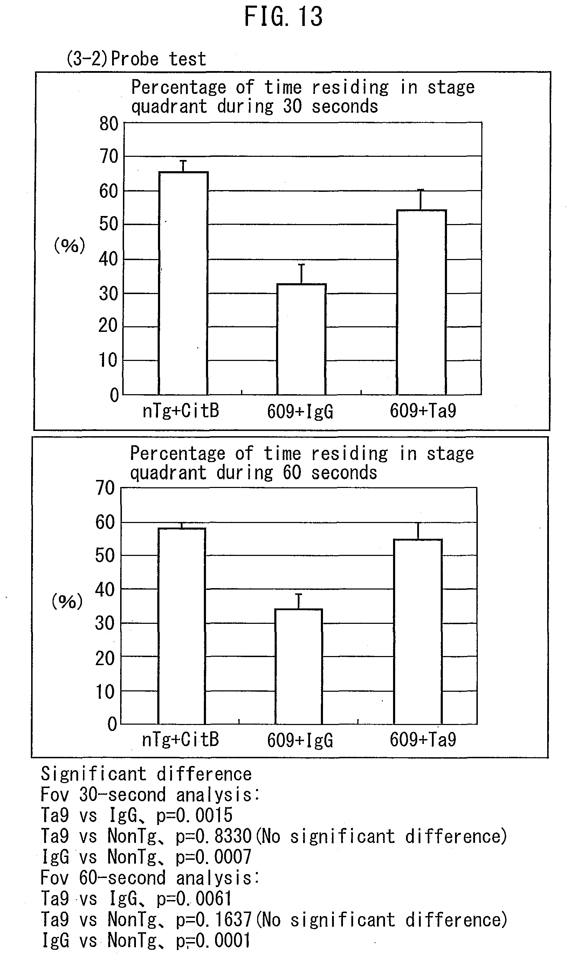

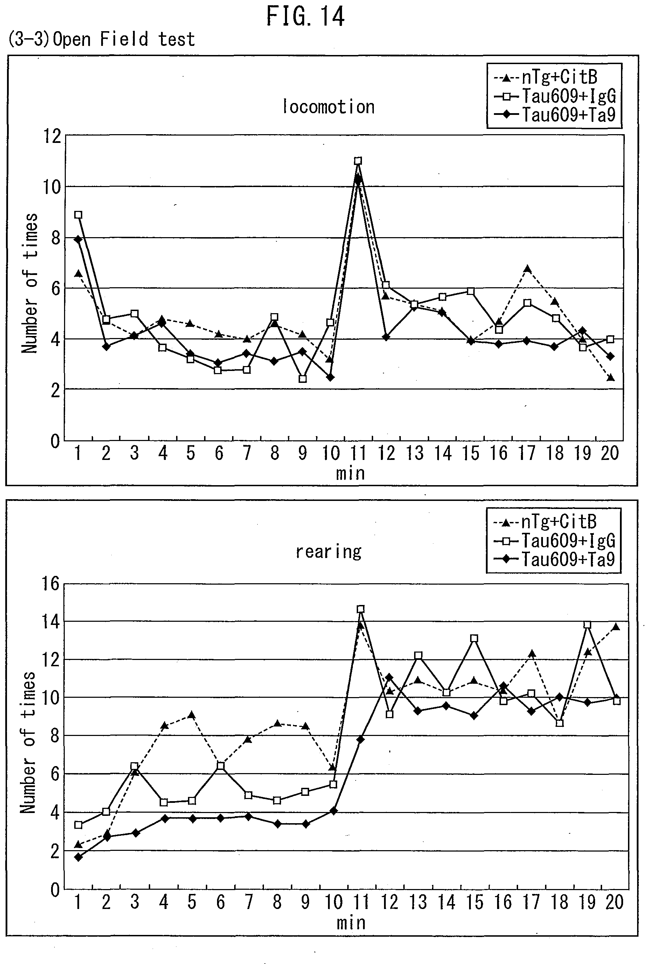

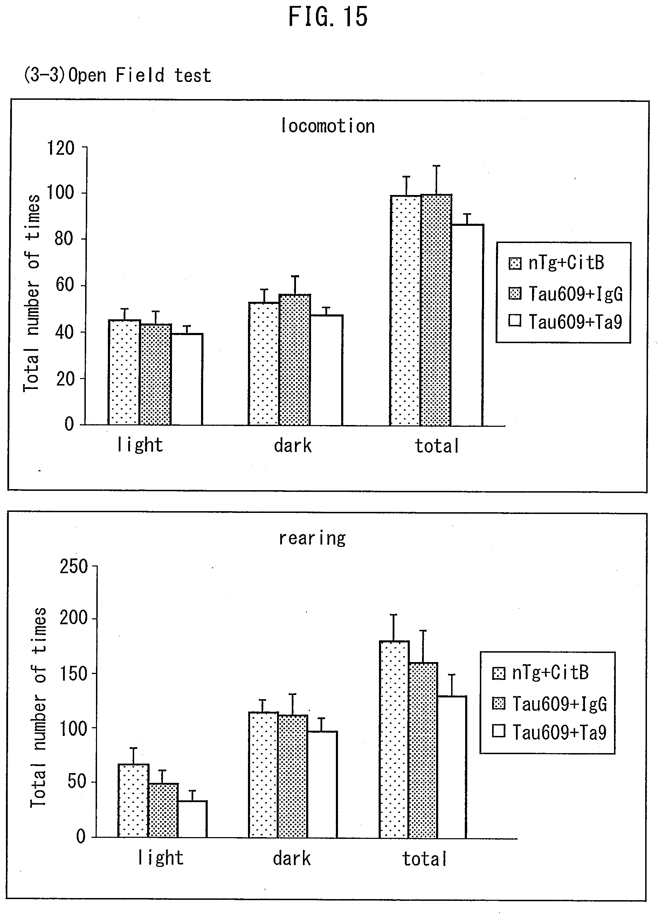

The results for (3-1) to (3-3) are shown in Fig. 12 to Fig. 15. To summarize, Ta9 administration significantly improved memory impairment to the same level as non-Tg. In (3-3), no significant difference in active movement and visual perception was seen between the groups.

When Ta9 is compared with Ta1505, however, its drug effect was found to be weaker. Although the antigen affinity was Ta9 > Ta1505 (see Examples 2 and 3), the drug effect was Ta9 < Ta1505 (Example 5), suggesting a significant difference in drug effect due to differences in the phosphorylation epitope.

### [Example 6] Effects of Ta1505 antibody administration on neural function

The effects of Ta1505 antibody administration on levels of synaptophysin, known as a marker reflecting neural function, was examined by immunostaining of anti-synaptophysin antibody in the hippocampal CA3 region, which is a center of memory. The fluorescence intensity was quantified by NIH-Image J.

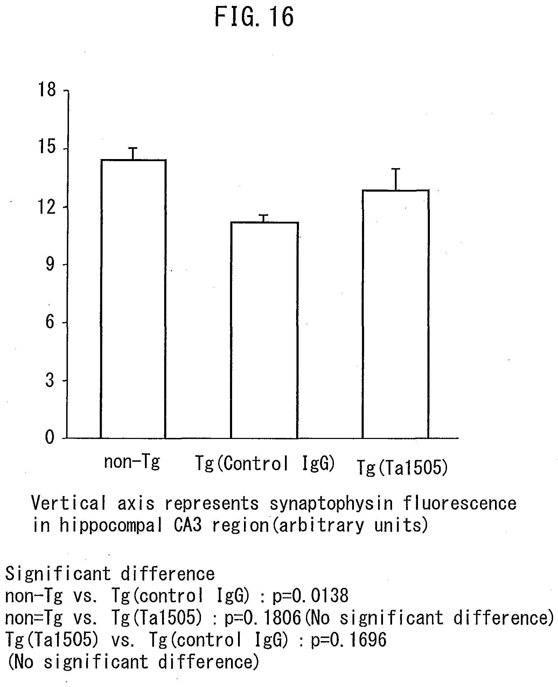

The results are shown in Fig. 16. With administration of Ta1505 antibody there was observed recovery in hippocampal synaptophysin levels, although not to a significant degree.

### [Example 7] Nucleotide sequencing of Ta15 Series monoclonal antibody cDNA

### (1) Purification of hybridoma total RNA

Hybridomas producing different monoclonal antibodies were cultured, and 1 mL of ISOGEN was used per well of a 6-well plate to lyse the cells. After adding 0.2 mL of chloroform to the lysate and mixing with a vortex, it was allowed to stand at room temperature for 2 to 3 minutes. Centrifugation was performed at 12,000 rpm, 4°C for 10 minutes, and the upper layer was transferred to a new tube. After then adding 0.5 mL of isopropyl alcohol and mixing, the mixture was allowed to stand at room temperature for 10 minutes. It was then centrifuged at 15,000 rpm, 4°C for 15 minutes to precipitate the total RNA. After then adding 1 mL of 75% ethanol to the pellet and thoroughly mixing, it was centrifuged at 10,000 rpm, 4°C for 5 minutes. The pellet was air-dried, dissolved in DNase/RNase-free water, and stored at -80°C.

### (2) Obtaining of H chain and L chain cDNA by 5'-RACE and 3'-RACE and nucleotide sequencing of these cDNAs

Primers were synthesized for 5'-RACE (Rapid Amplification of cDNA Ends) and 3'-RACE, based on the known cDNA sequences of the constant regions of the mouse IgG2a and IgG2b H chains. Primers were similarly synthesized for 5'-RACE (Rapid Amplification of cDNA Ends) and 3'-RACE, based on the cDNA sequence of the constant region of the mouse L chain.

Separately, 1 µg of total RNA obtained from the hybridomas was used for synthesis of cDNA for 5'-RACE and 3'-RACE using a SMART-RACE Kit (product of Clontech), and 5'-RACE and 3'-RACE were performed. PCR reaction was conducted using Advantage 2 Polymerase Mix (Clontech), according to the manufacturer's protocol. The PCR products obtained by 5'-RACE and 3'-RACE were electrophoresed on agarose gel, and the main amplified DNA fragment was cut out from the agarose gel, inserted into a TA cloning vector (Invitrogen) and used to transform *E. coli,* obtaining several clones. Plasmids were prepared from the transformants by an established method, and the nucleotide sequence of the inserted DNA fragment was determined.

### (3) Obtaining of full-length cDNA of H chain and L chain and nucleotide sequencing of these cDNAs

The cDNA sequences coding for the N-terminus and C-terminus of the H chain and L chain were determined based on nucleotide sequence information, and forward and reverse primers were designed for amplification of the full-length sequence. These primers were used for amplification of the full lengths of the H chain and L chain using Prime STAR MaxPCR (TaKaRa), and the PCR fragments were cloned in pEF4 vector. This was used for final determination of the full-length cDNA sequences.

Translation to amino acid sequences was carried out based on the obtained nucleotide sequence information, and IgBLAST (NCBI, http://www.ncbi.nlm.nih.gov/igblast/) was used to identify the CDR regions by the method of Kabat (Kabat, E.A. and Wu, T.T., J. Immunol., 147, 1709-1719, 1991).

The sequences of the obtained CDR regions are listed from SEQ ID NO: 14 onward.

Information relating to the CDR sequences of antibodies that specifically recognize pSer413 was obtained from homology with these sequences (Tables 4 to 6).

**[Table 4]**

| Monoclonal antibody CDR-H1, CDR-H2 and CDR-H3 amino acid sequences | | | |
|---|---|---|---|
| Monoclonal antibody clone | CDR-H1 | CDR-H2 | CDR-H3 |
| Ta1501 | SEQ ID NO: 7 | SEQ ID NO: 9 | SEQ ID NO: 13 |
| Ta1502 | SEQ ID NO: 8 | SEQ ID NO: 9 | SEQ ID NO: 13 |
| Ta1505 | SEQ ID NO: 8 | SEQ ID NO: 9 | SEQ ID NO: 13 |
| Ta1506 | SEQ ID NO: 7 | SEQ ID NO: 10 | SEQ ID NO: 13 |
| Ta1507 | SEQ ID NO: 8 | SEQ ID NO: 12 | SEQ ID NO: 13 |
| Ta1508 | SEQ ID NO: 7 | SEQ ID NO: 10 | SEQ ID NO: 13 |
| Ta1509 | SEQ ID NO: 7 | SEQ ID NO: 11 | SEQ ID NO: 13 |

**[Table 5]**

| Monoclonal antibody CDR-L1, CDR-L2 and CDR-L3 amino acid sequences | | | |
|---|---|---|---|
| Monoclonal antibody clone | CDR-L1 | CDR-L2 | CDR-L3 |
| Ta1501 | SEQ ID NO: 14 | SEQ ID NO: 16 | SEQ ID NO: 17 |
| Ta1502 | SEQ ID NO: 14 | SEQ ID NO: 16 | SEQ ID NO: 17 |
| Ta1505 | SEQ ID NO: 14 | SEQ ID NO: 16 | SEQ ID NO: 17 |
| Ta1506 | SEQ ID NO: 14 | SEQ ID NO: 16 | SEQ ID NO: 17 |
| Ta1507 | SEQ ID NO: 14 | SEQ ID NO: 16 | SEQ ID NO: 17 |
| Ta1508 | SEQ ID NO: 14 | SEQ ID NO: 16 | SEQ ID NO: 17 |
| Ta1509 | SEQ ID NO: 15 | SEQ ID NO: 16 | SEQ ID NO: 17 |

**[Table 6]**

| Monoclonal antibody VH and VL amino acid sequences | | |
|---|---|---|
| Monoclonal antibody clone | VH | VL |
| Ta1501 | SEQ ID NO: 18 | SEQ ID NO: 25 |
| Ta1502 | SEQ ID NO: 19 | SEQ ID NO: 26 |
| Ta1505 | SEQ ID NO: 20 | SEQ ID NO: 26 |
| Ta1506 | SEQ ID NO: 21 | SEQ ID NO: 27 |
| Ta1507 | SEQ ID NO: 22 | SEQ ID NO: 28 |
| Ta1508 | SEQ ID NO: 23 | SEQ ID NO: 29 |
| Ta1509 | SEQ ID NO: 24 | SEQ ID NO: 30 |

### [Example 8] Behavioral test: Effect of obtained antibodies on memory learning-impaired mice

The effect of administering the following two antibodies on memory learning-impaired mice was examined, with a dosage of 0.1 mg.
(4) Ta1505 (pSer413-recognizing monoclonal antibody): Tau-Tg (line 784) mice or non-Tg mice, 10 months of age, n = 9-10/group
(5) Ta9 (pSer396-recognizing monoclonal antibody): Tau-Tg (line 784) mice or non-Tg mice, 11 months of age, n = 8-10/group

For the experiment there were used male hetero mutant Tau-Tg mice (line 784), and their non-Tg littermates, 10-11 months of age. The groups were divided so that there was no difference in average weight between the groups. The hetero mutant Tau-Tg mice were administered antibody diluted with PBS, once per week, for a total of 5 times, into the abdominal cavity at 0.1 mg per mouse per administration. As a negative control group, the PBS used to prepare the antibody, or mouse IgG monoclonal antibody with no reactivity for tau, was administered into the abdominal cavity at the same dosage. As a positive control, non-Tg littermates were administered the PBS used to prepare the antibody, into the abdominal cavity at the same dosage.

The structures for groups (4) to (5) are shown below.

### (4) <Mice used>: Mutant Tau-Tg (line 784), 10 months of age.

### <Group structure>

Evaluation antibody group: 0.25 mg/ml of anti-tau pSer413 mouse monoclonal antibody Ta1505 in PBS (n = 10)
Control antibody group: 0.25 mg/ml of *anti-Pseudomonas aeruginosa* mouse monoclonal antibody 4C10F4 in PBS (n = 10)
Non-Tg group: PBS (n = 9)

### (5) <Mice used>: Mutant Tau-Tg (line 784), 11 months of age.

### <Group structure>

Evaluation antibody group: 0.25 mg/ml of anti-tau pSer396 mouse monoclonal antibody Ta9 in PBS (n = 10)
Control antibody group: 0.25 mg/ml of *anti-Pseudomonas aeruginosa* monoclonal antibody 6F11 in PBS (n = 8)
Non-Tg group: PBS (n = 8)

From Monday of the week following the final administration, a spatial reference memory test was conducted using a Morris water maze (water maze test).The water maze test was conducted in the same manner as Example 5.

### <Results>

### 1. Effects of each antibody administration on memory impairment

### (4) pSer413 mouse monoclonal antibody (1505 antibody)

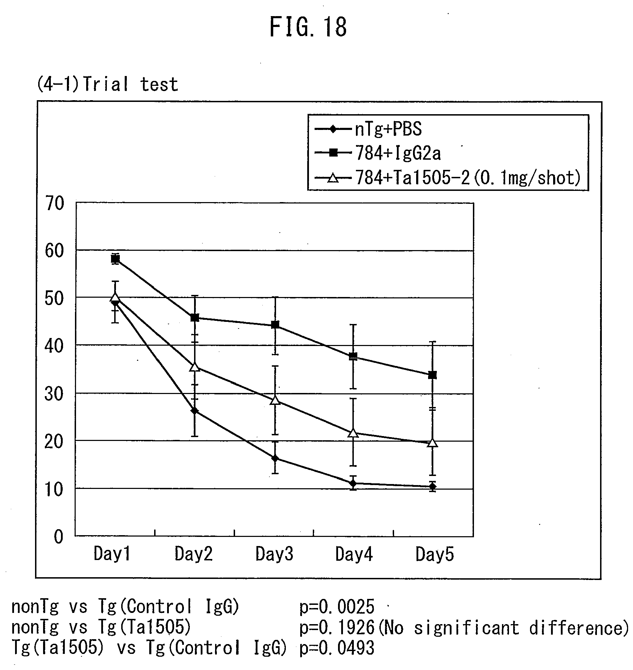

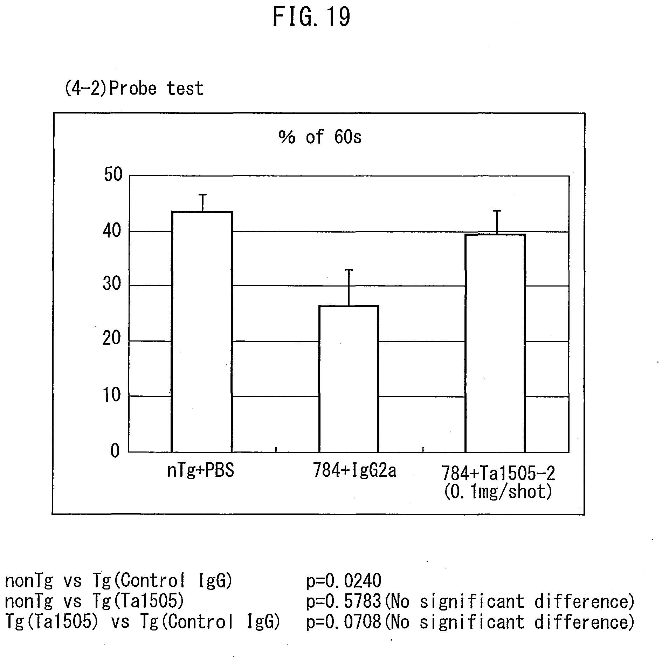

The results for the Trial test (4-1) and Probe test (4-2) are shown in Fig. 18 and Fig. 19. To summarize, passive immunization with anti-pSer413 monoclonal antibody improved memory impairment in the model mice to a level of 50% or greater compared to non-Tg.

The results of (4-1) and (4-2) confirmed a drug effect for pSer413 epitope monoclonal antibody even at a dose of 0.1 mg.

### (5) pSer396 mouse monoclonal antibody (Ta9 antibody)

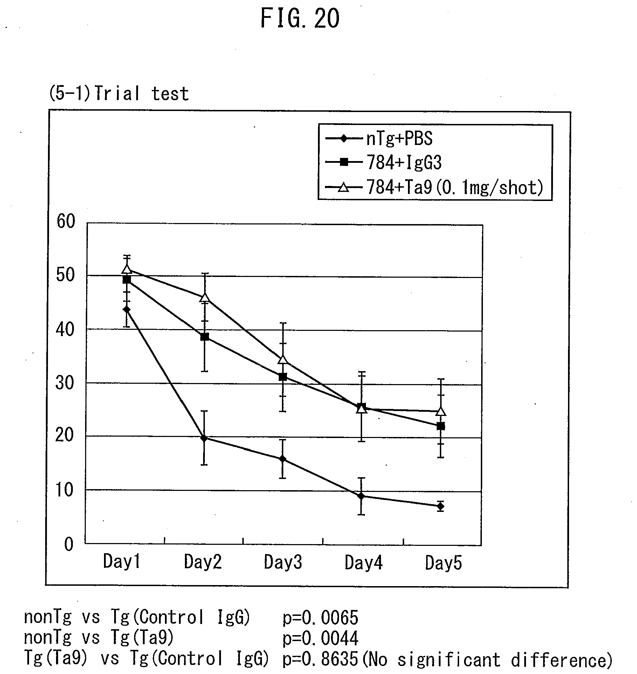

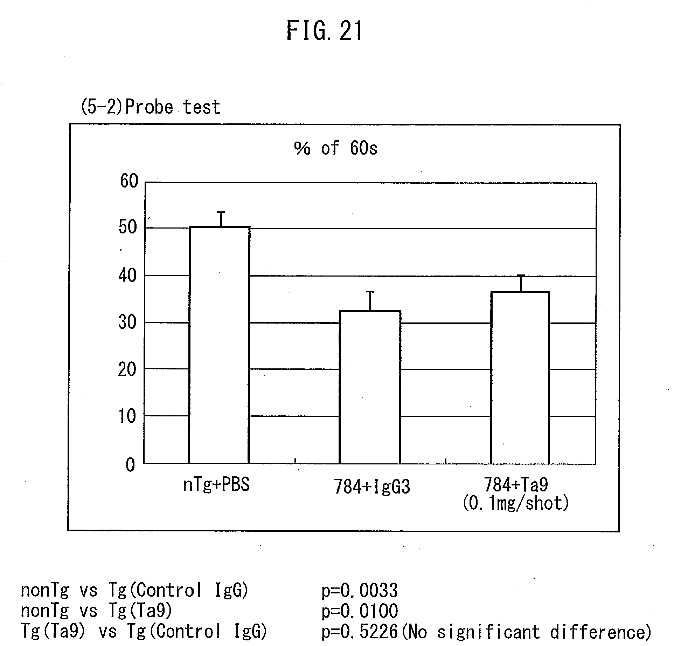

The results for the Trial test (5-1) and Probe test (5-2) are shown in Fig. 20 and Fig. 21. To summarize, memory impairment was not improved with Ta9 administration at a dosage of 0.1 mg.

These tests even more clearly suggest a difference in drug effect at a dosage of 0.1 mg, due to differences in the phosphorylation epitope.

Administration of antibody at 0.1 mg per mouse corresponds to administration at a dose of approximately 2.5 mg/kg.

### [Example 9] Change in level of phosphorylated Tau in brain by Ta1505 antibody administration

The effect of Ta1505 antibody administration on the levels of phosphorylated Tau in the brains of Tau-Tg mice was examined by immunohistostaining with pSer413-recognizing Ta1505 antibody and AT8 antibody (PHF-recognizing pSer202/pThr205 epitope), in the hippocampal region, which are centers of memory.

Upon completion of the behavioral test, 5 mice were selected from each group and perfusion-fixed with 4% paraformaldehyde/PBS. The brains were removed and embedded in paraffin, and 5 µm thin slices were prepared with a microtome. After treatment 4 times with xylene and ethanol for 10 minutes each time, deparaffinization treatment was performed and the slices were subjected to boiling treatment (antigen activating treatment) for 10 minutes at pH 2, room temperature. After restoration to room temperature, there were rinsed twice with Tris-HCl physiological saline (TS) for 10 minutes. Blocking was then performed for 60 minutes at room temperature using 20% bovine serum-containing TS.

Anti-tau antibodies (Ta1505, AT8) diluted to 1 µg/mL with 10% bovine serum-containing TS were mounted, for treatment overnight at 4°C. After rinsing twice with TS for 10 minutes, biotin-labeled anti-mouse antibody (Vector Co.) diluted 500-fold with TS containing 10% bovine serum was mounted, for treatment at room temperature for 60 minutes.

After rinsing twice with TS for 10 minutes, HRP-labeled ABC solution (Vector Co.) was mounted for reaction at room temperature for 30 minutes, and after additional rinsing, coloring was performed with diaminobenzidine (DAB). This was encapsulated with Entellan (Merck) and observed and photographed.

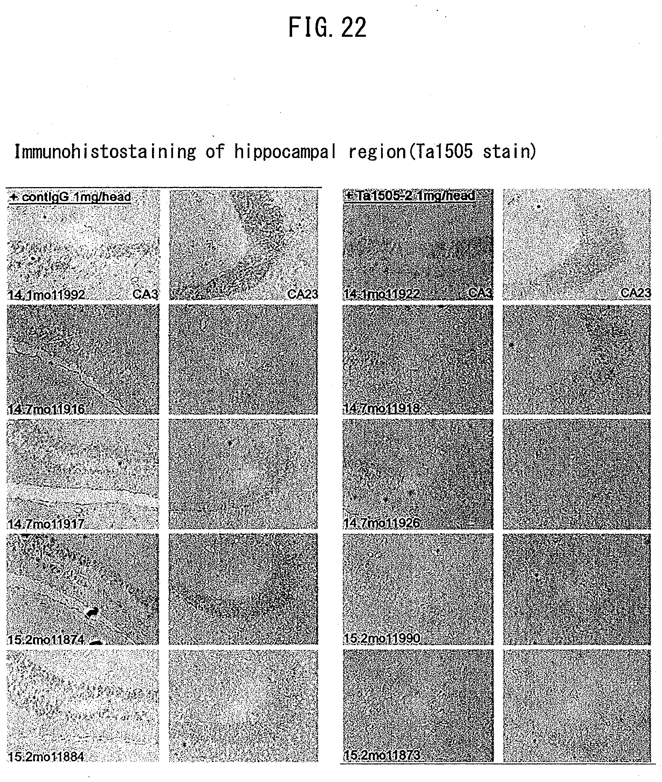

The results of immunohistostaining with Ta1505 are shown in Fig. 22 to Fig. 24.

As seen in Fig. 22, Ta1505-positive Tau staining was observed in the hippocampus CA3 region (first column from left) and the hippocampus CA23 region (second column from left) in 5 individuals of the control IgG-administered group, but the staining levels in the hippocampus CA3 region (third column from left) and hippocampus CA23 region (fourth column from left) in 5 individuals of the Ta1505-administered group were clearly lower than the control IgG-administered group. This confirmed that Ta1505 administration reduced Ta1505-positive Tau, i.e. Ser413-phosphorylated Tau, in the hippocampus CA3 region and hippocampus CA23 region. More specifically, the control IgG group showed accumulated fine brown points, staining in a thick line from left to right in CA3. With CA23, a thick curve stained from the top right toward the left side. The Ta1505-administered group had very fine thin brown points, with a thick staining line from the top left toward the bottom right in CA3. With CA23, a thick curve stained from the top right toward the left side. Virtually no staining was seen in four CA3 regions and three CA23 regions.

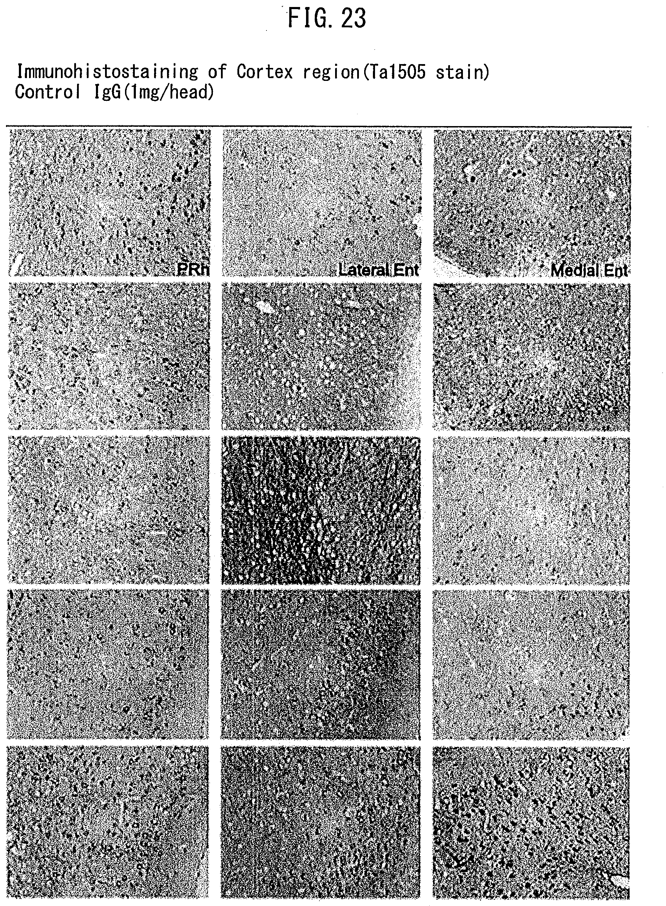

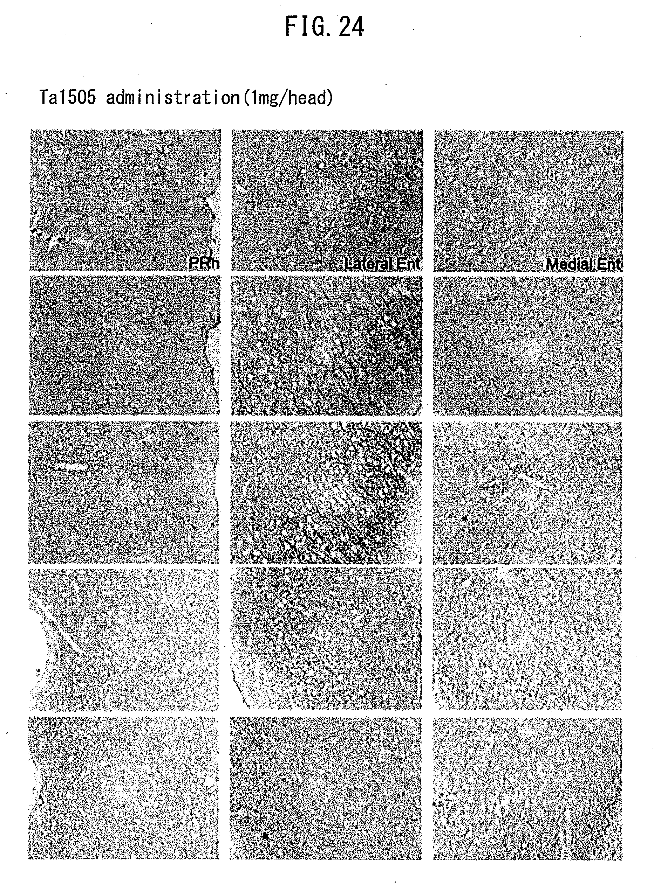

In Fig. 23 and Fig. 24, AT8-positive Tau staining was seen in the cortex regions (Perirhinal Cortex (first column from left in Fig. 23), Lateral Entorhinal Cortex (second column from left in Fig. 23), and Medial Entorhinal Cortex (third column from left in Fig. 23)), for five individuals in the control IgG-administered group.(Staining as brown spots in Fig. 23.)

The staining levels in the cortex regions (Perirhinal Cortex (first column from left in Fig. 24), Lateral Entorhinal Cortex (second column from left in Fig. 24) and Medial Entorhinal Cortex (third column from left in Fig. 24)), were clearly reduced below the control IgG-administered group, for five individuals in the Ta1505-administered group, to a level which exhibited no staining.

This confirmed that Ta1505 administration reduced Ta1505-positive Tau, i.e. Ser413 phosphorylated Tau, in the cortex regions (Perirhinal Cortex, Lateral Entorhinal Cortex, Medial Enotrhinal Cortex).

Brown spot staining is present in Fig. 23, but virtually no brown spot staining can be seen in Fig. 24.

Ta1505 antibody administration was confirmed to reduce Ser413 phosphorylated Tau levels in the brain (= hippocampus CA3 region, hippocampus CA23 region, PRh, Ent (Lateral, Medial)).
PRh = Perirhinal Cortex
Ent = Entorhinal Cortex

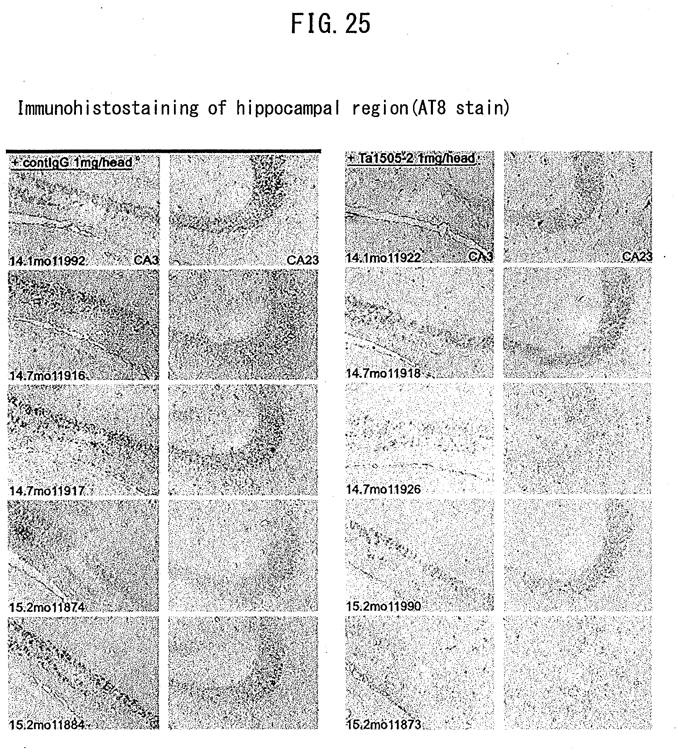

The results of immunohistostaining with AT8 are shown in Fig. 25 to Fig. 27.

As seen in Fig. 25, AT8-positive Tau staining was observed in the hippocampus CA3 region (first column from left) and the hippocampus CA23 region (second column from left) in 5 individuals of the control IgG-administered group, but the staining levels in the hippocampus CA3 region (third column from left) and hippocampus CA23 region (fourth column from left) in 5 individuals of the Ta1505-administered group were clearly lower than the control IgG-administered group. This confirmed that Ta1505 administration reduced AT8-positive Tau, i.e. Ser202/Thr205-phosphorylated Tau, in the hippocampus CA3 region and hippocampus CA23 region. More specifically, in Fig. 25, the control IgG group shows accumulated fine brown points, staining in a thick line from the top left to bottom right, with CA3. With CA23, a thick curve stained from the top right toward the left side. The Ta1505-administered group had very fine thin brown points, with a thick staining line from the top left toward the bottom right with CA3. With CA23, a thick curve stained from the top right toward the left side.

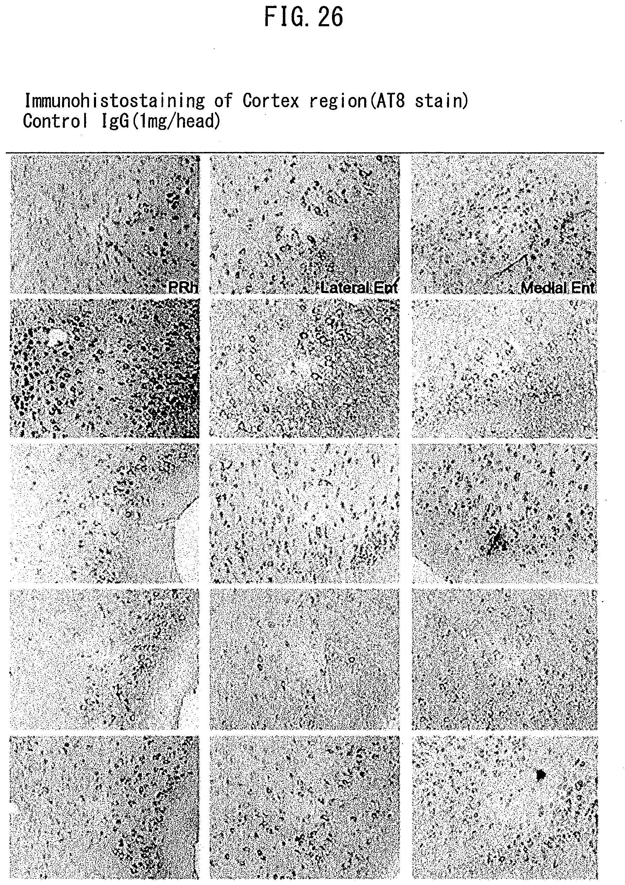

In Fig. 26, AT8-positive Tau staining is seen in the cortex regions (Perirhinal Cortex (first column from left in Fig. 26), Lateral Entorhinal Cortex (second column from left in Fig. 26), and Medial Entorhinal Cortex (third column from left in Fig. 26)), for five individuals in the control IgG-administered group.(Staining as brown spots in Fig. 26.)

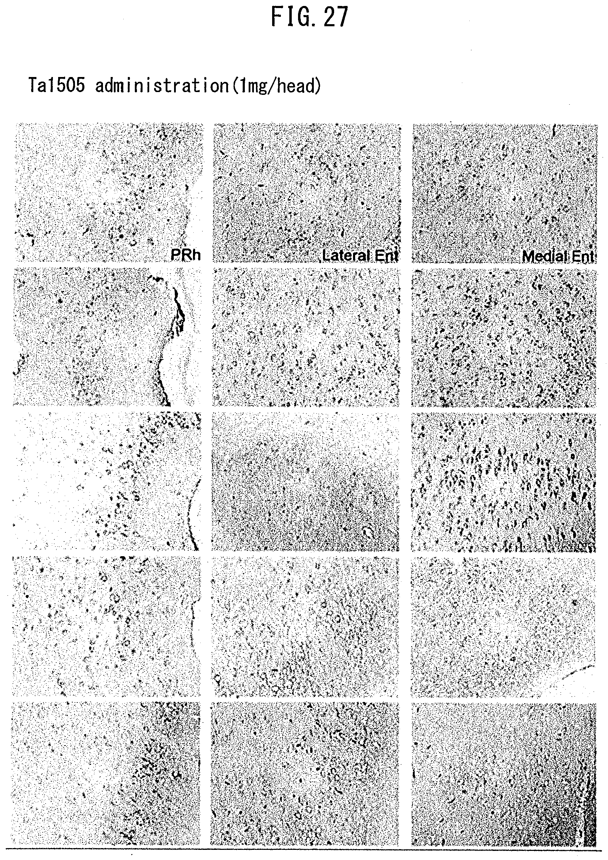

In Fig. 27, the staining levels in the cortex regions (Perirhinal Cortex (first column from left in Fig. 27), Lateral Entorhinal Cortex (second column from left in Fig. 27) and Medial Entorhinal Cortex (third column from left in Fig. 27)) are clearly reduced below the control IgG-administered group, for five individuals in the Ta1505-administered group.(Staining as thin brown spots in Fig. 27.)

This confirmed that Ta1505 administration reduced AT8-positive Tau, i.e. Ser202/Thr205 phosphorylated Tau, in the cortex regions (Perirhinal Cortex, Lateral Entorhinal Cortex, Medial Entorhinal Cortex).

Ta1505 antibody administration was confirmed to tend to reduce AT8-recognizing Ser202/Thr205 phosphorylated Tau levels in the brain (= hippocampus CA3 region, hippocampus CA23 region, PRh, Ent (Lateral, Medial)).

This result provides support that antibody administration ameliorates pathology in the brain and has an improving effect for memory impairment. The results indicate that intraperitoneally administered antibody acts in the brain.

### [Example 10] Effect of Ta1505 antibody administration on Tau levels in the brain

Using AT8 antibody, which is thought to recognize hyperphosphorylated Tau present in PHF (pSer202/pThr205-recognizing mouse monoclonal antibody, Innax Co.), G2 (antihuman specific N-terminal region-recognizing antibody: rabbit polyclonal antibody), PHF1 (pSer396/pSer404-recognizing mouse monoclonal antibody, thought to recognize hyperphosphorylated Tau present in PHF) and Ta1505, the effect of 1505 antibody administration on Tau and hyperphosphorylated Tau levels in brain homogenates was examined by Western blotting using antibody-administered Tau-Tg mice brain homogenates.

Sonication was performed on 100 to 200 mg of mouse cerebral hemisphere in a 5-fold amount of TBS (containing protease inhibitor cocktail and phosphatase inhibitor cocktail). This was centrifuged at 100,000 g for 15 minutes at 4°C, and the supernatant was collected as the TBS soluble fraction.

The precipitate was suspended in 1% sarkosyl/TBS (containing protease inhibitor cocktail and phosphatase inhibitor cocktail), and incubated for 1 hour at room temperature. This was centrifuged at 100,000 g for 15 minutes at room temperature, and the supernatant was used as the sarkosyl soluble fraction.

The TBS soluble fraction and sarkosyl soluble fraction were electrophoresed with 7% Tris-Acetate Gel and separated, transferred to a PVDF membrane, and subjected to blocking overnight at room temperature with 1% BSA/3% SkimMilk/0.05% Tween20/TBS. Next, the antibody solution was reacted using HRP conjugate antibody as the secondary antibody, reaction was conducted by the ECL method, and analysis was performed with an LAS3000 image analyzer for quantification.

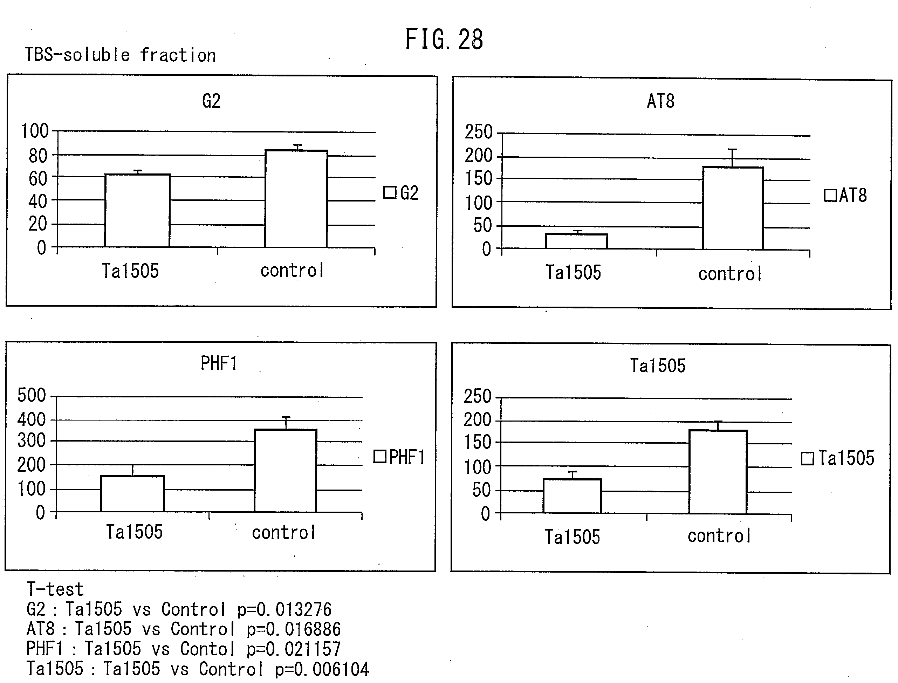

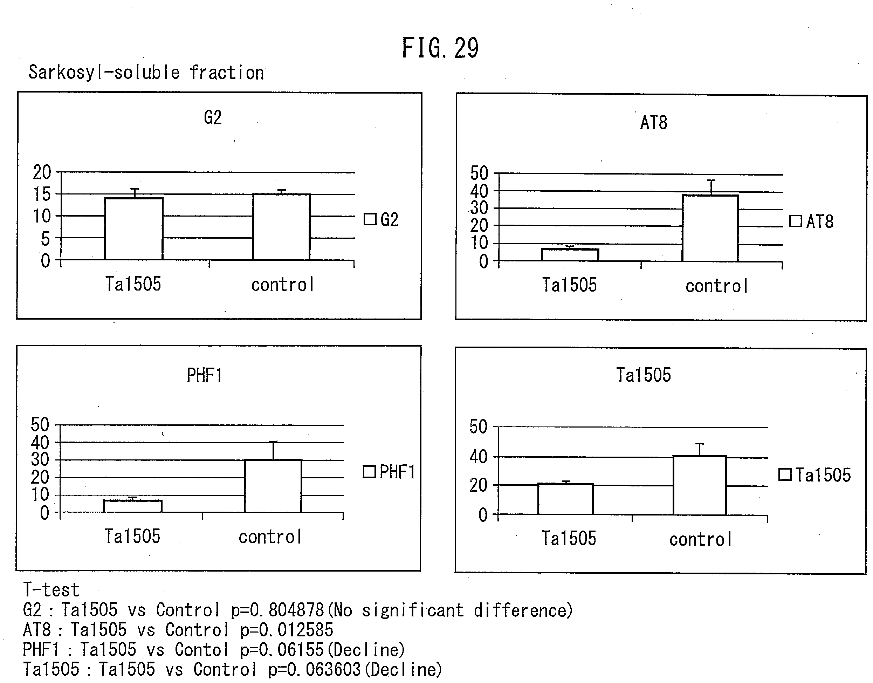

The results are shown in Fig. 28 and Fig. 29.

For the TBS soluble fraction, it was confirmed that Ta1505 antibody administration significantly reduced human Tau (recognized by G2 antibody), hyperphosphorylated Tau (recognized by AT8 (pS202/pT205 epitope) and PHF1 (pS396/pS404 epitope)) and pS413Tau (recognized by Ta1505) in Tau-Tg mice brain.

For the sarkosyl soluble fraction, it was confirmed that Ta1505 antibody administration significantly reduced hyperphosphorylated Tau (recognized by AT8) in Tau-Tg mice brain.

This result provides support that antibody administration ameliorates pathology in the brain and has an improving effect for memory impairment. The results also indicate that intraperitoneally administered antibody acts in the brain.

### [Production Example 1] Tg mice expressing human normal type tau (Tau-Tg mice), as cognitive function impairment model

The pharmacological effect of antibodies of the invention on improving cognitive function was examined using Tg mice having the characteristic of expressing human normal type tau, and particularly the same expression pattern as in ontogenesis in humans, which is expression of only type 3R tau during the embryonic stage and both types 3R and 4R tau with continuing growth, and exhibiting onset of cognitive function impairment at about 6 months after birth. The Tg mice were prepared by the following method.

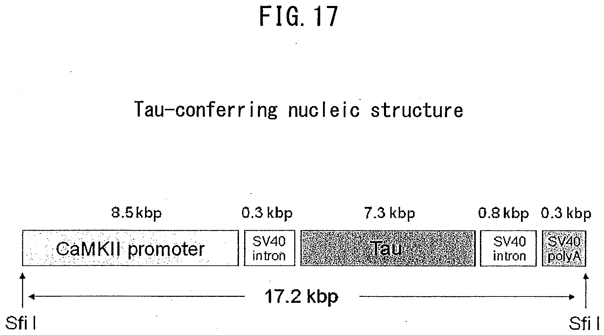

The gene structure used for preparation of the Tg mice was tau gene-conferring nucleic acid having the structure shown in Fig. 17, comprising the Simian virus 40 (SV40) 5'-intron (0.3 kb), tau gene (Tau; 7.3 kb), SV40 3'-intron (0.8 kb) and SV40 polyA signal (0.3 kb) in that order, downstream from the α-calmodulin kinase IIα (CaMKII) promoter (8.5 kb). The tau gene used was obtained by the same method described by Yamashita T. et al. (FEBS Letters, Vol.579, P.241-244, 2005), as a 7.3 kb-length gene comprising the nucleotide sequence of the portion corresponding to exons 1 to 9 from cDNA coding for human tau, a nucleotide sequence including the portion of the first 18 nucleotides and the last 3 kb of intron 9, the nucleotide sequence of exon 10, the portion of the first 3 kb and the last 38 nucleotides of intron 10(with cytosine substituting for thymine at base number 16 from the 5'-end of intron 10) and the nucleotide sequence of cDNA corresponding to exons 11 to 13. The tau gene was cloned at the restriction enzyme EcoRV site of pNN265, a vector including the SV40 5'-intron and 3'-intron and poly(A) signal sequence (Choi T et al., Mol. Cell. Biol. vol.11, pp.3070-3074, 1991). A DNA fragment containing the SV40 5'-intron, the tau gene, the SV40 3'-intron and the poly(A) signal sequence was cut out from the obtained plasmids with restriction enzymes XhoI and NotI, and the DNA fragment was cloned in pMM403 vector including CaMKII promoter (Mayford M et al., Cell vol.81 pp.891-904, 1995). Also, a gene fragment having the structure shown in Fig. 17, containing the tau gene, was cut out from the obtained plasmids with restriction enzyme SfiI (17.2 kb), isolated by agarose electrophoresis, and purified from the corresponding gel region using QIAGEN (R) QIAquick Gel Extraction Kit (Cat. No.28704). The obtained gene (DNA) fragment was incubated with pronuclear-stage embryos obtained by breeding male/female C57BL/6 mice, according to a known method [Hogan, B et al., "Manipulating the mouse embryo. A Laboratory Manual," Cold Spring Harbor Laboratory (1986)]. These were implanted into the uterine tubes of female C57BL mice rendered pseudopregnant by injection. Portions of the tails of the born mice were amputated, PCR was conducted to confirm whether the introduced gene had been transferred, and the female or male mice with the transferred gene were bred with normal female or male mice to establish Tg mice hetero for the transferred tau gene, which were used for experimentation. The Tau-Tg mice mentioned in line 609 and line 784 of the examples are mice produced by the same method and exhibiting equivalent traits (possibly delete reference to lines in examples).

## Claims

1. A therapeutic agent or prophylactic agent for use in a method of treatment or prevention of a tauopathy, wherein the therapeutic agent or prophylactic agent comprises, as an active ingredient, a monoclonal antibody that binds specifically to a tau protein phosphorylated at the amino acid residue corresponding to Ser413 of SEQ ID NO: 1, wherein the binding is defined by affinity represented by an equilibrium dissociation constant (KD) of at most 1 x 10⁻⁶ M, and wherein said binding to the phosphorylated tau protein is at least 10 times stronger than the binding to a tau protein that has not been phosphorylated at said Ser413.

2. The therapeutic agent or prophylactic agent for use according to claim 1, wherein the antibody comprises:
a heavy chain variable region (VH) comprising a CDR-H1 having a sequence selected from SEQ ID NO: 7 or 8; a CDR-H2 having a sequence selected from SEQ ID NO: 9, 10, 11 or 12; and a CDR-H3 having the sequence represented by SEQ ID NO: 13; and
a light chain variable region (VL) comprising a CDR-L1 having a sequence selected from SEQ ID NO: 14 or 15; a CDR-L2 having the sequence represented by SEQ ID NO: 16; and a CDR-L3 having the sequence represented by SEQ ID NO: 17.

3. The therapeutic agent or prophylactic agent for use according to claim 1, wherein the antibody comprises:
a VH comprising a CDR-H1 having the sequence represented by SEQ ID NO: 8; a CDR-H2 having a sequence at least 85% identical to the sequence represented by SEQ ID NO: 9; and a CDR-H3 having the sequence represented by SEQ ID NO: 13; and
a VL comprising a CDR-L1 having a sequence at least 85% identical to the sequence represented by SEQ ID NO: 14; a CDR-L2 having the sequence represented by SEQ ID NO: 16; and a CDR-L3 having the sequence represented by SEQ ID NO: 17.

4. The therapeutic agent or prophylactic agent for use according to any one of claims 1 to 3, wherein the antibody comprises:
a VH comprising a CDR-H1 having the sequence represented by SEQ ID NO: 8; a CDR-H2 having the sequence represented by SEQ ID NO: 9; and a CDR-H3 having the sequence represented by SEQ ID NO: 13; and
a VL comprising a CDR-L1 having the sequence represented by SEQ ID NO: 14; a CDR-L2 having the sequence represented by SEQ ID NO: 16; and a CDR-L3 having the sequence represented by SEQ ID NO: 17.

5. The therapeutic agent or prophylactic agent for use according to any one of claims 2 to 4, wherein the antibody comprises:
a VH having a sequence at least 85% identical to the sequence represented by SEQ ID NO: 20; and
a VL having a sequence at least 85% identical to the sequence represented by SEQ ID NO: 26.

6. The therapeutic agent or prophylactic agent for use according to any one of claims 1 to 5, wherein the antibody comprises:
a VH having the sequence represented by SEQ ID NO: 20; and
a VL having the sequence represented by SEQ ID NO: 26.

7. The therapeutic agent or prophylactic agent for use according to any one of claims 1 to 6, wherein the antibody is a humanized antibody or a chimeric antibody.

8. The therapeutic agent or prophylactic agent for use according to any one of claims 1 to 7, wherein the antibody binding to the tau protein phosphorylated at the amino acid residue corresponding to Ser413 of SEQ ID NO: 1 is at least 30 times, or at least 100 times stronger than the binding to a tau protein that has not been phosphorylated at said Ser413.

9. The therapeutic agent or prophylactic agent for use according to any one of claims 1 to 7, wherein the antibody binds to the tau protein phosphorylated at the amino acid residue corresponding to Ser413 of SEQ ID NO: 1 with an affinity represented by the KD of at most 1x10⁻⁷M or at most 1x10⁻⁸M.

10. The therapeutic agent or prophylactic agent for use according to any one of claims 1 to 9, wherein the binding affinity of the antibody is determined by a binding measurement using surface plasmon resonance.

11. A therapeutic agent or prophylactic agent for use in a method of treatment or prevention of a tauopathy containing, as an active ingredient, a peptide comprising a portion of tau protein, wherein said peptide comprises the amino acids 410-421 of SEQ ID NO: 1, wherein the amino acid residue corresponding to Ser413 is phosphorylated.

12. The therapeutic agent or prophylactic agent for use according to any one of claims 1 to 11, wherein the tauopathy is Alzheimer's disease, cortical-basal ganglia degeneration, progressive supranuclear palsy, Pick's disease, argyrophilic grain dementia (argyrophilic grain disease), Multiple system tauopathy with dementia (MSTD), chromosome 17-linked frontotemporal dementia with Parkinsonism (FTDP-17), neurofibrillary tangle dementia, diffuse neurofibrillary tangles with calcification (DNTC), white matter tauopathy with globular glial inclusions (WMT-GGI) or frontotemporal lobar degeneration with tau-positive inclusions (FTLD-tau).

## Patentansprüche

1. Therapeutikum oder Prophylaktikum zur Verwendung in einem Verfahren zur Behandlung oder Prävention einer Tauopathie, wobei das Therapeutikum oder Prophylaktikum einen monoklonalen Antikörper als einen Wirkstoff umfasst, der spezifisch an ein Tau-Protein bindet, das an dem Aminosäurerest, der Ser413 der SEQ ID Nr: 1 entspricht, phosphoryliert ist, wobei die Bindung durch eine Affinität definiert ist, die durch eine Gleichgewichtsdissoziationskonstante (KD) von höchstens 1 x 10⁻⁶M dargestellt wird, und wobei die Bindung an das phosphorylierte Tau-Protein mindestens 10 mal stärker ist als die Bindung an ein Tau-Protein, das nicht an Ser413 phosphoryliert wurde.

2. Therapeutikum oder Prophylaktikum zur Verwendung nach Anspruch 1, wobei der Antikörper umfasst:
eine variable Region der schweren Kette (VH), umfassend eine CDR-H1 mit einer Sequenz, ausgewählt aus SEQ ID Nr.: 7 oder 8; eine CDR-H2 mit einer Sequenz, ausgewählt aus SEQ ID Nr.: 9, 10, 11 oder 12; und eine CDR-H3 mit der durch SEQ ID Nr.: 13 dargestellten Sequenz; und
eine variable Region der leichten Kette (VL), umfassend eine CDR-L1 mit einer Sequenz, ausgewählt aus SEQ ID Nr.: 14 oder 15; eine CDR-L2 mit der durch SEQ ID Nr.: 16 dargestellten Sequenz; und eine CDR-L3 mit der durch SEQ ID Nr.: 17 dargestellten Sequenz.

3. Therapeutikum oder Prophylaktikum zur Verwendung nach Anspruch 1, wobei der Antikörper umfasst:
eine VH, umfassend eine CDR-H1 mit der durch SEQ ID Nr.: 8 dargestellten Sequenz; eine CDR-H2 mit einer Sequenz, die mindestens zu 85 % mit der durch SEQ ID Nr.: 9 dargestellten Sequenz identisch ist; und eine CDR-H3 mit der durch SEQ ID Nr.: 13 dargestellten Sequenz; und
eine VL, umfassend eine CDR-L1 mit einer Sequenz, die mindestens zu 85 % mit der durch SEQ ID Nr.: 14 dargestellten Sequenz identisch ist; eine CDR-L2 mit der durch SEQ ID Nr.: 16 dargestellten Sequenz; und eine CDR-L3 mit der durch SEQ ID Nr.: 17 dargestellten Sequenz.

4. Therapeutikum oder Prophylaktikum zur Verwendung nach einem der Ansprüche 1 bis 3, wobei der Antikörper umfasst:
eine VH, umfassend eine CDR-H1 mit der durch SEQ ID Nr.: 8 dargestellten Sequenz; eine CDR-H2 mit der durch SEQ ID Nr.: 9 dargestellten Sequenz; und eine CDR-H3 mit der durch SEQ ID Nr.: 13 dargestellten Sequenz; und
eine VL, umfassend eine CDR-L1 mit der durch SEQ ID Nr.: 14 dargestellten Sequenz; eine CDR-L2 mit der durch SEQ ID Nr.: 16 dargestellten Sequenz; und eine CDR-L3 mit der durch SEQ ID Nr.: 17 dargestellten Sequenz.

5. Therapeutikum oder Prophylaktikum zur Verwendung nach einem der Ansprüche 2 bis 4, wobei der Antikörper umfasst:
eine VH mit einer Sequenz, die mindestens zu 85 % mit der durch SEQ ID Nr.: 20 dargestellten Sequenz identisch ist; und
eine VL mit einer Sequenz, die mindestens zu 85 % mit der durch SEQ ID Nr.: 26 dargestellten Sequenz identisch ist.

6. Therapeutikum oder Prophylaktikum zur Verwendung nach einem der Ansprüche 1 bis 5, wobei der Antikörper umfasst:
eine VH mit der durch SEQ ID Nr: 20 dargestellten Sequenz; und
eine VL mit der durch SEQ ID Nr: 26 dargestellten Sequenz.

7. Therapeutikum oder Prophylaktikum zur Verwendung nach einem der Ansprüche 1 bis 6, wobei der Antikörper ein humanisierter Antikörper oder ein chimärer Antikörper ist.

8. Therapeutikum oder Prophylaktikum zur Verwendung nach einem der Ansprüche 1 bis 7, wobei die Antikörperbindung an das Tau-Protein, das an dem Aminosäurerest, der Ser413 der SEQ ID Nr.: 1 entspricht, phosphoryliert ist, mindestens 30 mal oder mindestens 100 mal stärker ist als die Bindung an ein Tau-Protein, das nicht an Ser413 phosphoryliert wurde.

9. Therapeutikum oder Prophylaktikum zur Verwendung nach einem der Ansprüche 1 bis 7, wobei der Antikörper an das Tau-Protein, das an dem Aminosäurerest, der Ser413 der SEQ ID Nr.: 1 entspricht, phosphoryliert ist, mit einer Affinität bindet, die durch die KD von höchstens 1 x 10⁻⁷ M oder höchstens 1 x 10⁻⁸ M dargestellt ist.

10. Therapeutikum oder Prophylaktikum zur Verwendung nach einem der Ansprüche 1 bis 9, wobei die Bindungsaffinität des Antikörpers anhand einer Bindungsmessung unter Verwendung von Oberflächenplasmonresonanz bestimmt wird.

11. Therapeutikum oder Prophylaktikum zur Verwendung in einem Verfahren zur Behandlung oder Prävention einer Tauopathie, das als einen Wirkstoff ein Peptid enthält, das einen Teil des Tau-Proteins umfasst, wobei das Peptid die Aminosäuren 410-421 der SEQ ID Nr.: 1 umfasst, wobei der Aminosäurerest, der Ser413 entspricht, phosphoryliert ist.

12. Therapeutikum oder Prophylaktikum zur Verwendung nach einem der Ansprüche 1 bis 11, wobei die Tauopathie die Alzheimer-Krankheit, Degeneration der kortikalen Basalganglien, progressive supranukleäre Blickparese, die Pick-Krankheit, Demenz mit argyrophilen Körnern (Silberkornkrankheit), Multiple Systemtauopathie mit Demenz (MSTD), frontotemporale Demenz mit Parkinsonismus des Chromosoms 17 (FTDP-17), Neurofibrillenbündel-Demenz, diffuse Neurofibrillenbündel mit Verkalkung (DNTC), Tauopathie der weißen Substanz mit globulären glialen Einschlüssen (WMT-GGI) oder Frontotemporale Lobärdegeneration mit Tau-positiven Einschlüssen (FTLD-Tau) ist.

## Revendications

1. Agent thérapeutique ou agent prophylactique pour l'utilisation dans un procédé de traitement ou de prévention d'une tauopathie, dans lequel l'agent thérapeutique ou l'agent prophylactique comprend, en tant que principe actif, un anticorps monoclonal qui se lie spécifiquement à une protéine tau phosphorylée au niveau du résidu d'acide aminé correspondant à Ser413 de SEQ ID N°: 1, dans lequel la liaison est définie par une affinité représentée par une constante de dissociation à l'équilibre (KD) d'au plus 1 x 10⁻⁶ M, et dans lequel ladite liaison à la protéine tau phosphorylée est au moins 10 fois plus forte que la liaison à une protéine tau qui n'a pas été phosphorylée au niveau de ladite Ser413.

2. Agent thérapeutique ou agent prophylactique pour l'utilisation selon la revendication 1, dans lequel l'anticorps comprend:
une région variable de chaîne lourde (VH) comprenant une CDR-H1 ayant une séquence choisie parmi la SEQ ID N°: 7 ou 8; une CDR-H2 ayant une séquence choisie parmi la SEQ ID N°: 9, 10, 11 ou 12; et une CDR-H3 ayant la séquence représentée par la SEQ ID N°: 13; et
une région variable de chaîne légère (VL) comprenant une CDR-L1 ayant une séquence choisie parmi la SEQ ID N°: 14 ou 15; une CDR-L2 ayant la séquence représentée par la SEQ ID N°: 16; et une CDR-L3 ayant la séquence représentée par la SEQ ID N°: 17.

3. Agent thérapeutique ou agent prophylactique pour l'utilisation selon la revendication 1, dans lequel l'anticorps comprend:
une VH comprenant une CDR-H1 ayant la séquence représentée par la SEQ ID N°: 8; une CDR-H2 ayant une séquence au moins 85 % identique à la séquence représentée par la SEQ ID N°: 9; et une CDR-H3 ayant la séquence représentée par la SEQ ID N°: 13; et
une VL comprenant une CDR-L1 ayant une séquence au moins 85 % identique à la séquence représentée par la SEQ ID N°: 14; une CDR-L2 ayant la séquence représentée par la SEQ ID N°: 16; et une CDR-L3 ayant la séquence représentée par la SEQ ID N°: 17.

4. Agent thérapeutique ou agent prophylactique pour l'utilisation selon l'une quelconque des revendications 1 à 3, dans lequel l'anticorps comprend:
une VH comprenant une CDR-H1 ayant la séquence représentée par la SEQ ID N°: 8; une CDR-H2 ayant la séquence représentée par la SEQ ID N°: 9; et une CDR-H3 ayant la séquence représentée par la SEQ ID N°: 13; et
une VL comprenant une CDR-L1 ayant la séquence représentée par la SEQ ID N°: 14; une CDR-L2 ayant la séquence représentée par la SEQ ID N°: 16; et une CDR-L3 ayant la séquence représentée par la SEQ ID N°: 17.

5. Agent thérapeutique ou agent prophylactique pour l'utilisation selon l'une quelconque des revendications 2 à 4, dans lequel l'anticorps comprend:
une VH ayant une séquence au moins 85 % identique à la séquence représentée par la SEQ ID N°: 20; et
une VL ayant une séquence au moins 85 % identique à la séquence représentée par la SEQ ID N°: 26.

6. Agent thérapeutique ou agent prophylactique pour l'utilisation selon l'une quelconque des revendications 1 à 5, dans lequel l'anticorps comprend:
une VH ayant la séquence représentée par la SEQ ID N°: 20; et
une VL ayant la séquence représentée par la SEQ ID N°: 26.

7. Agent thérapeutique ou agent prophylactique pour l'utilisation selon l'une quelconque des revendications 1 à 6, dans lequel l'anticorps est un anticorps humanisé ou un anticorps chimérique.

8. Agent thérapeutique ou agent prophylactique pour l'utilisation selon l'une quelconque des revendications 1 à 7, dans lequel la liaison de l'anticorps à la protéine tau phosphorylée au niveau du résidu d'acide aminé correspondant à Ser413 de SEQ ID N°: 1 est au moins 30 fois, ou au moins 100 fois plus forte que la liaison à une protéine tau qui n'a pas été phosphorylée au niveau de ladite Ser413.

9. Agent thérapeutique ou agent prophylactique pour l'utilisation selon l'une quelconque des revendications 1 à 7, dans lequel l'anticorps se lie à la protéine tau phosphorylée au niveau du résidu d'acide aminé correspondant à Ser413 de SEQ ID N°: 1 avec une affinité représentée par le KD d'au plus 1 x 10⁻⁷ M ou d'au plus 1 x 10⁻⁸ M.

10. Agent thérapeutique ou agent prophylactique pour l'utilisation selon l'une quelconque des revendications 1 à 9, dans lequel l'affinité de liaison de l'anticorps est déterminée par une mesure de liaison utilisant une résonance plasmonique de surface.

11. Agent thérapeutique ou agent prophylactique pour l'utilisation dans un procédé de traitement ou de prévention d'une tauopathie contenant, en tant que principe actif, un peptide comprenant une partie de protéine tau, dans lequel ledit peptide comprend les acides aminés 410-421 de SEQ ID N°:1, dans lequel le résidu d'acide aminé correspondant à Ser413 est phosphorylé.

12. Agent thérapeutique ou agent prophylactique pour l'utilisation selon l'une quelconque des revendications 1 à 11, dans lequel la tauopathie est la maladie d'Alzheimer, une dégénérescence des ganglions cortico-basaux, la maladie de Steele-Richardson, la maladie de Pick, la démence des grains argyrophiles (maladie des grains argyrophiles), la tauopathie systémique multiple associée à une démence (MSTD), la démence fronto-temporale liée au chromosome 17 associée à un syndrome parkinsonien (FTDP-17), la démence à dégénérescence neurofibrillaire, les enchevêtrements neurofibrillaires diffus avec calcification (DNTC), la tauopathie de la substance blanche avec inclusions gliales globulaires (WMT-GGI) ou la dégénérescence lobaire fronto-temporale avec inclusions tau-positives (FTLD-tau).
